# EUROPEAN PATENT SPECIFICATION

(11) **EP 4 308 693 B1**
(45) Date of publication and mention of the grant of the patent: **11.06.2025**
(21) Application number: 22716392.0
(22) Date of filing: 18.03.2022
(51) Int. Cl.: C12N 5/0786, A61K 35/15, A61P 25/00

(54) **METHOD FOR OBTAINING TUMOR-HYPOXIA EDUCATED REGENERATIVE MACROPHAGES AND USE THEREOF IN REGENERATIVE MEDICINE**
VERFAHREN ZUR HERSTELLUNG VON TUMORHYPOXIE-EDUZIERTEN REGENERATIVEN MAKROPHAGEN UND VERWENDUNG DAVON IN DER REGENERATIVEN MEDIZIN
PROCÉDÉ POUR OBTENIR DES MACROPHAGES RÉGÉNÉRATEURS ÉDUQUÉS À L'HYPOXIE TUMORALE ET LEUR UTILISATION EN MÉDECINE RÉGÉNÉRATIVE

(30) Priority: 18.03.2021 IT 202100006569
(43) Date of publication of application: 24.01.2024
(73) Proprietor: HEMERA S.R.L., 37122 Verona (IT); Humanitas Mirasole S.p.A., 20089 Rozzano (MI) (IT)
(72) Inventor: DECIMO, Ilaria, 37122 Verona (VR) (IT); BIFARI, Francesco, 37122 Verona (VR) (IT); DOLCI, Sissi, 37060 Castel D'Azzano (VR) (IT); FUMAGALLI, Guido Francesco, 37138 Verona (VR) (IT); LOCATI, Massimo, 21042 Caronno Pertusella ( VA) (IT)
(74) Representative: Currado, Luisa
(86) International application number: PCT/EP2022/057246
(87) International publication number: WO 2022/195114

(56) References cited:
- EP-A1- 3 299 453
- EP-A1- 3 508 207
- WO-A2-2020/169472
- SETTEN E ET AL: "Hypoxia effect on macrophages acivation and fibroblasts cross-talk in chronic inflammation and tumor microenvironment", CANCER MICROENVIRONMENT, vol. 11, no. S1, 19 May 2018 (2018-05-19), pages S73, XP55928950, ISSN: 1875-2292, DOI: 10.1007/s12307-018-0212-6
- GENSEL J C & ZHANG B: "Macrophage activation and its role in repair and pathology after spinal cord injury", BRAIN RESEARCH, vol. 1619, 4 September 2015 (2015-09-04), pages 1 - 11, XP055857878, ISSN: 0006-8993, DOI: 10.1016/j.brainres.2014.12.045
- MA S-F ET AL: "Adoptive transfer of M2 macrophages promotes locomotor recovery in adult rats after spinal cord injury", BRAIN, BEHAVIOR AND IMMUNITY, vol. 45, March 2015 (2015-03-01), pages 157 - 170, XP055857883, ISSN: 0889-1591, DOI: 10.1016/j.bbi.2014.11.007
- JIN M C ET AL: "Stem cell therapies for acute spinal cord injury in humans: a review", NEUROSURGICAL FOCUS, vol. 46, no. 3, 1 March 2019 (2019-03-01), pages E10, XP055857882, ISSN: 1092-0684, DOI: 10.3171/2018.12.FOCUS18602
- SETTEN E & LOCATI M: "Hypoxia effect on macrophages activation and fibroblasts cross-talk in chronic inflammation and tumor microenvironment", CANCER MICROENVIRONMENT, vol. 11, no. Suppl. 1, P27, 19 May 2018 (2018-05-19), 8th International Conference on Tumor Microenvironment; Lisbon, Portugal; 10-14 June 2018, pages S73, XP055928950, ISSN: 1875-2292, DOI: 10.1007/s12307-018-0212-6

## Description

### FIELD OF THE INVENTION

The present invention relates to a method for making macrophages acting as regenerative bioreactor for use in regenerative medicine. The method disclosed in the present invention relates in particular to the activation of macrophages in vitro by incubation takes under hypoxic conditions and by means of a medium comprising factors released by tumor cell line or tumor cells dissociated from tumor mass, or dissociated tumor explant or tumor resections . The macrophages thus obtained assume a unique regenerative phenotype not present in other polarized phenotypes, including M2 macrophages.

The *regenerative bioreactor* phenotype of mouse derived cells as defined herein comprises a broad expression of metalloproteases (including Mmp8, Mmp9, Mmp10, Mmp12, Mmp14, Mmp19, Mmp27), trophic factors (including VEGFs, FGFs, IGFs), and cell contact and adhesion molecules (including Rap2A, Ninj 1, Antxr2, Itga1, Itga6, Itga9, ItgaM, Adamtsl4, Adamtsl6), mediators which promote survival (including Rtn4r12), and immunomodulation (including Arg1, Cxcl1, Cxcl2, Cxcl3, Cxcl16, Fcgr1, Fcgr4, Ltb4r1, Jmjd6). The phenotype of human derived cells as defined herein comprises a broad expression of genes related to wound healing (including Adm, Bnip3, Pdgfb, Vegfa), angiogenesis (including Vegfa, Angptl4, Cxcl8, Lep, Rora, Apln), detoxification and regulation of defence response (including Ndrg1, Mt1e, Mt1f, Mt1g, Mt1h, Mt1x, Mt2a, Mt3, Ddit4, Nupr1), response to hypoxia (including Hk2, Pfkfb3, Slc2a1, Slc2a3, Cxcr4, Plin2, Adm, Bnip3, Lep, Rora, Ndrg1, Egln3, Mt3, Plod2, Hilpda, Angptl4), extracellular matrix remodelling (including Mmp9, Vcan, Fgf11, Cxcl8, Lep, Pdgfb, Plod2, Vegfa, Angptl4, Sulf2, Egln3), and neuronal survival and myelination (including Mt3, Jam2, Vldlr, Nupr1, Egln3). In addition, the invention relates to the use of the bioreactor thus produced for the regeneration of tissues, including nervous tissue.

### PRIOR ART

The central nervous system (CNS) comprises the brain, which coordinates higher-level functions (cognitive), and the spinal cord, which serves primarily as a communication pathway between the brain and the periphery (exteroceptive and motor). Unlike other human tissues, the regenerative capacity of nervous tissue is poor and nerve tissue lesions do not heal spontaneously [1]. Nervous tissue lesions do not heal spontaneously. In the CNS, the lost tissue is replaced by scar tissue with a glial, fibrotic component and composed of inflammatory immune cells [2]. The evolution of the lesion involves the replacement of neural tissue with scar tissue with consequent loss of continuity of neuronal connections, the function thereof and the appearance of disability. Disabilities due to CNS injuries depend on the mode, severity and anatomical position of the injury.

Each year around 250-500,000 people worldwide suffer a spinal injury resulting in high medical care costs (estimated at around 1-4 million euros/patient) [3, 4]. Despite high social and economic costs, there are currently few possible treatments developed for spinal injury care. In addition, at the moment the available therapies are only capable of limiting the secondary damage caused by spinal trauma and preserving the few axonal connections left intact at the site of the lesion. In the last 30 years the scientific community has developed many approaches which have led to a considerable increase in the survival rate following spinal cord injuries (SCI), however there is still no effective therapy for the treatment of spinal injury and thus for optimal complete locomotor recovery. At the pathological level, the spinal injury causes the loss of the neuronal component at the injury site, which leads to an interruption of the transmission of impulses between the CNS and the periphery (muscles). Nervous tissue degeneration is a result of a complex phenomenon involving tissue ischemia, cell death and inflammation.

### Pathological microenvironment of the CNS lesion which induces progression of neural damage and prevents regeneration of neural tissue (Biological need)

In addition to the primary damage caused by the injury to the neural tissue, there is secondary damage due to the decrease in blood perfusion, which involves an inadequate supply of substrates (O2 and nutrients) to maintain the metabolism of the neural cells, and the increase in vascular permeability which causes edema and inflammatory infiltrate which further compresses the neural tissue remaining around the lesion and aggravates the lesion. These complex conditions increase the death of neural tissue cells surrounding the area primarily involved. To circumscribe the area of damage, a strong inflammation develops, with reactive activation of the astrocytes delimiting the lesion. The astrocytes which form the glial scar, together with the inflammatory immune cells, in particular the inflammatory macrophages (called M1), and the stromal cells then produce a characteristic extracellular matrix which fills the lesion area but inhibits axonal growth and neural cell differentiation. Furthermore, the inflammatory components are chronically activated by cell death and contribute to the chronic progression of microenvironment impairment of the lesion and neural tissue damage. Therefore, the patho-physiological processes indicate a complex involvement of multiple factors which contribute to the formation, progression and chronicity of neural tissue lesion [4-6]. Currently, the methods of regenerative medicine for diseases characterized by loss of neural tissue are mainly based on the use of stem cells aimed at replacing *(cell replacement)* the lost neural tissue. For this, embryonic stem cells (ESCs) and/or fetal neural stem cells (NSCs) and the neural differentiated derivatives thereof (e.g., oligodendrocyte precursors) or neural differentiated induced pluripotent cells (iPSCs) (iPSC-NSCs) have been tested [7, 8]. These therapies have not yet shown clinical efficacy in the severe neurodegeneration model used by the present inventors, characterized by complete contusive spinal cord injury.

In the search for a cell source which could be taken directly by the patient himself to then be transplanted for regenerative purposes (autologous setting), numerous other adult stem cell populations have also been tested. However, these studies showed a partial benefit of these treatments, supported not by a direct differentiation of the cells injected into neural cells but by a *"bystander"* effect, i.e., trophic and inflammation modulating. The stem cells showing these properties include adult neural stem cells (aNSCs, which exist only in mice), hematopoietic stem cells (HSCs) and mesenchymal stem cells (MSCs) originating from bone marrow (BM-) or adipose tissue (A-)[7]. With varying efficacy, these cell types show a certain regenerative capacity, providing the damaged tissue with trophic factors and decreasing inflammation. However, these approaches have not been sufficiently effective in the therapies of severe chronic neurodegenerative diseases. As a last class of cells, the regenerative capacity of immune cells including macrophages has also recently been tested. Macrophages induced towards the M2 phenotype injected intravenously or at the site of neural tissue injury have shown a partial regenerative efficacy, although the models used did not correspond to a severe and chronic neurodegenerative disease [9]. The therapeutic effect of M2 macrophages transplantation was tested in a phase II clinical trial with spinal cord injured patient. Although M2 transplantation did not show any significant adverse events, the study failed to demonstrate a significant difference in the primary outcome of M2 treated compared to control untreated patients [10] . Therefore the study by Lammertse at al. did not support the efficacy of the treatment of acute complete SCI with autologous M2 macrophages .

In conclusion, the prior art analysis indicates that there is still a need for a therapy capable of treating diseases characterized by severe loss of neural tissue, as no effective therapy exists for these conditions.

### DESCRIPTION OF THE INVENTION

The present inventors have developed a therapy for diseases characterized by severe loss of neural tissue based on the use of a complex bioreactor, "Tumor hypoxia educated macrophages" (THEMs) (that may be also defined as "Tumor Educated Macrophages" (TEMs)), which have shown to be capable of partially regenerating a severe loss of neural tissue and promoting motor recovery. As a paradigm of neural tissue loss, the present authors tested the treatment efficacy in an experimental animal model of severe (complete) contusive spinal cord injury (SCI).

The properties of the tumor, considered the best example of "regeneration" existing in nature, were used to transform immune cells into effective bioreactors in the regeneration of degenerative diseases characterized by severe loss of neural tissue. In particular, immune cells are "educated" with a tumor conditioning and hypoxia, as will be described below.

The THEMs (Tumor and Hypoxia Educated Macrophages) have been then injected in SCI animals at the lesion site. The administration of THEMs is therapeutic, i.e., the cells are injected days after the injury. The THEMs were tested in more than 200 animals in 7 independent experiments and the effect thereof was evaluated using multidisciplinary methods.

There are two main findings related to the present approach:
1- the level of effectiveness is higher in terms of level of motor recovery and frequency of positive cases with respect to what inventors found with the currently proposed therapies, including the various types of transplanted stem cells;
2- unlike other therapeutic intervention models in which the treatment is administered before or immediately after the lesion (24 hours), in the present case the cell therapy is initiated 4 days after the lesion, i.e., in a phase corresponding to a realistic condition for human therapy.

To assess the role of hypoxia on THEM regenerative effect, inventors performed *in vitro* and *in vivo* experiments aimed to characterize the specific hypoxia-induced effect on THEM molecular and functional phenotype.

In particular, inventors defined THEM with hypoxia specific signature by analyzing the whole transcriptomic of THEM (cultured with hypoxia) and compared it with that of THEM cultured without hypoxia (Tumor Conditioned macrophages, TCM) and macrophages polarized towards M0, and M2 phenotype.

They also performed a migratory assay comparing THEM (cultured with hypoxia), TCM (cultured without hypoxia), and M2 macrophages to verify that hypoxia increased the chemotactic response to SDF-1 of THEM. They performed co-culture experiments with human motor neurons derived by iPSCs, and compared the effect on neuronal process generation and elongation of both murine and human THEM cultured with hypoxia, TCM cultured without hypoxia, and M2 macrophages to verify that hypoxia treatment confers to THEM *in vitro* neuronal regenerative function on human motor neurons. Further, inventors compared the *in vivo* effect on spinal cord injured mice motor recovery of THEM (cultured with hypoxia), and TCM and verified that the hypoxia treatment confer to THEM *in vivo* neuronal regenerative functions.

Therefore, an object of the invention is an *in vitro* or *ex vivo* method for incubating a population of mononuclear phagocytes isolated from biological samples characterized in that the incubation takes place under hypoxic conditions and the culture medium comprises factors released from solid tumor or tumor explants or tumor cell lines.

Preferably, the incubated mononuclear phagocyte population is an *in vitro* culture of monocytes (that may be defined as circulating precursors of macrophages) isolated from biological samples. The monocytes can for example be drawn from peripheral blood by means of centrifugation gradient or thawed from previous isolation of mononuclear cells from peripheral blood.

Preferably the mononuclear phagocyte is a monocyte.

The culture medium preferably comprises factors capable of differentiating the monocytes into macrophages, preferably Macrophage Colony-Stimulating factor. (M-CSF).

Preferably the mononuclear phagocytes are macrophages, preferably murine or human, preferably obtained from cells taken from the bone marrow or derived from blood e.g. by means of repeated centrifugation gradient.

Hypoxic conditions refer to the use of oxygen concentrations lower than atmospheric concentrations.

The population is preferably incubated for from 6 hours or 12 hours to 15 or 20 days, more preferably 6 hours-10 days or preferably 3-8 days or 6 hours-72 hours, even more preferably 18 hours-24 hours or 6 days, even more preferably 7 days or 18 hours.

The mononuclear phagocytes induced by phenotypic and/or functional change are preferably macrophages. Therefore in a preferred embodiment the obtained cell is a macrophage.

The culture medium comprises a tumor supernatant, said supernatant preferably being obtained from cultures of solid tumor, tumor explants or tumor cell lines, preferably from fibrosarcoma or glioma.

Preferably the tumor supernatant is a medium conditioned from the tumor ( also herein defined as conditioned tumor medium, CTM) preferably produced from tumor cell lines, tumor explants, or solid tumor, or from resections of dissociated and plated in vitro tumors preferably for a period of about 12 hours-20 days, more preferably about 12- 72 hours.

Preferably the culture medium consists of cell culture medium, e.g., Eagle's minimal essential medium or derivatives thereof, and/or Roswell Park Memorial Institute (RPMI) 1640, and/or Media 199 and/or Fischer's medium, and 1-99%, preferably 10-90%, more preferably 30-50% even more preferably 30% or 50% medium conditioned from the tumor (CTM) or tumor supernatant.

The macrophages are preferably differentiated into a medium comprising M-CSF o and optionally Dulbecco modified Eagle medium (D-MEM) and/or heat-inactivated fetal bovine serum and/or penicillin/streptomycin, and/or glutamine, preferably for 6 days.

The macrophages, preferably differentiated, are preferably cultured in a medium conditioned from the tumor (CTM) and D-MEM in a 1:9-9:1 ratio, preferably 1:1 ratio, and preferably grown under hypoxic conditions (e.g., 1% O2) preferably for 18 h.

In a preferred aspect, the macrophages are preferably cultured *in vitro* for 6 days preferably in an RPMI 1640 medium containing 10% heat-inactivated fetal calf serum, and 100 ng/ml Macrophage Colony-Stimulating Factor (M-CSF) and optionally 100 U/mL penicillin/streptomycin, 2 mM L-glutamine, with 30% CTM.

The monocyte culture is incubated in hypoxic conditions (0.5-20% O2, preferably 1% O2) for the entire culture period 7 days, or for the last 6-96 hours, preferably the last 18-24 hours, more preferably the last 18 hours.

Another object of the invention are mononuclear phagocytes, preferably macrophages, obtainable by the method according to the invention.

Said phagocytes:
i) express at least one of the following genes: CXCR4, CYTIP, SLC2A3 and MT2A; and
ii) express low/no level of at least one of the following genes: PLXNA2, HSPH1, CYCS and TIGAR, wherein for low level is intended a gene expression level which is at least five fold lower than the level of expression the housekeeping gene beta-actin,

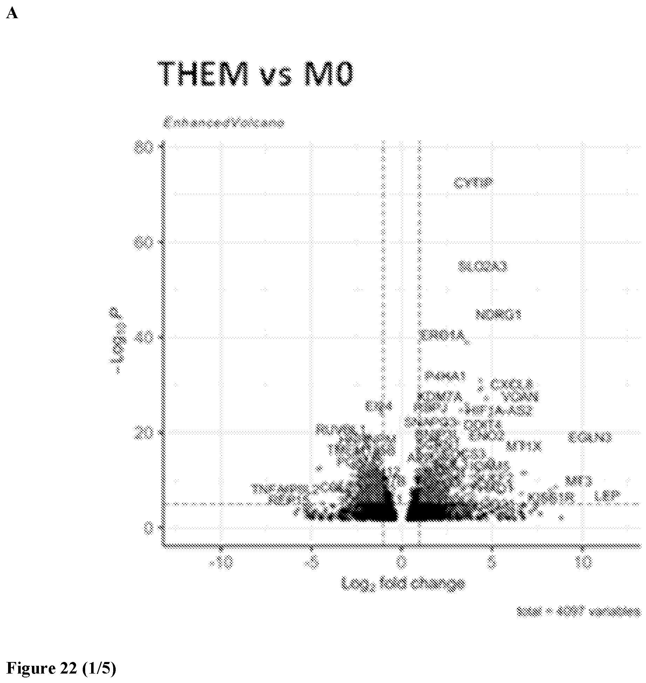

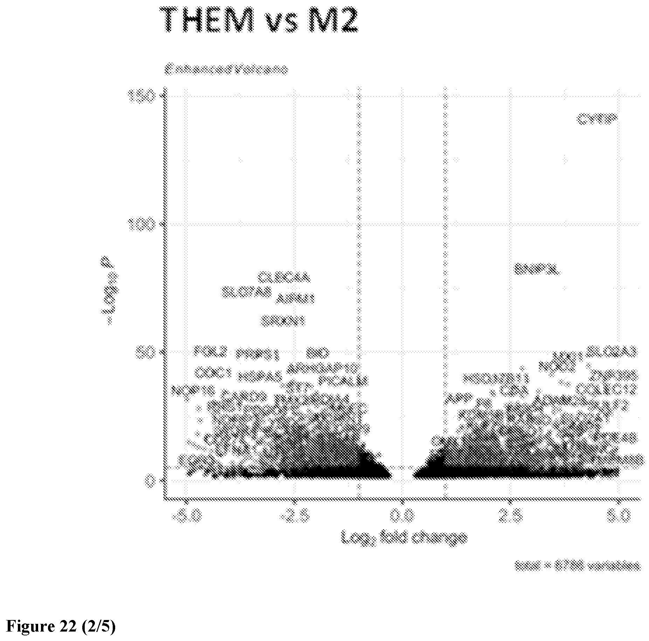

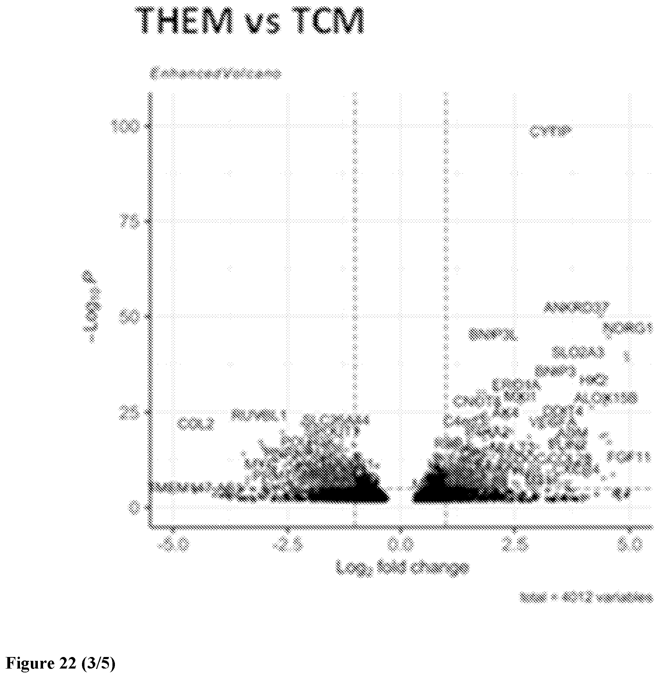

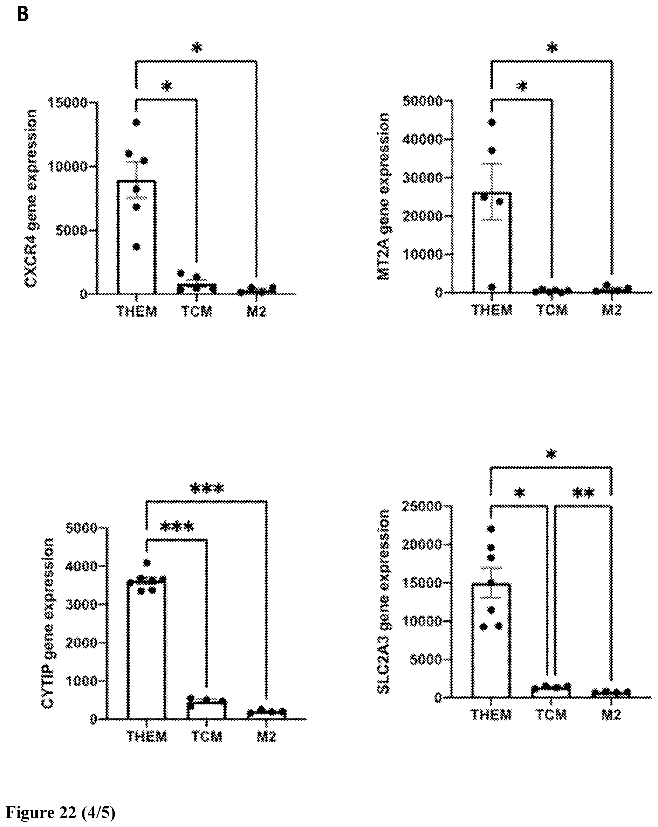

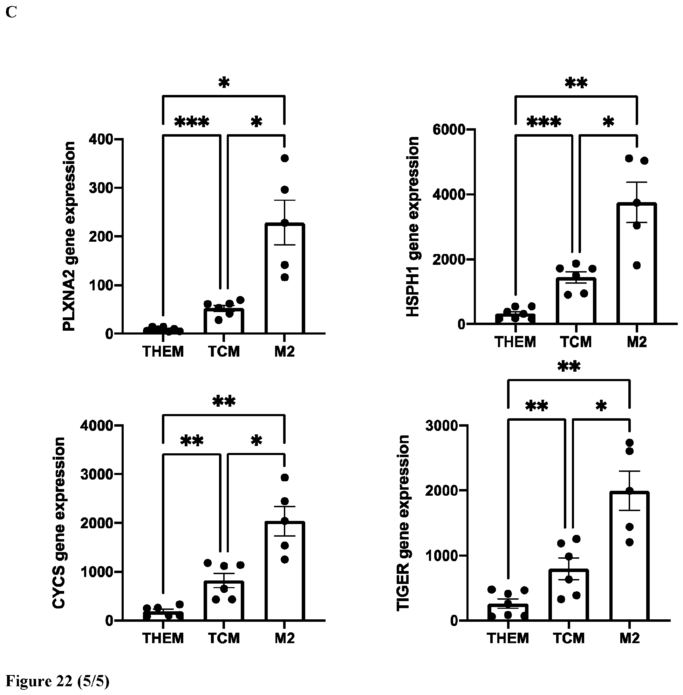

M2 cells indeed express very low/no level of CXCR4, CYTIP, SLC2A3 and MT2A and express PLXNA2, HSPH1, CYCS and TIGAR (see figures 22 b and c).

A further object of the invention is an isolated mononuclear phagocyte, preferably macrophage, which:
i) expresses at least one of the following genes: CXCR4, CYTIP, SLC2A3 and MT2A; and
ii) expresses low/no level of at least one of the following genes: PLXNA2, HSPH1, CYCS and TIGAR herein for low level is intended a gene expression level which is at least five fold lower than the level of expression the housekeeping gene beta-actin,

Preferably the mononuclear phagocyte of the invention, preferably a macrophage, expresses: CXCR4, CYTIP, SLC2A3 and MT2A; and expresses low/no level of: PLXNA2, HSPH1, CYCS and TIGAR.

A further object of the invention is population of mononuclear phagocytes comprising at least one mononuclear phagocyte as defined herein.

The mononuclear phagocytes or the population of isolated mononuclear phagocytes of the invention, preferably said mononuclear phagocytes being macrophages, are preferably characterized by the expression of at least one of the genes of Table 1 and/or Table 2 and/or Table 3, and/or Table 6 and / or by the secretion of at least one of the molecules of Table 2.

The mononuclear phagocytes or the population of isolated mononuclear phagocytes of the invention, preferably said mononuclear phagocytes being macrophages, are preferably characterized by the expression of metalloproteases (for example Mmp8, Mmp9, Mmp10, Mmp12, Mmp14, Mmp19, Mmp27) and / or of trophic factors (for example VEGFs, FGFs, IGFs) and / or cell contact and adhesion molecules (for example Rap2A, Ninj1, Antxr2, Itga1, Itga6, Itga9, ItgaM, Adamtsl4, Adamtsl6), and / or mediators that promote survival (for example Rtn4rl2) and immunomodulation (for example Arg1, Cxcl1, Cxcl2, Cxcl3, Cxcl16, Fcgr1, Fcgr4, Ltb4r1, Jmjd1) and / or from having acquired regenerative properties.

The mononuclear phagocytes or the population of isolated mononuclear phagocytes of the invention, preferably said mononuclear phagocytes being macrophages, are preferably characterized by the expression of at least one of the genes related wound healing (for example Adm, Bnip3, Pdgfb, Vegfa) and/or angiogenesis (for example Vegfa, Angptl4, Cxcl8, Lep, Rora, Apln) and/or detoxification and regulation of defence response (for example Ndrg1, Mt1e, Mt1f, Mt1g, Mt1h, Mt1x, Mt2a, Mt3, Ddit4, Nupr1) and/or response to hypoxia (for example Hk2, Pfkfb3, Slc2a1, Slc2a3, Cxcr4, Plin2, Adm, Bnip3, Lep, Rora, Ndrg1, Egln3, Mt3, Plod2, Hilpda, Angptl4) and/or extracellular matrix remodelling (for example Mmp9, Vcan, Fgf11, Cxcl8, Lep, Pdgfb, Plod2, Vegfa, Angptl4, Sulf2, Egln3), and/or neuronal survival and myelination (for example Mt3, Jam2, Vldlr, Nupr1, Egln3).

The mononuclear phagocytes or the population of isolated mononuclear phagocytes, preferably macrophages, according to the invention or as described herein are preferably for medical use, more preferably for use in cell therapy and/or regenerative medicine.

The mononuclear phagocytes or the population of isolated mononuclear phagocytes, preferably macrophages, according to the invention or as described herein are preferably for use in tissue or cell repair, in tissue or cell regeneration, in tissue remodeling, in the prevention and/or treatment and/or in the repair and/or in the healing of wounds, tissue loss in wounds, surgical ulcers, diabetic wounds, of conditions of degeneration, including neurodegeneration, retinal degeneration, degeneration due to genetic diseases (such as ALS), autoimmune diseases (such as arthritis, collagenopathies) or even diabetes in which degeneration of pancreatic islets occurs; in the prevention and/or treatment and/or resolution of inflammation, of inflammation of the tissues, of damages, damaged tissues and the like.

The mononuclear phagocytes or the population of isolated mononuclear phagocytes, preferably macrophages, according to the invention or as described herein are preferably for use in the prevention and/or treatment of lesions characterized by loss of central nervous system embedded tissue.

The mononuclear phagocytes or the population of isolated mononuclear phagocytes, preferably macrophages, according to the invention or as described herein are preferably for use in the prevention and/or treatment of a spinal lesion or injury, such as a severe spinal injury, or spinal cord injury, preferably a severe or contusive spinal cord injury.

The phagocytes or the population of isolated mononuclear phagocytes, preferably macrophages, are preferably administered from 2 days to 60 days or to 1 year after the lesion (or injury), preferably 4-21 or 15-60 days after the lesion (or injury).

A further object of the invention is a pharmaceutical composition comprising at least one mononuclear phagocyte or the population of isolated mononuclear phagocytes, preferably macrophages, according to the invention and at least one pharmaceutically acceptable excipient. The method according to the invention can further comprise verifying that the mononuclear phagocyte culture has undergone the phenotypic and/or functional change after the removal of the culture medium. Such verification can be performed by quantification of the expression of the genes characterizing the phenotype and in a set of functional tests which predict increased survival, neuronal differentiation, and the ability to reshape the extracellular matrix in human neuron cultures derived from iPSCs.

As used herein, the term "macrophage" has its general meaning in the art and refers to a type of antigen-presenting cell of the mammalian immune system that have phagocytic activities. These cells are characterized by their distinctive morphology and high levels of surface MHC-class II, CD68, CD11b expression. A macrophage is a monocyte-derived phagocyte which is not a dendritic cell or a cell that derives from tissue macrophages by local proliferation. In the body these cells are tissue specific and refer to e. g. Kupffer cells in the liver, alveolar macrophages in the lung, microglia cells in the brain, osteoclasts in the bone etc. The skilled person is aware how to identify macrophage cells, how to isolate macrophage cells from the body of a human or animal, and how to characterize macrophage cells with respect to their subclass and subpopulation.

Macrophages have historically been divided into two phenotypically diverse populations, i.e. a Ml polarized or "classically activated" population, and a macrophage M2 polarized or "alternatively activated" population.

Macrophages exhibiting a Ml phenotype are pro-inflammatory, and are capable of either direct (pathogen pattern recognition receptors) or indirect (Fc receptors, complement receptors) recognition of pathogens and tumor antigens (i.e. they exhibit anti -tumor activity). Ml macrophages produce reactive oxygen species and secrete pro-inflammatory cytokines and chemokines, such as, for example, but without limitation, TNFα, IL-1, IL-6, IL-15, IL-18, IL- 23, and iNOS. Ml macrophages also express high levels of MHC, costimulatory molecules, and FCyR. The Ml phenotype is triggered by GM-CSF and further stimulated by interferon-y (IFN-y), bacterial lipopolysaccharide (LPS), or tumor necrosis factor a (TNFα), and is mediated by several signal transduction pathways involving signal transducer and activator of transcription (STAT), nuclear factor kappa-light-chain-enhancer of activated B cells (NFKB), and mitogen-activated protein kinases (MAPK). These events enhance the production of agents such as the reactive oxygen species and nitric oxide and promote subsequent inflammatory immune responses by increasing antigen presentation capacity and inducing the Thl immunity through the production of cytokines such as IL-12.

In contrast, macrophages exhibiting a M2 phenotype are often characterized as being anti-inflammatory and immunosuppressive as they suppress T-cell responses and are involved in the Th2-type immune response. The M2 macrophage phenotype facilitates tissue repair, wound healing, and is profibrotic. M2 macrophages are characterized by high surface expression of I1-4R, FcsR, Dectin-1, CD 136, CD206, and CD209A. M2 macrophages include IL-4/IL-13-stimulated macrophages, IL-10-induced macrophages, and immune complex-triggered macrophages.

The method of the present invention is thus suitable for inducing one or more phenotypic changes in a population of macrophages to adjust a population of macrophages to have a desired phenotype. As used herein, the phrase "phenotypic and/or functional change" encompasses an observable or detectable change in a feature, property, attribute, or function of the mononuclear phagocytes or macrophages in the population. For example, phenotypic features/properties/functions of macrophages which can be modified or modulated according to the methods of the present invention include, without limitation, pro-inflammatory activity, anti-inflammatory activity, immunogenic activity, phagocytic activity, tolerogenic activity, tissue healing activity, migratory activity, angiogenic activity, suppressive activity, antigen presentation activity or phagocytic activity, extracellular matrix remodelling activity, extracellular matrix deposition activity, trophic activity, stem/progenitor cell self-renewal, cell proliferation, cell differentiation, inhibition of cell death, cell survival, cell secretion, growth/trophic factor secretion, axonal regrowth stimulating activity, myelinating stimulating activity, metabolic support activity.

A phenotypic and/or functional change in macrophages can be observed or detected in several manners. For example, a phenotypic and/or functional change can be observed or detected by performing a test, observation, or measurement on the macrophages themselves, or by performing a test, observation, or measurement on other cells, tissues, organs, etc. which can be influenced by the macrophages, or by performing a test, observation, or measurement on a subject containing the phenotypically modified macrophages.

The phenotypic and/or functional change or modulation can be assessed by detecting or measuring, for example, (i) a change in the expression of one or more genes (metalloproteases, including Mmp8, Mmp9, Mmp10, Mmp12, Mmp14, Mmp19, Mmp27); trophic factors (including VEGFs, FGFs, IGFs) and cell contact and adhesion molecules (including Rap2A, Ninj1, Antxr2, Itga1, Itga6, Itga9, ItgaL, ItgaM, Adamtsl4, Adamtsl6) mediators that promote survival (Rtn4rl2), and immunomodulation (Arg1, Cxcl1, Cxcl2, Cxcl3, Cxcl16, Fcgr1, Fcgr4, Ltb4r1, Jmjd6); (ii) the change in secretion of one or more molecules (e.g., cytokines TGFb3, Il16, Cxcl16, Isg15, Cxcl3, Cxcl1), growth factors (VEGFs, FGFs, IGFs) mediators of inflammation (Ptgs2, Ptges, Nos2, Arg1, etc.); (iii) ability to modulate inflammatory phenotypes (type M1) in immunity cells, preferably macrophages, and to induce a repair phenotype (type M2) in these cells; (iv) ability to induce neuronal differentiation of human and/or murine neural stem cells or inducible pluripotent human cells and to increase survival under conditions of nutrient deprivation (oxygen and glucose) and/or (v) ability to induce angiogenesis and (vi) remodel the extracellular matrix and/or (vii) chemotactic response to SDF1.

The phenotypic and/or functional change or modulation can also be assessed by detecting or measuring, for example, a change in the expression of one or more genes related to wound healing (for example Adm, Bnip3, Pdgfb, Vegfa) and/or angiogenesis (for example Vegfa, Angptl4, Cxcl8, Lep, Rora, Apln), and/or detoxification and regulation of defence response (for example Ndrg1, Mt1e, Mt1f, Mt1g, Mt1h, Mt1x, Mt2a, Mt3, Ddit4, Nupr1) and/or response to hypoxia (for example Hk2, Pfkfb3, Slc2a1, Slc2a3, Cxcr4, Plin2, Adm, Bnip3, Lep, Rora, Ndrg1, Egln3, Mt3, Plod2, Hilpda, Angptl4) and/or extracellular matrix remodelling (for example Mmp9, Vcan, Fgf11, Cxcl8, Lep, Pdgfb, Plod2, Vegfa, Angptl4, Sulf2, Egln3), and neuronal survival and myelination (Mt3, Jam2, Vldlr, Nuprl, Egln3).

As used herein, the terms CXCR4, CYTIP, SLC2A3, MT2A, PLXNA2, HSPH1, CYCS and TIGAR have their general meaning in the art. An exemplary and non-limiting human sequence is represented by the sequences disclosed with NCBI Accession NOs (Gene ID): 7852, 9595, 6515, 4502, 5362, 10808, 54205, 57103 respectively.

As used herein, the herein mention genes have their general meaning in the art. Exemplary and limiting sequences are represented by the sequences disclosed with NCBI Accession NOs (Gene ID) indicated in the Tables.

In the method of the invention any method known to the skilled man for generating/cultivating macrophages may be used, for example isolating monocytes from bone marrow or blood or any derivative (e.g. leukapheresis, buffy coat) and/or differentiating them to macrophages by culturing *in vitro* in the presence of M-CSF or GM-CSF or any biological source of such cytokines, including transfected cell lines.

In a preferred aspect of the invention, the mononuclear phagocytes are isolated from bone marrow. Preferably the mononuclear phagocytes are differentiated to macrophages, e.g. by culturing them in a medium comprising M-CSF, e.g. for 6 days. The macrophages are preferably cultured in CTM under hypoxic conditions, e.g. for 18 hours.

In a preferred aspect of the invention, the mononuclear phagocytes are isolated from peripheral blood and sorted for CD14+ monocytes. The monocytes are preferably cultured with CTM, e.g. 30%, in normoxia, e.g. for 6 days and in hypoxia, for e.g. 18-24h.

Alternatively, the culture with CTM may be carried out firstly under hypoxia conditions and subsequently in normoxia.

In a preferred aspect of the invention, mouse macrophages are obtained from bone marrow cells differentiated for 6 days in a high-glucose Dulbecco modified Eagle medium (D-MEM) or Iscove's Modified Dulbecco's Medium (IMDM) containing 10% heat-inactivated fetal bovine serum, 100 U/mL penicillin/streptomycin, 2 mM L-glutamine and 100 ng/ml M-CSF. To generate the regenerative bioreactor and "educate" the macrophages with tumor conditioning, the differentiated cells are preferably cultured in a medium containing CTM and D-MEM, preferably in 1:1 ratio, and cultivated under hypoxic conditions (e.g. .5-20% O2, preferably 1% O2) for 6-96 hours, preferably 18 hours. The human macrophages are preferably derived from circulating monocytes. The (circulating) monocytes are preferably isolated from peripheral blood by repeated gradient centrifugation (e.g. Lympholyte, Cederlane, #CL5020, and Percoll, Cytiva, #17089101) and/or immune-magnetic enrichment of CD14+ monocyte (e.g. Milteniy Biotec). The monocytes obtained are preferably cultured in vitro for 6 hours-15 days, preferably 7 days in a RPMI 1640 medium containing 10% heat-inactivated fetal bovine serum, 100 U/mL penicillin/streptomycin, 2 mM L-glutamine and 100 ng/ml M-CSF with 20 - 50% CTM, preferably 30%. The monocyte culture may be incubated in hypoxic conditions (e.g. 0.5-20% O2, preferably 1% O2) for the entire culture period 7 days or for the last 6-96 hours, preferably the last 18 hours. CTM is preferably produced from tumor cell lines or tumor explants, or solid tumor, or from resections of dissociated and plated *in vitro* tumors for a period of about 6 hours - 20 days, preferably 12-72 hours.

The cells of the invention are preferably derived by monocytes, preferably from blood-derived CD14+ monocytes. In an embodiment the cells of the invention are obtained by *in vitro* culturing blood-derived CD14+ monocytes with culture medium comprising a tumor conditioned media, e.g. with 70% culture medium (preferably RPMI + 10%FBS+ 100ng/ml M-CSF) and 30% tumor conditioned media. Preferably the culturing is for 1-15 days, preferably for 6 days, in normoxia, followed by 6 hours-5 days, preferably 18-24h of hypoxia. Tumor conditioned media is preferably obtained from the supernatant of tumor lines or of resections of dissociated and plated *in vitro* tumors, preferably for a period of about 6 hours-20 days, preferably 12-72 hours.

The cells of the invention are preferably derived from fresh monocyte, e.g. isolated from bone barrow, preferably from dT-Tomato male mouse bone marrow. In an embodiment the cells of the invention are obtained by culture in cell culture medium (e.g. IMDM) preferably supplemented 10% heat-inactivated fetal bovine serum, 100 U/mL penicillin/streptomycin, 2 mM L-glutamine and murine Macrophage Colony-Stimulating Factor (M-CSF), preferably in adhesion for 1-15 days, preferably for 6 days, in normoxia condition, preferably 37°C, 5% CO₂. Then the medium is preferably modified with a medium consisting of 50% tumor supernatant (e.g. obtained from cultures of solid fibrosarcoma and glioma explants, from which the tumor-rich supernatant was selected) and 50% IMDM and the culture is maintained under hypoxic conditions (1% O2) for 6-72 hours, preferably 18 hours.

In the present method, the hypoxic conditions may be administered for all the incubation with CTM or for a part of the incubation (e.g. 6-96 hours, preferably 18 hours).

Preferably the method of the invention comprises a further step (which may be carried out concomitantly or before or after the incubation with the a culture medium comprising factors released from tumor cultures or explant) wherein the monocytes are induced to differentiate towards macrophages, e.g. by culturing them with M-CSF.

The invention provides an *in vitro* or *ex vivo* method for inducing a phenotypic and/or functional change in a population of macrophages deriving from monocytes, preferably CD14+, isolated from a biological sample, such as blood sample, comprising the incubation of said population, in a culture medium comprising factors released from tumor cultures or explants, wherein the incubation takes place under hypoxic conditions, and where said incubation induces a phenotypic and/or functional change in the mononuclear phagocytes of the population.

The mononuclear phagocytes induced with a phenotypic and/or functional change are preferably macrophages.

The macrophages or cells obtainable by the method of the present invention or the macrophages or cells of the present invention are preferably characterized by the expression of one or more genes indicated in Tables 1, 2, 3, 5 and/or 6 in fact, the tables indicate genes which are up-regulated in THEM cells.

The macrophages or the cells obtainable by the method of the present invention or the macrophages (or the cells) of the present invention are preferably characterized by the low/no expression of one or more genes indicated in Table 4, which reports genes, which are not or very low expressed in THEM cells and/or of one or more of: ALOX15, CCL8, and CCL26.

The macrophages (or the cells) obtainable by the method of the present invention or the macrophages (or the cells) of the present invention are preferably characterized by the secretion of one or more molecules indicated in Table 2.

The methods for observing, detecting and measuring these phenotypic and/or functional changes are known in the art and described herein.

For example, gene expression profiles can be evaluated at the RNA level using microarray analysis of cDNA or oligonucleotides, Northern blot, RT-PCR, sequencing, etc.

The protein expression can be measured using, for example, immunoblotting, immunohistochemistry, protein microarrays, etc.

Various tests based on cells and animal models readily known and used in the art can also be used. The cells of the composition of the invention can come from the patient himself (the composition therefore contains autologous cells) or from a donor (the composition thus contains allogeneic cells).

For the allogeneic cells, HLA compatibility and matching between the donor and the patient receiving the cells is required.

In the context of the present invention "tumor supernatant" preferably indicates the medium in which tumor derived cells are cultured in vitro, and includes the term "tumor-rich supernatant", "medium conditioned by tumor", "factors released from tumor cultures or explants". The tumor supernatant is preferably obtained from cultures of solid tumors, such as fibrosarcoma, or tumor explants, such as glioma, or of tumor cell lines or tumor resections preferably dissociated and in vitro cultured. The culture is preferably carried out for 6h-20 days, preferably for 12-72 hours.

In the context of the present invention "normoxia" preferably indicates the level of oxygen (O2) in air at normal atmospheric pressure (21%, 160 mmHg), e.g. it indicates the presence of 5%CO2, preferably at a temperature of 37°C.

In the context of the present invention "hypoxia" or "hypoxic conditions" preferably indicates the level of oxygen (O2) in air below the normal atmospheric pressure (21%, 160 mmHg), e.g. it indicates the presence of 0.5-20% O2, preferably 1% O2.

In the context of the present invention M2 may be defined as alternatively activated macrophages, polarized mouse macrophages expressing high level of CD206, CD163, and Arg1 genes as previously described or human macrophages expressing high level of CD206, CD163, CD86, TREM2, IL1RN, IL10, STAT3, STAT6, fabp4, sphk1, HOMOX1, IRF4, PPAR-CCL22 and very low/no low level of CCR7, IL2RA, and CXCL11 as previously described (11, 12).

In the context of the present invention TCM may be defined as mouse or human macrophages incubated with CTM (without hypoxia), expressing high level of TGFBI, DAB2, FUCA1, CYP1B1, MAFB, CCL2 and very low/no level of MT2A and MTFP1.

In the context of the present invention M0 may be defined as differentiated not polarized macrophages, expressing high level of NINJ1, F13A1, SCARB1, and STAB1, AOAH, TLR7, A2M, FNBP1, CD209, SH3KBP1, and ITSN1 as previously described (13).

In the context of the present invention THEM may be defined as the cells obtained by the method of the invention or the cells characterized by at least one of the phenotype or function or phenotypic and/or functional change herein disclosed for the cells of the invention.

In a preferred aspect of the invention the method further comprises before the incubation under hypoxic conditions, the incubation of the population in a culture medium comprising factors released from tumor cultures or explants. This means that e.g. the cells or population of cells are incubated with a culture medium comprising factors released from tumor cultures or explants in normoxia for a period of time, e.g. 1-15 days, preferably 6 days. The cells or population of cells are subsequently maintained or cultivated under hypoxic conditions, e.g. for 6 hours-15 days, preferably 18-96 hours.

The culture medium preferably comprises a tumor supernatant, said supernatant being preferably obtained from cultures of tumor solid explants or of tumor cell lines, preferably from fibrosarcoma or glioma, or preferably being a medium conditioned from the tumor (CTM), preferably produced from tumor cell lines, or tumor explants, or solid tumor, or from resections of dissociated and plated in vitro tumors, preferably for a period of about 6 hours-20 days, more preferably of about 12 -72 hours.

In the context of the present invention the term "incubated with" preferably means that the cells are cultivated in, or grown in or treated with. As used herein, the term "therapeutically effective amount" refers to an amount effective, at dosages and for periods of time necessary, to achieve a desired therapeutic result. A therapeutically effective amount of the agent of the present invention may vary according to factors such as the disease state, age, sex, and weight of the individual, and the ability of the agent of the present invention to elicit a desired response in the individual. A The efficient dosages and dosage regimens for the agent of the present invention depend on the disease or condition to be treated and may be determined by the persons skilled in the art. A physician having ordinary skill in the art may readily determine and prescribe the effective amount of the pharmaceutical composition required. For example, the physician could start doses of agent of the present invention employed in the pharmaceutical composition at levels lower than that required achieving the desired therapeutic effect and gradually increasing the dosage until the desired effect is achieved. In general, a suitable dose of a composition of the present invention will be that amount of the compound, which is the lowest dose effective to produce a therapeutic effect according to a particular dosage regimen. Such an effective dose will generally depend upon the factors described above. In a particularly preferred embodiment, the cells of the invention are expanded in a particular growth medium specifically appropriate for the method of the invention, optionally with supplements. Such a growth medium may comprise hematopoietic growth factor that regulates the proliferation, differentiation, and/or functional activation of monocytes, such as M-CSF.

The method of the invention yields cells with particular properties, which are also described herein. Thus, it is particularly preferred that the cell obtainable by the method of the invention is a cell as described herein.

The present invention relates also to a macrophage. The macrophage of the invention can also be referred to as the "cell of the invention", or simply as the "cell". The cell or macrophage of the invention is preferably characterized in that it (a) expresses at least one of the following genes: CXCR4, CYTIP, SLC2A3, MT2A genes and/or (b) does not express at least one of the following genes: PLXNA2, HSPH1, CYCS, TIGAR. Preferably, the cell is an isolated cell. More preferably the cell is derived from CD14+ monocytes, preferably derived from blood. Preferably, the cell is a primate cell. Most preferably, the cell is a human cell.

In the case of a human cell it is preferred that the human cell expresses at least one, preferably at least any combination of two, and most preferably all of the genes disclosed in Table 3 and/or 6. Further, in the case of a human cell, it is preferred that the cell does not display or express at least one, preferably at least any combination of two, and most preferably all of the genes disclosed in Table 4.

In a preferred embodiment, the cell is a macrophage.

The cell according to the invention is a cell obtainable by the method according to the invention.

The invention also relates to a population of cells. The population comprises at least one cell according to the invention. More preferably the cell population comprises a multitude of cells, of which at least 70% (by cell number), preferably 80 % (by cell number), preferably at least 90 % (by cell number), more preferably at least 95 %, such as at least 96 %, at least 97 %, at least 98 %, at least 99 % (each by cell number) are cells according to the invention. All preferred embodiments of the second aspect apply also to the third aspect of the invention. Preferably at least 70 %, preferably at least 80 %, more preferably at least 90 %, such as at least 95 %, at least 96 %, at least 97 %, at least 98 % or at least 99 %, most preferably 100 %, of the total number of cells are cells as defined herein, and optionally one or more its preferred embodiments, alone or in combination. The cell population is preferably positive for expression of CXCR4 and/or CYTIP and/or SLC2A3 and/or MT2A genes and/or negative for expression of PLXNA2 and/or HSPH1 and/or CYCS and/or TIGAR. The cell population is preferably positive for expression of at least one of the genes of Table 1 and/or 2 and/or 3 and/or 4 and/or 6. The cell population is preferably negative for expression of at least one of the genes of Table 4.

Preferably, the population is an isolated population of cells.

The term "allogeneic" is used herein to describe anything that is derived from a different individual of the same species. Two or more individuals are said to be allogeneic to one another when the genes at one or more loci are not identical.

The term "autologous" is used herein to describe anything that is derived from the same subject. As an example, "autologous cell" refers to a cell derived from the same subject. Transplantation of autologous cells is sometimes considered advantageous because it overcomes the immunological barrier which otherwise results in rejection.

The terms "cell population" and "population of cells" interchangeably refer to an ensemble of a plurality of cells. In the narrowest embodiment, a cell population comprises two cells, but more typically a multitude of cells.

The terms "expand" or "expanding", as used herein, generally refer to the proliferation of a cell or of a population of cells. Typically, expansion within the context of the present invention occurs in vitro or ex vivo. Typically, expansion is an activity or process that is subsequent to the isolation of cells. Typically, during expansion the total number of cells increases. It may include also the term "culturing".

The terms "express", "expressed", and "expression", "gene expression" and the like, as used herein, relate to the use of information from a gene in the synthesis of a functional gene product. Gene expression comprises at least the transcription, and optionally comprises one of more additional features, optionally selected from the open list comprising RNA editing, translation and post-translational modification. When gene expression is determined, the presence of an expression product, such as non-edited or edited RNA, or even the encoded protein, is determined. The above terms, used in connection with a particular gene or locus, intend to specify the expression of the genetic information from that gene or locus; for example, when it is said that CXCR4 is expressed, it is meant to say that the CXCR4 gene is expressed.

For low level of expression it is preferably intended gene expression level, which is at least five fold lower than the level of expression of the housekeeping gene, for example beta-actin.

The term "does not express" also includes the term express low/no level or may mean that a particular gene is downregulated e.g. compared to M2 cells.

**In** a certain aspect the term "expresses low/no level" may have the same meaning of "does not express".

The term "express" also means that a particular gene is upregulated e.g. compared to M2 cells.

By "isolated" is meant material that is substantially or essentially free from components that normally accompany it in its native state. For example, an "isolated cell" , as used herein, refers to a cell, which has been purified from the cellular and extracellular environment, such as tissue or fluid, which surround it in a naturally-occurring state. In an alternative description, an "isolated cell" or and the like, as used herein, refer to in vitro isolation and/or purification of a cell from its natural cellular environment, and from association with other components of the tissue in which the cell normally resides. Unless the context dictates otherwise, an "isolated cell" need not necessarily be single cell devoid of any other cells; in contrast, even a population of two or more cells, such as a multitude of cells, can be referred to as "isolated" when said population of cells is isolated in the sense that it is substantially or essentially free from components that normally accompany such population of cells in its native state. In accordance with this definition of the word "isolated", "to isolate", as used herein, is the verb that describes the activity to obtain "isolated" material, such as e.g. an isolated cell.

As used herein, the term "medium", is meant to refer to an aqueous mixture suitable for cultivation of cells, particularly mammalian cells. The aqueous mixture is typically a solution, although suspensions and colloidal mixtures are also comprised. The medium is typically liquid, although some media can also be temporarily frozen, e.g. for storage purposes; in any case, a medium is used for cell culture when it is liquid. A medium may either be a "basal medium", which is a defined aqueous mixture, or a "growth medium", which is an aqueous mixture comprising a basal medium and at least one supplement, and which usually has the capacity to sustain viability of cells ex vivo, and optionally expansion of cells, also ex vivo. A basal medium is preferably a chemically defined medium. A growth medium preferably comprises a basal medium (typically 85 % to 99.5 % vol./vol.) plus at least one human-derived, animal-derived or plant-derived supplement, such as serum, cell lysate.

The terms "secrete" or release", when used herein in connection with a cell, generally refer to any material that is externalized from the cell into the external environment. Said material has typically been produced and/or modified by the cell, although this is not a requirement. For example, some cells secrete proteins and/or regulatory molecules such as hormones, prostaglandins and neurotransmitters, and/or microvescicles and/or exososomes, into their respective external environment. For example, a prostaglandin produced by a cell may be externalized into the cell's environment. Said terms, as used herein, are neither limited to in vivo secretion /in vivo environment nor to in vitro secretion in vitro environment, unless the context dictates otherwise.

Additional steps are optionally but preferably comprised, in particular an expansion step, which is described in detail below.

The first step of the method according to the method of the invention is step (a) characterized by isolating (a population of) mononuclear phagocytes from biological samples.

Thus, according to the present invention, the cells are preferably isolated from blood or bone marrow.

The growth or culture medium according to the present invention will typically comprise a basal medium and one or more supplements. In a broad sense, the type of basal medium used is not particularly limited.

Preferably, the basal medium is chemically defined. A chemically defined basal medium is a basal medium suitable, for the in vitro cell culture of human or animal cells in which all of the chemical components are known, in particular, chemically defined media require that all of the components must be identified and have their exact concentrations known. Therefore, a chemically defined medium must be entirely free of animal-derived components and cannot contain serum or serum-derived proteins, such as fetal bovine serum, bovine serum albumin or human serum albumin. A chemically defined basal medium does not comprise with only recombinant proteins and/or hormones To achieve this, chemically defined media do not comprise components from human or animal sources, such as proteins and/or growth factors; particularly, it does not comprise albumin from human or animal sources, nor growth factors from human or animal sources. Preferably it comprises recombinant albumin and/or recombinant growth factors, usually derived from non-human, non-animal sources such as plant cells and bacteria, and/or synthetic chemicals such as e.g. polyvinyl alcohol.

Basal media can e.g. be selected from the list comprising but not limited to Eagle's minimal essential medium (EMEM), Dulbecco's modified Eagle's medium (DMEM), alpha-MEM, RPMI-1640, IMDM, and others. If desired, one or more serum-substituting ingredients are added to these media. Thereby, serum-free basal media are obtained.

The cells can be frozen, e.g. snap-frozen in liquid nitrogen, according to standard procedures. It is well possible to de-frost such frozen cells and to e.g. subject them to further culture passages after de-frosting. The freezing can occur either as entire batch or in aliquots. In one embodiment, the cell is characterized by at least one feature-structural or functional-that is not normally found in a macrophage cell. For example, such a feature may be a feature specific for the method by which the cell of the invention is obtainable. Such structural or functional feature may be selected from the features explicitly described in this disclosure, and/or from features not explicitly described in this disclosure, but inherent to the cell of the invention, e.g. inherent to the cell obtainable by the method of the invention. A cell according to the present invention can be expanded in vitro by culture. Thereby a derived cell can be obtained. The derived cell is also a cell of the invention, as long as it complies with the claimed characteristics defining the cell of the invention. Thus, due to the properties of the cell of the invention, the present invention can cover multiple generations of cells. Multiple generations are obtainable by expansion as described herein. Indeed, a specific embodiment of the cell according to the present invention comprises expansion of the cell ex vivo. Thus, although the cell is isolated, it is capable to give offspring to same or similar cells, and thus to generate a cell population.

In general, a cell of the invention can originate from various tissues, such as bone marrow, and fluids such as blood. Preferably, the cell according to the present invention originates from or is derived from the bone marrow or blood.

In one embodiment, the cell of the invention is a freshly isolated cell. A freshly isolated cell is a cell that has been directly obtained by isolation from the tissue it originates from, such as by the method described herein. More preferably, however, the cell of the invention is a cell that is derived from a freshly isolated cell, most typically by expansion or cultivation in vitro.

The cell according to the present invention may be characterized by a gene expression pattern. As used herein, "gene expression pattern" means that at least one gene is expressed, or not expressed, or expressed at a certain level, as the case may be. Thus, the fact that this particular gene is expressed, or not expressed, or expressed at a certain level, characterizes the cell according to the invention, e.g. for the purpose of distinction from a reference cell. The same applies for the expression of more than one gene, such as e.g. two genes, three genes, four genes, five genes, six genes, seven genes, eight genes, nine genes, ten genes, etc. When the cell of the invention is characterized by the expression of one or more genes, it is not desired to make a specific statement on the expression of any other gene not mentioned in that context. As an illustrative example, wherever it is said herein that the cell of the invention expresses CXCR4, this should not be taken as a statement that any specific other gene is also expressed, or not expressed, or expressed at a certain level, unless the context dictates otherwise.

In general, the expression of a gene can be tested by various methods, both on nucleic acid level and on protein level. Gene expression can be relatively commonly tested in molecular biology, biochemistry and cell biology, and any method known or suitable therefor may be used in the context of the present invention. Some methods are described below. Methods relating to gene expression can also be referred to as transcriptomic methods. Hence, the gene expression profile of a cell can also be referred to as transcriptome. In one embodiment, the cell of the invention is characterized by its gene expression profile. Different terms can be used to describe that the expression of a gene is being analyzed: for example, the expression can be said to be tested, analyzed, assessed, assayed, measured, determined etc. These terms include both the qualitative determination, i.e. the question whether the respective gene is expressed or not, and the quantitative determination, i.e. the question at what the level the respective gene is expressed. Typically, for determination of gene expression, a sample of one cell or a multitude of cells (which is preferred) is taken from a cell population, and the gene expression is subsequently analyzed in that sample, whereby the cells in that sample may be destroyed or not, as the case may be. Since the cell population according to the present invention is normally homogeneous, meaning that substantially all individual cells comprised therein are much alike or similar, the finding on gene expression in the cells comprised in the sample taken from the cell population (representative sample) will usually also apply to the remaining cells in that population, unless the context dictates otherwise and/or the cell population does not appear to be substantially homogeneous.

Determination of the expression of any respective gene may either be an undirected determination, i.e. where gene expression is assayed in general (e.g. by a broad approach such as microarray) without focusing on one gene, or a directed determination, where it is specifically tested whether the respective gene is expressed. In the second case particularly, the respective gene may also be said to be a "gene of interest". Several transcriptomic technologies can be used to generate the data for gene expression analysis. For example, DNA microarrays measure the relative activity of previously identified target genes. Sequence based techniques, like RNA-Seq, provide information on the sequences of genes in addition to their expression level.

The cell(s) of the invention may express one or more markers or genes and they may lack expression of one or more markers or genes. In a specific case, they express at least one marker or gene selected from the Table 1 and/or 2 and/or 3 and/or 5 and/or 6 and a combination thereof; and/or they may lack expression of at least one marker or gene selected from the Table 4 and a combination thereof.

The NCBI or Ensembl references provided herein or in the Tables are exemplary and not limiting references to their sequences. Indeed, each genes/marker/protein herein indicated may be characterized by its Accession numbers (i.e. the Ensembl or NCBI gene ID) but also includes in all isoforms, variants, analogues, orthologues, fragments, derivatives

In the context of the present disclosure, reference to the herein disclosed genes is a reference to all forms of these molecules and to functional fragments, mutants or variants thereof. In addition, reference to any isoform that may arise from alternative splicing of their mRNA or isomeric or polymorphic forms of these molecules.

Reference to "phenotypic profile" or "phenotype" should be understood as a reference to the presence or absence of the transcription of the genes encoding the subject markers and/or the cell surface expression of the expression product translated therefrom. It should be appreciated that although most cells falling within the scope of the claimed cell populations will be characterized by the presence or absence of the subject marker as a cell surface anchored expression product, some cells falling within the defined populations may initially exhibit changes only at the transcriptome level, such as when the transcription of a given marker has been upregulated but may not yet have resulted in a cell surface anchored expression product. In general, cells which progress to a new differentiative stage will transiently exhibit gene expression changes which are not yet evident in the context of changes to levels of an expression product. However, these cells nevertheless fall within the scope of the claimed cellular populations.

A "conditioned medium" is e.g. a medium in which a specific cell or population of cells has been cultured, and then removed. When cells are cultured in a medium, they may secrete cellular factors that can provide trophic support to other cells. A trophic factor is a substance that promotes or at least supports, survival, growth, proliferation and/or maturation of a cell, or stimulates increased activity of a cell. Such trophic factors include, but are not limited to hormones, cytokines, extracellular matrix (ECM), proteins, vesicles, antibodies, and granules. The medium containing the cellular factors is the conditioned medium. In some embodiments of the current compositions or methods, a trophic factor is used.

It is contemplated that any of the compositions or methods of the current disclosure comprise cell encapsulation. In some embodiments, cells are individually encapsulated. In some embodiments, many cells are encapsulated within the same membrane. In some embodiments in which the cells are to be removed following implantation, a relatively large size structure encapsulating many cells, such as within a single membrane, may be employed to provide a convenient means for retrieval. A wide variety of materials may be used in various embodiments for microencapsulation of stem cells. Such materials include, for example, polymer capsules, alginate-poly-F-lysine-alginate microcapsules, barium poly-F-lysine alginate capsules, barium alginate capsules, polyacrylonitrile/polyvinylchloride (PAN/PVC) hollow fibers, and polyethersulfone (PES) hollow fibers. Techniques for microencapsulation of cells that may be used for administration of stem cells are known to those of skill in the art and are described, for example, in Chang, P., et al., 1999; Matthew, H. W., et al., 1991; Yanagi, K., et al., 1989; Cai Z. H., et al., 1988; Chang, T. M., 1992 and in U.S. Pat. No. 5,639,275 (which, for example, describes a biocompatible capsule for long-term maintenance of cells that stably express biologically active molecules. Additional methods of encapsulation are in European Patent Publication No. 301,777 and U.S. Pat. Nos. 4,353,888; 4,744,933; 4,749,620; 4,814,274; 5,084,350; 5,089,272; 5,578,442; 5,639,275; and 5,676,943. Certain embodiments of the current disclosure incorporate cells of the invention into a polymer, such as a biopolymer or synthetic polymer. Examples of biopolymers include, but are not limited to, fibronectin, fibrin, fibrinogen, thrombin, collagen, and proteoglycans. Other factors, such as the cytokines, can also be incorporated into the polymer. In other embodiments of the disclosure, cells may be incorporated in the interstices of a three-dimensional gel. A large polymer or gel, typically, will be surgically implanted. A polymer or gel that can be formulated in small enough particles or fibers can be administered by other common, more convenient, non- surgical routes. In some embodiments of the current disclosure, standard growth conditions are utilized. As used herein, the term "standard growth conditions" refers to culturing of cells e.g. at 37°C., in a standard atmosphere comprising 5% CO₂. Relative humidity is maintained e.g. at about 100%. While foregoing the conditions are useful for culturing, it is to be understood that such conditions are capable of being varied by the skilled artisan who will appreciate the options available in the art for culturing cells, for example, varying the temperature, CO2, relative humidity, oxygen, growth medium, and the like.

Embodiments include pharmaceutical, therapeutic compositions, formulations, preparations and related methods for use in regenerative medicine comprising the cells of the invention or products derived thereof.

Compositions take the form of a solution, suspension, a film for example, and contain about 10% to about 95% or about 25% to about 70% of cells or products derived thereof. In embodiments, the compositions are administered in any of a number of suitable ways. Compositions may be administered systemically by means such as intravenous and intraarterial, locally, by means such as intrathecally, intraventricularly, or by means of an Ommaya reservoir. Other means including transdermally, rectally, vaginally, sublingually, and intranasally.

Compositions are administered in a manner compatible and in such amount as will be therapeutically effective, tolerable and safe. The quantity administered depends on the subject to be treated. Precise amounts of cells required to be administered depend on the judgment of the practitioner.

In many instances, it may be desirable to have multiple administrations of at most about or at least about 3, 4, 5, 6, 7, 8, 9, 10, or more (or any range derivable therein). The administration may range from one day to multiple week interval of 1, 2, 3, 4, 5, 6, 7, 8, 9, 10 or more weeks (or any range derivable therein). The course of administration may be followed by assessment of symptoms, pain, mood, behavior, or catastrophizing for example.

A patient may be administered a composition or a combination of compounds described herein in an amount that is, is at least, or is at most about 0.00001, 0.0001, 0.001, 0.01, 0.1, 0.2, 0.3, 0.4, 0.5, 0.6, 0.7, 0.8, 0.9, 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 110, 120, 130, 140, 150, 160, 170, 180, 190, 200, 210, 220, 230, 240, 250, 260, 270, 280, 290, 300, 310, 320, 330, 340, 350, 360, 370, 380, 390, 400, 410, 420, 430, 440, 441, 450, 460, 470, 480, 490, 500 mg/kg/day (or any range derivable therein).

The cell of the invention is a cell from an animal, preferably mammal, preferably primate, more preferably human. Thus, in one embodiment, the cells of the invention are a mammalian cell. Therefore, unless specified otherwise, all indications to a specific gene mean the respective mammalian gene.

More preferably, the cell of the invention is a cell from a primate, more particularly from a human. Thus, more preferably, the cell of the invention is a human cell. Therefore, in that embodiment, all indications to a specific gene mean the respective human gene.

Preferably, the cell of the invention is not a transgenic cell. Therefore, in that embodiment, all indications concerning expression of a specific gene mean that the respective gene is expressed from the genome, where it is encoded, more specifically, where it is encoded through Mendelian inheritance. However, in some alternative embodiments, the cell of the invention is a transgenic cell.

Homogeneous in this context means that substantially all cells are much alike or similar. The expression of a gene may be tested directly, such as typically by molecular biology methods including the determination of presence and, if necessary or desired, levels of a gene product, such as particular messenger RNA (mRNA) or a precursor or degradation product thereof; or, alternatively, the expression of a gene may be tested indirectly, such as typically by biochemical methods including the determination of presence and, if necessary or desired, levels of a protein encoded by a particular gene, such as a particular cell surface protein or a precursor or degradation product thereof. Suitable surface proteins for the characterization of the cell of the invention include particularly clusters of differentiation (CD). Any known method for testing the expression of a gene may also be used in the present invention. Suitable methods include those selected from the list comprising but not limited to gene expression profiling, polymerase chain reaction, such PCR, such as preferably quantitative PCR (qPCR), including RT-qPCR, sequencing of transcription products, any type of transcriptome analysis, and others. On nucleic acid level, preferred methods include microarrays and qPCR.

In one embodiment, the gene expression is determined by gene expression profiling. In the field of molecular biology, gene expression profiling refers to the measurement of the expression of several, often thousands, of genes, typically all at once, to create a global picture of cellular gene expression. For example, gene expression profiling is the measurement of the expression of several, typically but not necessarily thousands, of genes at once, to create a global picture of a cell. Gene expression profiling can, for example, distinguish between cells of the invention and other cells. It is even possible to determine expression of an entire genome simultaneously, that is, every gene present in a particular cell. Many experiments of this sort measure an entire genome simultaneously, that is, every gene present in a particular cell. Several transcriptomics technologies can be used to generate the necessary data to analyze. Microarrays are preferred in some embodiments of the present invention

Sequence based techniques, including high-throughput sequencing, like Next Generation Sequencing (NGS), can be used to determine gene expression in the present invention. For example, RNA-Seq (RNA sequencing), also called whole transcriptome shotgun sequencing, can provide information on the sequences of genes in addition to their expression level. RNA-Seq uses next-generation sequencing (NGS) to reveal the presence and quantity of RNA in a biological sample at a given moment in time. In one embodiment, gene expression is determined in a sequence-based technique, like RNA-Seq or PCR and any variation thereof.

The expression of some or all genes, e.g. as revealed by gene expression profiling, such as in microarray, can then be verified by quantitative RT PCR (qRT PCR).

As is commonly known, a quantitative polymerase chain reaction (qPCR, also known as real-time polymerase chain reaction (Real-Time PCR), is a laboratory method based on the polymerase chain reaction (PCR), which is suitable for determining the amount of a nucleic acid. In particular, for determining the amount of a transcript (e.g. mRNA), the method of choice is quantitative reverse transcription polymerase chain reaction (qRT-PCR). Compared to other RNA quantification methods, such as northern blot, qRT-PCR is normally considered to be the most powerful, sensitive, and quantitative assay for the detection of RNA levels, and it is therefore preferred in the present invention, qRTPCR is also much more sensitive, for determination of gene expression, than indirect determination of the gene expression product on protein level, such as immunodetection, e.g by immunodecoration with fluorescent primary or secondary antibodies and flow cytometry. qRT PCR typically requires specific primers for the respective nucleic acid, the sequence of which is, however, normally available through sequence databases, e.g. for human genes, or can be designed by the skilled person based on such information. Commercial kits and machines are available for qRT-PCR, and these can be used in the present invention. The nucleic acid which serves as template (also termed "target") for determination of gene expression in qRT PCR is a transcription product, typically mRNA.

Preferably the cell of the invention expresses at least one gene specific for macrophages. The cell of the invention is characterized inter alia in that it expresses at least one of the following genes: CXCR4, CYTIP, SLC2A3, MT2A. More preferably, said at least one gene is expressed at high levels, compared to the housekeeping gene beta-actin, relative to the expression ratio or said at least one gene vs. beta-actin in reference cells, or upregulated compared to their expression level in M2 macrophages.

In one embodiment, gene expression is determined indirectly, i.e. not by determination of the primary transcription product, the mRNA or its precursor or its degradation product, but by determination of a specific product further downstream. Typically, the specific product further downstream is a protein encoded by the mRNA; however, it may also be any other specific product, such as e.g. a metabolite which is specific to the presence of a particular, e.g. enzymatically active, protein.

Preferably, the presence of a protein is determined. More preferably, the presence of a protein on the cell surface is determined. In other words, it is determined whether a protein is displayed on the cell surface.

Cells displaying a particular protein on the cell surface can be analyzed e.g. by immunologically active molecules, such as specific antibodies and other immunoreactive molecules. "Cell surface" is used herein in accordance with its normal meaning in the art, and thus specifically includes the outside of the cell which is accessible to binding by proteins and other molecules. A protein is displayed on the surface of cell if it is at least partially located at the surface of said cell and is accessible to binding by antigen-binding molecules such as antigen-specific antibodies added to the cell. In one embodiment, a protein displayed on the surface of cell is an integral membrane protein having an extracellular portion that can be recognized by an antibody. The term "extracellular portion" or "exodomain" in the context of the present invention means a part of a molecule, particularly a protein, that faces the extracellular space of a cell and preferably is accessible from the outside of said cell, e.g., by binding molecules such as antibodies located outside the cell. Preferably, the term refers to one or more extracellular loops or domains or a fragment thereof. The term "portion" is used herein and refer to a continuous or discontinuous element of a structure such as an amino acid sequence. A portion or part of a protein sequence preferably comprises at least 5, in particular at least 8, at least 12, at least 15, at least 20, at least 30, at least 50, or at least 100 consecutive and/or non-consecutive amino acids of the amino acid sequence making up the protein.

A protein detectable by an antibody or other immunoreactive molecule may also be referred to as an antigen. In some embodiments, the cell of the invention may be characterized by displaying- or not displaying-one or more specific antigens. In the context of the present invention, such antigen is preferably displayed on the surface of the cell. Such an antigen can also be referred to as "surface antigen".

Examples thereof are provided in Table 5.

Although the word "express", in the strict sense" means that a gene is expressed, the word "express" can also mean to describe that a protein, particularly a cell surface protein, which is encoded by said gene, is displayed on the cell.

According to the invention, an antigen is displayed on a cell if the level of expression is above the detection limit and/or if the level of expression is high enough to allow binding by antigen-specific antibodies added to the cell. According to the invention, an antigen is said to be not expressed on a cell if the level of expression is below the detection limit and/or if the level of expression is too low to allow binding by antigen-specific antibodies added to the cell. Preferably, an antigen expressed in a cell is expressed or exposed, i.e. is present, on the surface of said cell and, thus, available for binding by antigen-specific molecules such as antibodies or other immune reactive molecule added to the cell. In some cases, a secondary molecule that aids in the detection, such as e.g. an optionally labelled secondary antibody, is also added.

An antibody or other immune reactive molecule may recognize an epitope on the cell. The term "epitope" refers to an antigenic determinant in a molecule such as an antigen, i.e., to a part in or fragment of the molecule that is recognized, i.e. bound, by the immune system, for example, that is recognized by an antibody or other immunoreactive molecule. Detection of an epitope specific for any particular antigen normally allows to conclude that that particular antigen is expressed on the cell being analyzed.

In one embodiment, the cell of the invention can be characterized by immunophenotyping. "immunophenotyping" generally means that the cell can be characterized by antigen-specific molecules such as antibodies or other immune reactive molecules, which are added to the cell to determine if an antigen is expressed on a cell. Immunophenotyping includes cell sorting using various methods including flow cytometry.

A preferred method for immunophenotyping is flow cytometry, in particular FACS. an analyte, in particular a cell surface protein, is recognized, normally with an antibody or other immunorective molecule. The antibody or other immunorective molecule is either fluorophore-labelled itself, or recognized by a fluorophore-labelled secondary antibody or other immunorective molecule, which is added for that purpose.

The cell according to the invention is characterized in that it expresses at least one of: CXCR4, CYTIP, SLC2A3, MT2A. Their expression is preferably detected on gene expression level, most preferably by RNAseq. An example thereof is shown in Figure 22.

Without wishing to be bound to any particular theory, it is believed that CXCR4, CYTIP, SLC2A3 or MT2A are important for the cell's chemotactic capacity, regulation of cell adhesion, cell metabolism, and anti-oxidant defence. As shown in the Examples, the cells according to the present invention are distinguished from M2 cells inter alia by the expression of CXCR4, CYTIP, SLC2A3 and/or MT2A. Thus, the CXCR4, CYTIP, SLC2A3 and/or MT2A -expressing cell according to the present invention is clearly different from macrophages described by the prior art.

The cells according to the present invention does, however, express low or no levels of PLXNA2, HSPH1, CYCS and/or TIGAR. These genes are expressed by M2 and/or TCM and/or M0 macrophages. Thus, the cell of the invention is not M2, TCM, M0 macrophages.

Preferably, the cell expresses at least one gene specific for macrophage cells. Preferably, the cell displays at least one surface marker specific for macrophage cells on its surface, e.g. CD45, CD68, CD11b and MHCII.

Preferably, the cell of the invention is capable to promote cell survival, cell growth, including neuronal cell growth, angiogenesis, extracellular matrix remodelling, immune cell modulation. For example, it presents pro-inflammatory activity, anti-inflammatory activity, immunogenic activity, phagocytic activity, tolerogenic activity, tissue healing activity, migratory activity, angiogenic activity, suppressive activity, antigen presentation activity or phagocytic activity, extracellular matrix remodelling activity, extracellular matrix deposition activity, trophic activity, stem/progenitor cell self-renewal, cell proliferation, cell differentiation, inhibition of cell death, cell survival, cell secretion, growth/trophic factor secretion, axonal regrowth stimulating activity and/or metabolic support activity.

The cells of the invention confer preferably present *in vitro* neuronal regenerative function on human motor neurons and/or *in vivo* neuronal regenerative function.

The cell according to the invention can also be characterized by the method by which it is obtainable. This is possible because the cell according to the present invention has properties that are different from cells obtained according to previously described methods (see examples) and thus, the method for obtaining the cell according to the present invention renders a cell with unique properties.

In an aspect, the invention relates to a population of cells (cell population). The cell population comprises at least one cell according to the second aspect of the invention.

The cell population comprises at least one cell which expresses CXCR4, CYTIP, SLC2A3 and/or MT2A. This can normally be confirmed by showing that a sample of the cell population is positive for expression of said gene(s). In other words, the cell population is characterized by expression of the above gene(s) and/or potreins. In one embodiment, the majority of the cells in the cell population express the above gene(s) and/ or proteins. Preferably, at least 80% (by cell number), preferably at least 90% (by cell number), more preferably at least 95%, such as at least 96%, at least 97%, at least 98%, at least 99% (each by cell number) of the cells comprised in the cell population express the above gene(s) and/or proteins.

The cell population is preferably further characterized in that all cells are characterized by essentially uniform physical characteristics. "essentially uniform" means that at least 90% of the cells, such as at least 91% of the cells, such as at least 92% of the cells, such as at least 93% of the cells, such as at least 94% of the cells, such as at least 95% of the cells, such as at least 96% of the cells, such as at least 97% of the cells, such as at least 98% of the cells, and preferably at least 91% of the cells, comply with one or more desired physical characteristic.

Preferably, the macrophages are autologous.

The pharmaceutical composition contains the transformed cells described above as an active ingredient. It also contains a pharmaceutically acceptable excipient.

The term "pharmaceutically acceptable excipient" means an excipient which is useful in preparing a pharmaceutical composition which is generally safe, non-toxic and desirable, and includes excipients which are acceptable for veterinary use as well as human pharmaceutical use.

Such excipients can be solid, liquid, semi-solid or, in the case of an aerosol composition, gaseous. The compositions for tissue or cell regeneration can generally be administered parenterally, topically, intravenously, orally, subcutaneously, intra-arterially, intracranially, intraparenchymally, intraperitoneally, intranasally or intramuscularly. A typical route of administration is intravenous or intraparenchymal, although other routes can be equally effective. For intravenous administration, the composition of the invention will be in liquid form.

It will therefore contain, in addition to the cells, a pharmaceutically acceptable diluent which does not affect the biological activity of the cells of the invention. Examples of such diluents are phosphate-buffered saline, Ringer's solutions, dextrose solution and Hank's solution. Furthermore, the pharmaceutical composition or formulation can also include other non-toxic, non-therapeutic, non-immunogenic vehicles, adjuvants or stabilizers and the like.

**In** a preferred embodiment, the composition of the invention is in a liquid form.

The pharmaceutical compositions of the invention can be administered alone or combined with another pharmaceutical composition or another active ingredient.

**In** particular, the pharmaceutical compositions of the invention can contain the cellular compositions of the invention as well as another active ingredient, combined in the same container. As used herein, the term "combined" does not imply that the cells of the invention and the other active ingredient are necessarily administered simultaneously.

It also extends to any use or presentation which includes their administration at different intervals of time or in separate containers.

"Concomitant administration" of said active ingredient with the pharmaceutical composition of the present invention means administration with the cells of the invention at a time such that both the active ingredient and the composition of the present invention will have a therapeutic effect.

Such concomitant administration can involve the simultaneous (i.e. contemporaneous), prior or subsequent administration of the active ingredient with respect to the administration of the cells of the invention.

A person of ordinary skill in the art would have no difficulty determining the appropriate timing, sequence, and administration dosages for particular drugs and compositions of the present invention.

The in vivo release of the cell composition of the invention, in a subject in need thereof, is herein proposed as a simple and effective manner of regenerating tissues and cells.

Usually, the aforementioned subject is a human, but non-human mammals, e.g., companion animals such as dogs, cats, horses, etc., laboratory mammals such as rabbits, mice, rats, etc. and the like can also be treated.

Therefore, a "subject in need thereof" as understood herein is a mammal, preferably a human, having, for example, a tissue lesion.

The present invention comprises treatment methods in which the cell composition of the invention is administered to said subject in need thereof by injection.

The method of the invention can further comprise collecting and isolating mononuclear phagocytes or the precursors thereof or stem cells from the blood, bone marrow or umbilical cord, or from the subject's reprogrammed pluripotent somatic cells in need thereof or from a healthy donor, prior to incubation of these cells as disclosed above, so as to obtain / isolate a population of phagocytes, preferably monocytes.

Optionally, the method comprises packaging and/or storing the cells thus obtained, and possible administration to the patient.

Effective doses of the therapeutic entity of the present invention vary depending on many different factors, including means of administration, target site, physiological state of the patient, whether the patient is human or animal, other medications administered, and whether the treatment is prophylactic or therapeutic.

The treatment dosages can be titrated to optimize safety and efficacy.

The composition of the invention and/or the formulations thereof can be administered in a variety of manners including, but not limited to, intraspinal, intraencephalic, epidural, intrathecal, intracranial, parenteral, intraperitoneal, intravenous, intradermal, intrastromal, intraarticular, intrasynovial, intralesional, intraarterial, intracardiac, intramuscular, intranasal, cutaneous or subcutaneous, intraorbital, intracapsular, thematic, ophthalmological or ocular, by surgical implant, internal surgical paint, infusion pump or by catheter.

The composition of the present invention can be formulated for administration to an animal, preferably a mammal, including humans, in a variety of manners known in the state of the art. Examples of preparations could include any solid composition (tablets, pills, capsules, sachets, vials, powders, granules, bars, pencils, vaporizers, aerosols, etc.), semi-solid (ointment, cream, conditioner, gel, hydrogel, foam, lotion, soap, gelatin, etc.), or liquid (aqueous or non-aqueous solutions, hydroalcoholic or hydroglycolic solutions, suspensions, emulsions, syrups, anhydrous compositions, aqueous dispersions, oils, milks, conditioners, liniments, serums, etc.) for topical or parental administration, preferably intra-tissue administration. The composition of the present invention can also be in the form of sustained release formulations or any other conventional release system. The term "sustained release" is used in the conventional sense in reference to a cellular delivery compound or system which allows for the gradual release of said cells over a period of time and preferably, although not necessarily, with relatively constant cellular release over a period of time. Illustrative examples of sustained release vehicles or systems include, but are not limited to, liposomes, mixed liposomes, oleosomes, niosomes, ethosomes, millicapsules, microcapsules, nanocapsules, sponges, cyclodextrins, blisters, micelles, mixed surfactant micelles, mixed surfactant phospholipid micelles, millispheres, microspheres, nanospheres, lipospheres, microemulsions, nanoemulsions, miniparticles, milliparticles, microparticles, nanoparticles, solid lipid nanoparticles, nanostructured lipid media, polymeric materials, biodegradable or non-biodegradable patches or implants, or biodegradable microparticles such as biodegradable microparticles.

Each of the documents cited herein (including all patents, patent applications, scientific publications, manufacturer's specifications, instructions, presentations, etc.), whether above or below, are hereby incorporated by reference in their entirety.

As used herein, unless specified otherwise, the terms "about", "ca." and "substantially" all mean approximately or nearly, and in the context of a numerical value or range set forth herein preferably designates +/- 10 %, more preferably +/- 5 %, around the numerical value or range recited or claimed. Terms like "substantially not", "substantially absent" etc., with reference to a characteristic of a cell, mean that the respective characteristic cannot be detected on a cell with analytical methods normally used in the respective area according to the state of the art, and/or that substantially all cells in a population so described do not have the respective characteristic.

Unless expressly specified otherwise, the word "comprise", or variations such as "comprises" or "comprising" is used in the context of the present document to indicate that further members may optionally be present in addition to the members of the list introduced by "comprising". It is, however, contemplated as a specific embodiment of the present invention that the term "comprising" encompasses the possibility of no further members being present, i.e. for the purpose of this embodiment "comprising" is to be understood as having the meaning of "consisting of".

The present invention will now be illustrated by means of non-limiting examples, with reference to the following figures.
**Figure 1****. Weight of the animals at the time of surgery.**
   The graph depicts the weight of the animals considered in the experiments at the time of the first surgical operation (spinal injury) on day 0.
**Figure 2****. Diagram of THEM and M2 macrophage injection sites in the severe spinal injury model.**
   Animals subjected to severe spinal injury received multiple THEM or M2 cell transplantation or saline infusion. The black arrows indicate where the injections were performed. The same points identified in the control animals refer to the saline infusion. The dotted line indicates the separation between the dorsal region (where the injections were performed) and ventral region of the spinal parenchyma.

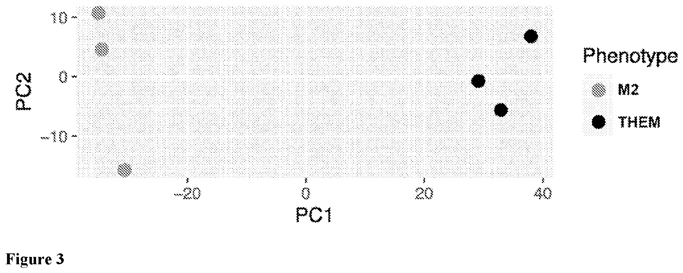

**Figure 3****. Analysis of global transcripts variability by Principal Component Analysis shows a clearly distinct transcriptome in THEM cells with respect to non-therapeutically active M2 cells, associated with the expression at higher levels of genes involved in angiogenesis and extracellular matrix remodeling**
   Principal Component Analysis (PCA) of the sequencing RNA related to the THEM cells (black dots) and M2 macrophages before (gray dots). The analysis shows that the clusters of THEM (black dots) and M2 macrophages (gray dots) are separated from each other, highlighting the difference in phenotype between the cells before transplantation.

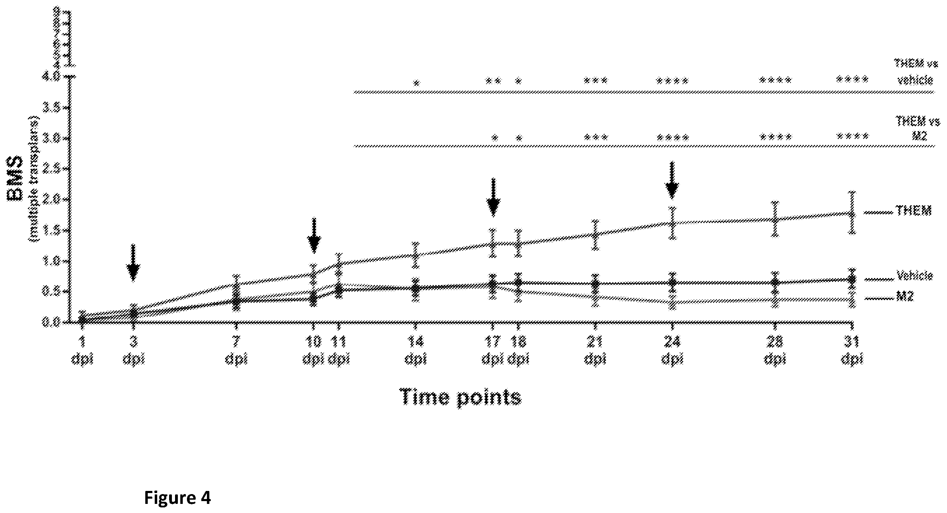

**Figure 4****. Improved locomotor recovery in animals subjected to severe spinal injury following multiple THEM transplantation.**
   The graph depicts the scores of BMS (Basso Mouse Scale), a scale for measuring the locomotor capacities measurable in mice subjected to bone marrow lesions; the scale is validated, stable and reliable [14]. In the graph the BMS of the animals subjected to multiple THEM cell transplantation (dark gray line, n=24 out of 5 experiments), of animals subjected to multiple M2 cell transplantation (light gray line, n=12 out of 4 experiments) and of control animals subjected to sterile saline infusion (black line, n=26 out of 7 experiments). The arrows indicate the time-points at which the transplants were performed. The BMS of the animals subjected to multiple THEM transplantation showed a gradual improvement in locomotor performance, which is significantly higher (1.792 ± 0.327) with respect to that of the animals subjected to multiple M2 transplantation (0.375 ± 0.109) and control animals (0.712 ± 0.157). The differences between the experimental conditions were analyzed with the 2wayANOVA test and post-hoc Sidak *post-test* statistical analysis methods. The data are shown in the form of means ± SEM. *p≤0.05, **p≤0.01, ***p≤0.001, ****p≤0.0001.

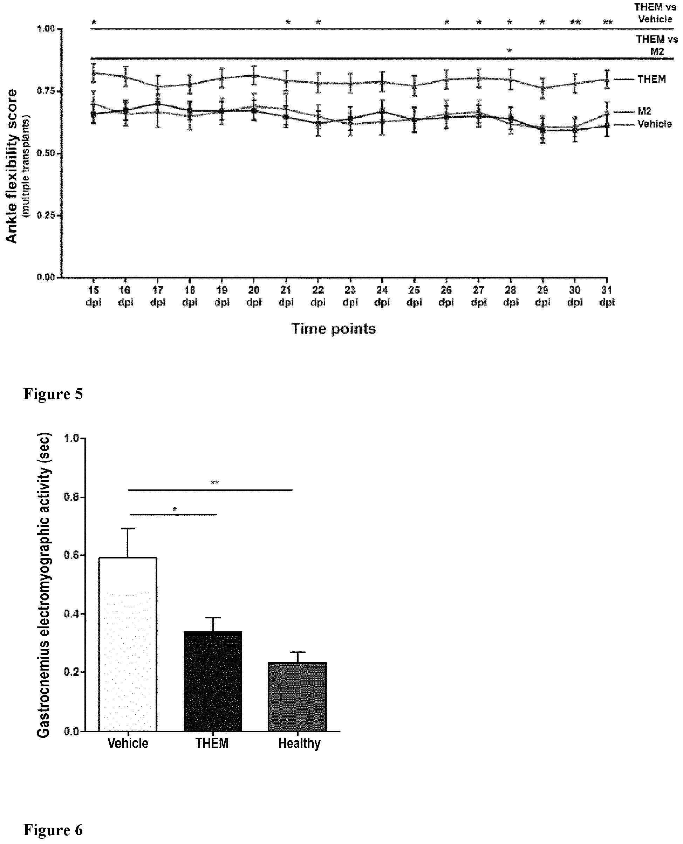

**Figure 5** **Improved flexibility of the ankle joint in animals subjected to severe spinal injury following multiple THEM transplantation.**
   The graph depicts the ankle joint flexibility score of animals subjected to multiple THEM transplantation (dark gray line, n=24 out of a total of 7 experiments), to multiple M2 cell transplantation (light gray line, n=12 out of a total of 3 experiments) and control animals subjected to sterile saline infusion (black line, n=26 out of a total of 7 experiments) from 15 dpi until the end of the experiment. At 31 dpi, the ankle flexibility in the animals subjected to multiple THEM transplantation (0.799 ± 0.037) was significantly greater than in the animals subjected to multiple M2 transplantation (0.659 ± 0.049) and in the control group (0.612 ± 0.045). The differences between the experimental conditions were analyzed with the 2wayANOVA test and post-hoc Tukey post-test statistical analysis methods. The data are shown in the form of means ± SEM. *p≤0.05, **p≤0.01.
**Figure 6****. Multiple THEM transplant animals following severe spinal injury show more physiological muscle activation duration values by electromyography.**
   The graph shows the electromyographic activation durations of the lateral gastrocnemius muscle in the control animals (white), in the animals subjected to multiple THEM transplantation (black) and in the healthy animals (gray). In the animals subjected to multiple THEM transplantation, the duration of electromyographic activation of the lateral gastrocnemius muscle is statistically shorter (0.338s ± 0.048s) with respect to that of the control animals (0.593s ± 0.101s), which is closer to the physiological condition of the muscle in healthy animals (0.231s ±0.038s). The differences between the experimental conditions were analyzed with the ONEwayANOVA test and Tukey post-test statistical analysis methods. The data are shown in the form of means ± SEM. *p≤0.05, **p≤0.01.

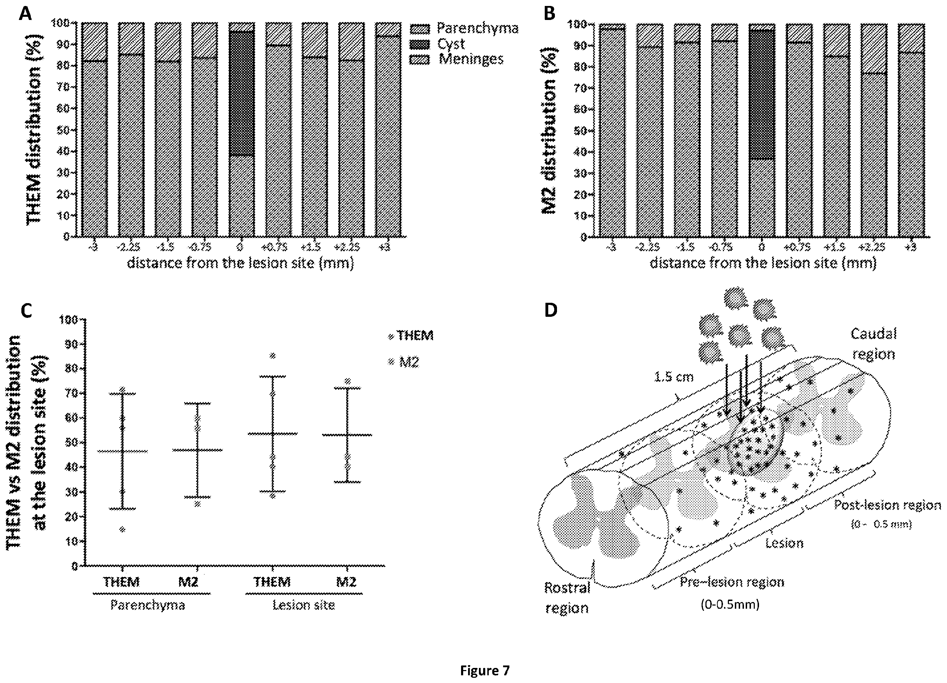

**Figure 7****. Following transplantation, the THEM and M2 cells persist and diffuse radially and longitudinally in the spinal parenchyma.**
   Graph depicting the percentage of THEM distribution in the parenchyma along the spinal cord. The different distribution areas (parenchyma, cysts, meninges) are indicated with different colors (legend in the figure). As shown in the graph, the THEMs spread longitudinally from the transplant site to different areas of the spinal cord, concentrating in the section corresponding to the spinal trauma in the area of the cyst. (B) Graph depicting the percentage distribution of M2 in the parenchyma along the spinal cord. As shown in the graph, the M2s diffuse longitudinally from the transplant site in different areas of the spinal cord, concentrating in the section corresponding to the spinal trauma in the area of the cyst. (C) Graph depicting the percentage distribution of THEM and M2 in the spinal parenchyma and cyst at the lesion site. Experimental THEM group (dark gray, parenchyma 46.46% ± 10.40%, cysts 53.54% ± 10.40%, n=5), experimental M2 group (light gray, parenchyma 46.90% ± 10.95%, cysts 53.10% ± 10.95%, n=3). (D) Image representative of the radial and longitudinal distribution of the THEMs along the spinal cord. The differences between the experimental conditions analyzed were analyzed by Unpaired t-test. The data are expressed as mean ± SEM; n=3 animals, 8 sections/animal analyzed for each group. *P<0.05. M2= phenotype 2 macrophages; THEM= Tumor-Hypoxia Educated Macrophages.

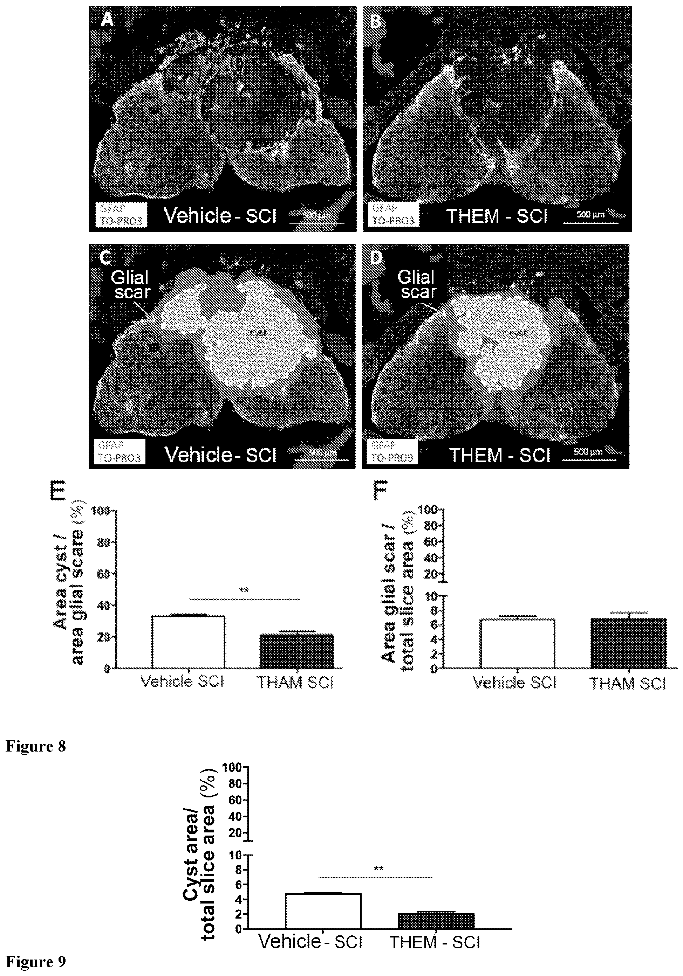

**FIGURE 8****. Following severe spinal injury, the animals subjected to multiple THEM transplantation show decreased lesioned area with respect to the control animals.**
   (A-D) Spinal cord cross-sections subjected to immunostaining with the specific marker for the GFAP astrocytes (light gray) and TO-PRO3 (dark gray) in control animals and in animals subjected to multiple THEM transplantation where the areas considered for quantification are depicted. (A-B) Spinal cord cross-sections subjected to immunostaining with the specific marker for the GFAP astrocytes (light gray) and TO-PRO3 (dark gray) in control animals (A) and in animals subjected to multiple THEM transplantation (B). (C-D) Spinal cord cross-sections subjected to immunostaining with the specific marker for the GFAP astrocytes (light gray) and TO-PRO3 (dark gray) in control animals and in animals subjected to multiple THEM transplantation (D). The dark gray area indicates the area of the glial scar. The dashed area indicates the cyst area. Together these two regions represent the total area of the lesion. (E) Graph depicting the percentage ratio of cyst area (light gray area) to total glial scar area in control animals (white) and in animals subjected to multiple THEM transplantation (black). The multiple THEM transplantation results in a significant reduction in cyst area in the treated animals (21.38% ± 2.05%, n=3) with respect to the control animals (33.24% ± 0.87%, n=3). (F) Graph depicting the percentage ratio of glial scar area (dashed area) to total section area in control animals (white) and in animals subjected to multiple THEM transplantation (black). There were no differences in the percentage of the glial scar area on the total area of the section between the animals subjected to multiple THEM transplantation (6.79% ± 0.85%, n=3) and the control animals (6.69% ± 0.53%, n=3). The differences between the experimental conditions were analyzed by Unpaired t-test. The data are expressed as mean ± SEM; 8 sections/animal are analyzed for each group. **P<0.01. GFAP= Glia Fibrillary Acid Protein.
**FIGURE 9****. Following severe spinal injury, the animals subjected to multiple THEM transplantation show a reduction in cyst area with respect to the control animals.**
   Graph depicting the percentage ratio of cyst area (light gray area) to total section area in control animals (white) and in animals subjected to multiple THEM transplantation (black). The multiple THEM transplantation results in a significant reduction in cyst area in the treated animals (2.03% ± 0.30%, n=3) with respect to the control animals (4.73% ± 0.15%, n=3). The differences between the experimental conditions analyzed were analyzed by Unpaired t-test. The data are expressed as mean ± SEM; 8 sections/animal are analyzed for each group. **P<0.01.

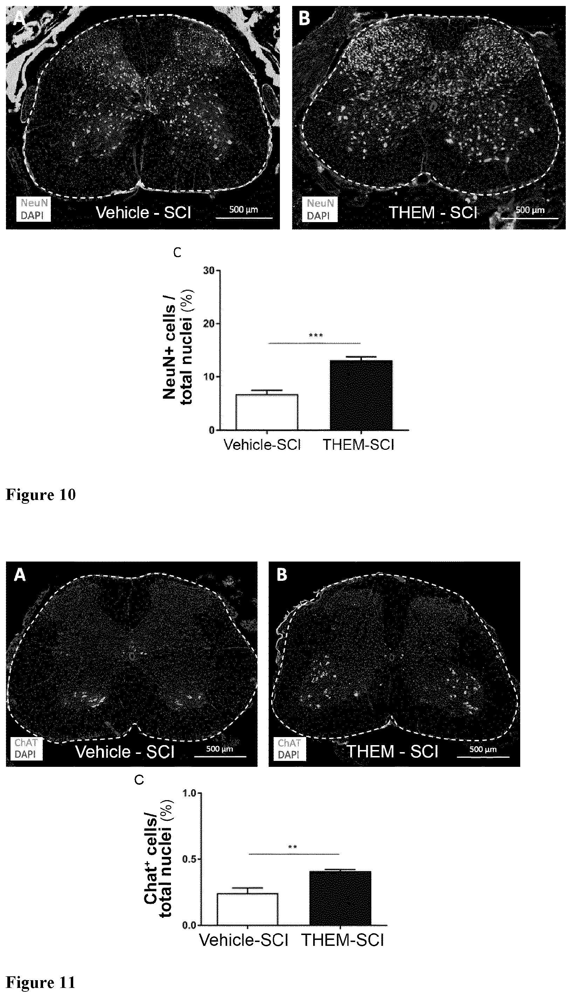

**FIGURE 10****. Following severe spinal injury, the animals subjected to multiple THEM transplantation show an increase in the number of total neurons with respect to the control animals.** (A-B) Spinal cord cross-sections subjected to immunostaining with the neuronal cell specific marker NeuN (light gray) and DAPI (dark gray) in control animals (A) and in animals subjected to multiple THEM transplantation (B). The dashed line delimits the area considered for quantification (total cross-sectional area). (C) Percentage number of NeuN+ cells present in the medullary parenchyma of control animals (white) and animals subjected to multiple THEM transplantation (black). The percentage of neuronal cells (NeuN+) is normalized for the number of total nuclei. As shown in the figure, the percentage of total neurons was significantly higher in the animals subjected to multiple THEM transplantation (13.13% ± 0.632%, n=5) with respect to the control animals (6.81% ± 0.726%, n=4). The differences between the experimental conditions analyzed were analyzed by Unpaired t-test. The data are expressed as mean ± SEM; n=5 animals, 8 sections/animal analyzed for each group. ***P<0.001.
**FIGURE 11****. Following severe spinal injury, the animals subjected to multiple THEM transplantation show an increase in the number of cholinergic neurons with respect to the control animals.**
   (A-B) Spinal cord cross-sections subjected to immunostaining with the neuronal cell specific marker ChAT (light gray) and DAPI (dark gray) in control animals (A) and in animals subjected to multiple THEM transplantation (B). The dashed line delimits the area considered for quantification (total cross-sectional area). (C) Graph depicting the percentage number of ChAT+ cells present in the medullary parenchyma of control animals (white) and animals subjected to multiple THEM transplantation (black). The percentage of cholinergic neurons (ChAT+) is normalized for the number of total nuclei. As shown in the figure, the percentage of cholinergic neurons was significantly higher in the animals subjected to multiple THEM transplantation (0.412% ± 0.011%, n=4) with respect to the control animals (0.245% ± 0.038%, n=4). The differences between the experimental conditions considered were analyzed by Unpaired t-test. The data are expressed as mean ± SEM; 8 sections/animal are analyzed for each group. **P<0.01. ChAT= Choline acetyltransferase.

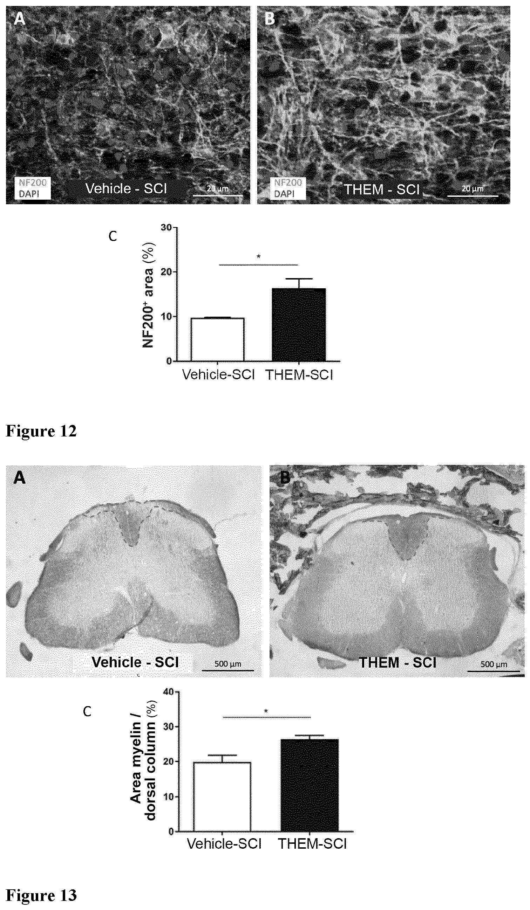

**FIGURE 12****. Following severe spinal injury, the animals subjected to multiple THEM transplantation show an increase in neurofilament content with respect to the control animals.**
   (A-B) Enlargement of a peri-lesion region obtained from longitudinal sections of the spinal cord subjected to immunostaining with the marker specific for the NF200 (light gray) and DAPI (dark gray) neurofilament proteins in control animals (A) and in animals subjected to multiple THEM transplantation (B). (C) Graph depicting the percentage ratio between the area expressing the NF200 marker (NF200 + area) and the total area considered for the analysis in control animals (white) and animals subj ected to multiple THEM transplantation (black). The percentage of myelin was calculated as NF200-positive pixels present in the ROI with respect to the total pixels of the considered ROI. As shown, the percentage of NF200 was significantly higher in animals subjected to multiple THEM transplantation (16.36% ± 2.09%, n=3) with respect to the control animals (9.723% ± 0.133%; n=3). The differences between the experimental conditions studied were analyzed by Unpaired t-test. The data are expressed as mean ± SEM; 8 sections/animal are analyzed for each group. *P<0.05. NF200= Neurofilament-200, ROI= Region Of Interest.
**FIGURE 13****. Following severe spinal injury, the animals subjected to multiple THEM transplantation show an increase in myelin with respect to the control animals.**
   (A-B) Spinal cord cross-sections subjected to Luxol Fast Blue (LFB) staining in control animals (A) and in animals subjected to multiple THEM transplantation (B). LFB allows detecting the myelin (dark gray area) contained in the parenchyma of the spinal cord and specifically, in the area of the posterior cord (ROI). (C) Percentage of myelin present in the ROI in control animals (white) and in animals subjected to multiple THEM transplantation (black). The percentage of myelin was calculated as myelin-positive pixels present in the ROI with respect to the total pixels of the considered ROI. As shown in the figure, the percentage of myelin contained in the posterior cord of the spinal cord was significantly higher in the animals subjected to multiple THEM transplantation (26.470% ± 1.075%, n=5) with respect to the control animals (19.870% ± 1.999%, n=3). The differences between the experimental conditions analyzed were analyzed by Unpaired t-test. The data are expressed as mean ± SEM; n=5 animals, 8 sections/animal analyzed for each group. *P<0.05. ROI= Region Of Interest.

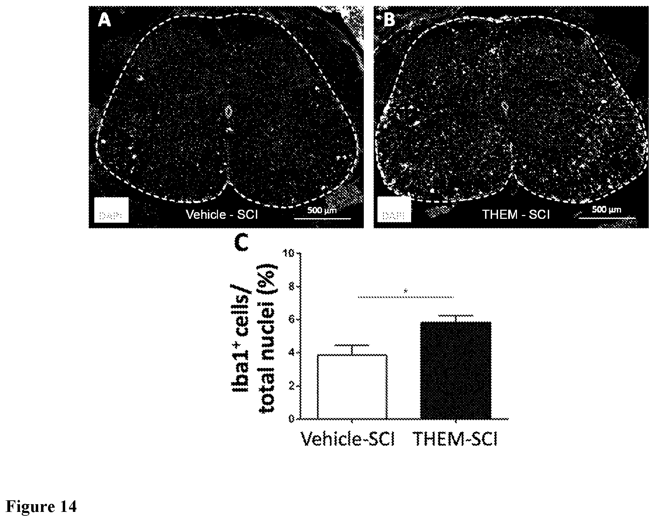

**FIGURE 14****. Following severe spinal injury, the animals subjected to multiple THEM transplantation show intensive recruitment of immune cells with respect to the control animals.**
   (A-B) Spinal cord cross-sections subjected to immunostaining with the specific marker for cells belonging to the microglial and macrophage population Iba1⁺ (light gray) and DAPI (dark gray) in control animals (A) and in animals subjected to multiple THEM transplantation (B). The dashed line delimits the area considered for quantification (total cross-sectional area). (C) Percentage of Iba1+ cells present in the marrow parenchyma in animals subjected to multiple THEM transplantation (black) and in control animals (white). The percentage of activated microglia cells (Iba1+), normalized for the number of total nuclei, was significantly higher in the animals subjected to multiple THEM transplantation (5.820% ± 0.411%, n=5) with respect to the control animals (3.847% ± 0.597%, n=3). The differences between the experimental conditions studied were analyzed by Unpaired t-test. The data are expressed as mean ± SEM; n=5 animals, 8 sections/animal analyzed for each group. *P<0.05.

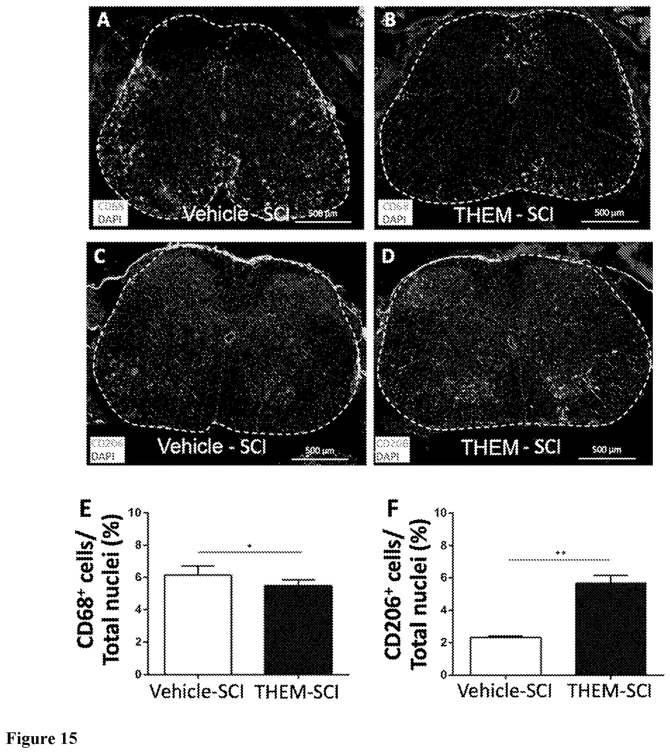

**FIGURE 15****. Following severe spinal injury, the animals subjected to multiple THEM transplantation show intensive recruitment of immunity cells with respect to the control animals.**
   (A-B) Spinal cord cross-sections subjected to immunostaining with the specific marker for cells belonging to the macrophage population CD68 (light gray) and DAPI (dark gray) in control animals (A) and in animals subjected to multiple THEM transplantation (B). The dashed line delimits the area considered for quantification (total cross-sectional area). (C-D) Spinal cord cross-sections subjected to immunostaining with the specific marker for cells belonging to the macrophage population of CD206 (light gray) and DAPI (dark gray) pro-regenerative phenotype (M2) in control animals (C) and in animals subjected to multiple THEM transplantation (D). The dashed line delimits the area considered for quantification (total cross-sectional area). (E) Percentage of CD68+ cells in the marrow parenchyma in animals subjected to multiple THEM transplantation (black) and in control animals (white). The percentage of macrophages (CD68+), normalized for the number of total nuclei, was higher in the control animals (6.143% ± 0.554%,
n=3) with respect to the animals subjected to multiple THEM transplantation (5.484% ± 0.367%, n=5). (F) Percentage of CD206+ cells in the marrow parenchyma in animals subjected to multiple THEM transplantation (black) and in control animals (white). The percentage of phenotype 2 macrophages (CD206+), normalized for the number of total nuclei, was significantly higher in the control animals (2.320% ± 0.080%, n=3) with respect to the animals subjected to multiple THEM transplantation (5.655% ± 0.497%, n=4). The differences between the experimental conditions were analyzed by Unpaired t-test. The data are expressed as mean ± SEM; n=5 animals, 8 sections/animal analyzed for each group. *P<0.05, **P<0.01.

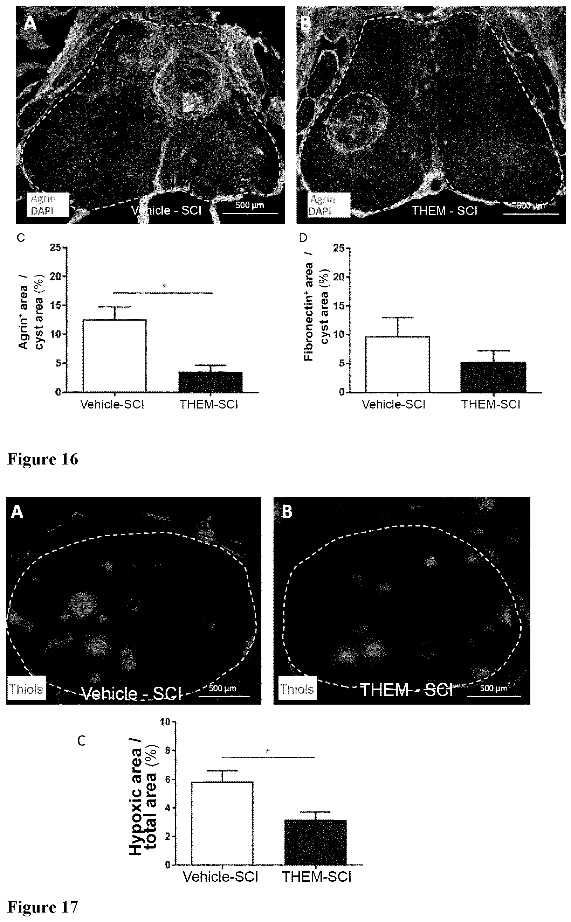

**FIGURE 16****. Following severe spinal injury, the animals subjected to multiple THEM transplantation show a reduction in the extracellular matrix deposition.**
   (A-B) Spinal cord cross-sections subjected to immunostaining with the extracellular matrix specific marker Agrina (light gray) and DAPI (dark gray) in control animals (A) and in animals subjected to multiple THEM transplantation (B). The dashed line in orange delimits the area of interest considered for the quantification (total area of the cyst). (C) Percentage of Agrin present in the cyst of control animals (white) and animals subjected to multiple THEM transplantation (black), normalised for the total area of the section (white dashed line). As shown in the figure, the percentage of Agrin contained in the region considered was significantly lower in the animals subjected to multiple THEM transplantation (3.500% ± 1.112%, n=3) with respect to the control animals (12.570% ± 2.140%, n=3). (D) Percentage of Fibronectin present in the cyst in control animals (white) and in animals subjected to multiple THEM transplantation (black) normalized for the total area of the section (white dashed line). As shown in the figure, the percentage of Fibronectin contained in the region considered showed a trend of reduction in the animals subjected to multiple THEM transplantation (5.190% ± 2.019%, n=3) with respect to the control animals (9.625% ± 3.375%, n=3). The percentage of the various components of the extracellular matrix was calculated as positive pixels for the specific component of the extracellular matrix (Agrin, Fironectin) present in the cyst with respect to the total pixels of the ROI considered. The differences between the experimental conditions were analyzed by Unpaired t-test. The data are expressed as mean ± SEM; n=3 animals, 8 sections/animal analyzed for each group. *P<0.05.
**FIGURE 17****. Following severe spinal injury, the animals subjected to multiple THEM transplantation show reduced hypoxic areas with respect to the control animals.**
   (A-B) Spinal cord cross-sections stained for the detection of free thiols in control animals (A) and in animals subjected to multiple THEM transplantation (B). The dashed line delimits the area considered for quantification (total cross-sectional area). (C) Percentage of hypoxic area in the marrow parenchyma of control animals (white) and animals subjected to multiple THEM transplantation (black). The percentage hypoxic area was calculated as positive pixels per hypoxic area present in the total section with respect to the total pixels of the total section. As shown in the figure, the percentage hypoxic area was significantly lower in the animals subjected to multiple THEM transplantation (3.173% ± 0.528%, n=3) with respect to the control animals (5.866% ± 0.745%, n=3). The differences between the experimental conditions were analyzed by Unpaired t-test. The data are expressed as mean ± SEM; 8 sections/animal are analyzed for each group. *P<0.05.

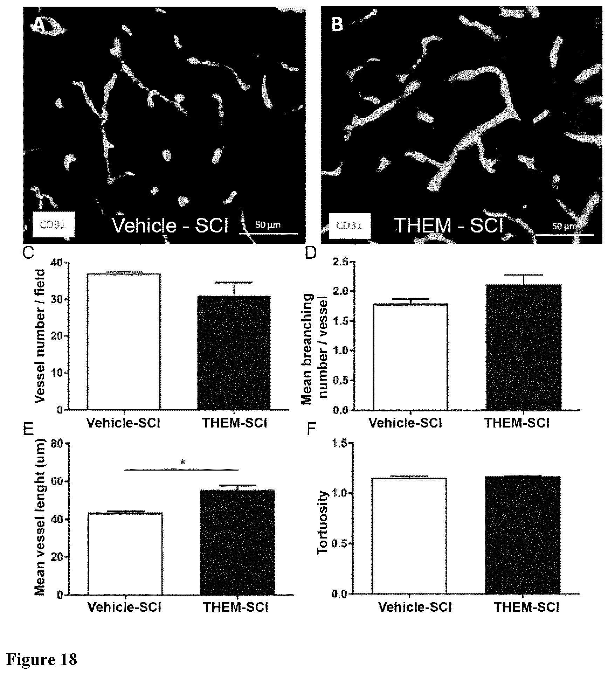

**FIGURE 18****. Following severe spinal injury, the animals subjected to multiple THEM transplantation show vessels with an increased number of branches and a greater maximum length with respect to the control animals.**
   (A-B) Representative regions of interest (ROI) obtained from spinal cord cross-sections subjected to immunostaining with the specific marker for CD31 endothelial cells (gray) in control animals (A) and in animals subjected to multiple THEM transplantation (B). (C) Average number of vessels/field in the area under consideration (ROI) in control animals (white) and animals subjected to multiple THEM transplantation (black). As shown, the mean number of vessels/field was higher in the control animals (37.070 ± 0.379, n=3) with respect to the animals subjected to multiple THEM transplantation (30.940 ± 3.592, n=3). (D) Average number of branches/vessel in the area under consideration (ROI) in control animals (white) and animals subjected to multiple THEM transplantation (black). As shown, the mean number of vessels/field was higher in the animals subjected to multiple THEM transplantation (2.110 ± 0.165, n=3) with respect to the control animals (1.793 ± 0.073, n=3). (E) Maximum average length of the vessels in the area under consideration (ROI) in control animals (white) and animals subjected to multiple THEM transplantation (black). As shown, the mean number of branches/vessel and the maximum mean length of vessels/field were significantly greater in the animals subjected to multiple THEM transplantation (55.410 µm ± 2.500 µm) with respect to the control animals (43.450 µm ± 0.751 µm). (F) Average value of the tortuosity of the vessels in the area under consideration (ROI) in control animals (white) and in animals subjected to multiple THEM transplantation (black). The tortuosity value showed no difference between the experimental groups considered (1.168 ± 0.005 animals subjected to multiple THEM transplantation, 1.153 ± 0.015 controls). The differences between the experimental conditions were analyzed by Unpaired t-test. The data are expressed as mean ± SEM; n=5 animals, 8 sections/animal analyzed for each group. *P<0.05. ROI= Region Of Interest.

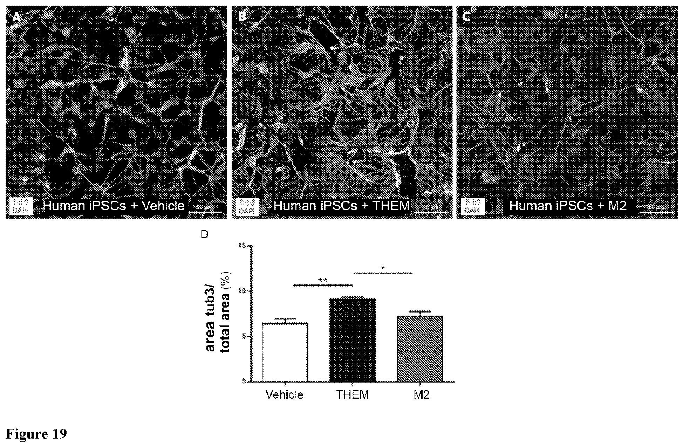

**FIGURE 19****. The addition of mouse THEM to human cultures of iPSCs promotes motor neurons differentiation.**
   A-B) Immunostaining of human IPSCs differentiated into motor neurons with the marker specific for the neurons βIII Tubulin (Tub3; light gray) and DAPI (dark gray) in control cultures (A), in co-cultures with THEM (B) and in co-cultures with M2 (C). (D) Area of expression of Tub3 normalized for the value of the total area considered for the analysis in control samples (white), in co-cultures with THEM (black) and with M2 macrophages (gray). As shown, the area of Tub3 expression in the co-cultures with THEM is significantly greater with respect to the controls and co-cultures with M2. (6.423% ± 0.494% controls, co-cultures with THEM 9.097% ± 0.253%, co-cultures with M2 7.235% ± 0.492%). The differences between the experimental conditions studied were analyzed by Unpaired t-test. The data are expressed as mean ± SEM; n=3 donors, analyzed 40 fields/donor, 4 images/field. *P<0.05, **P<0.01.

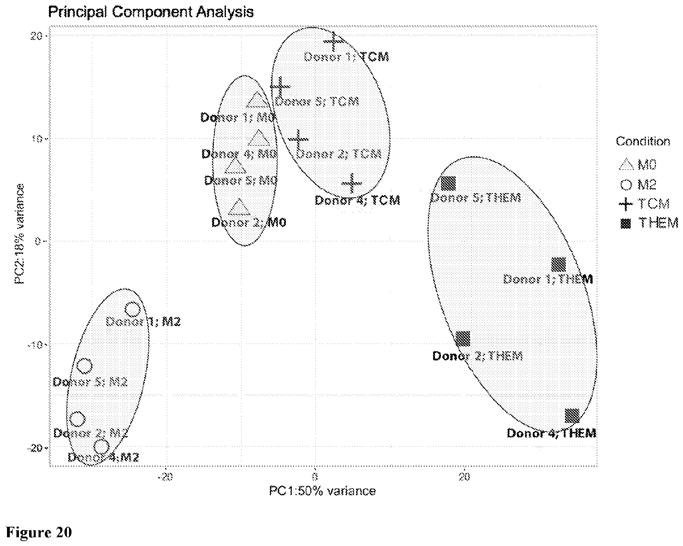

**Figure 20****: PCA on whole transcriptomic profile shows that THEM have a e unique molecular signature which requires tumor conditioning and hypoxia.** Principal component analysis of the transcriptome of undifferentiated M0 macrophages (triangle), M2 polarized macrophages (circle), TCM cultured without hypoxia (cross), and THEM (square). The PCA analysis showed that THEM cluster is well separated from the other groups, highlighting the THEM unique molecular signature and demonstrates the need of hypoxia to generate THEM.

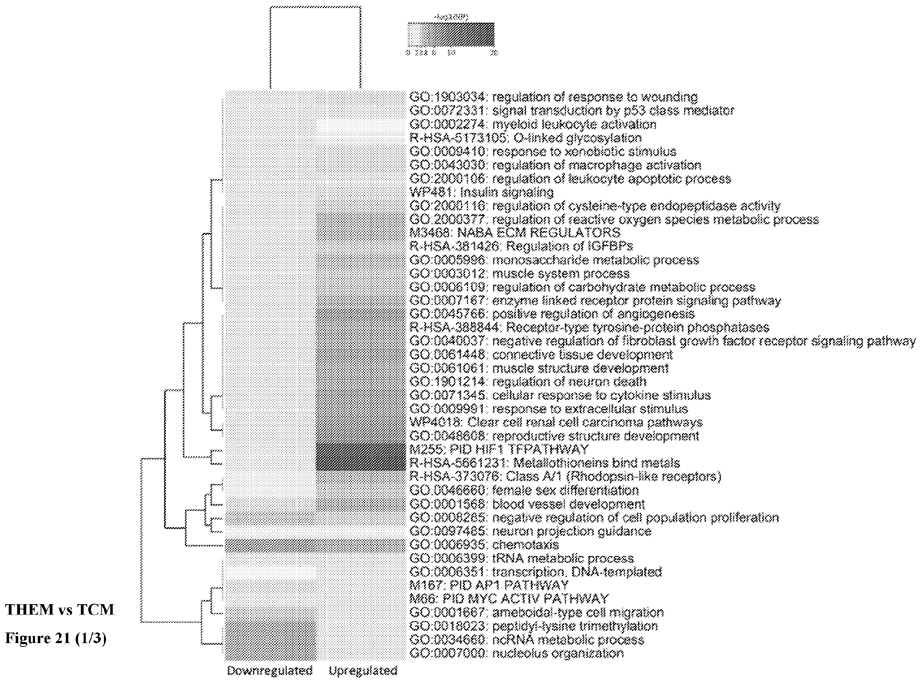

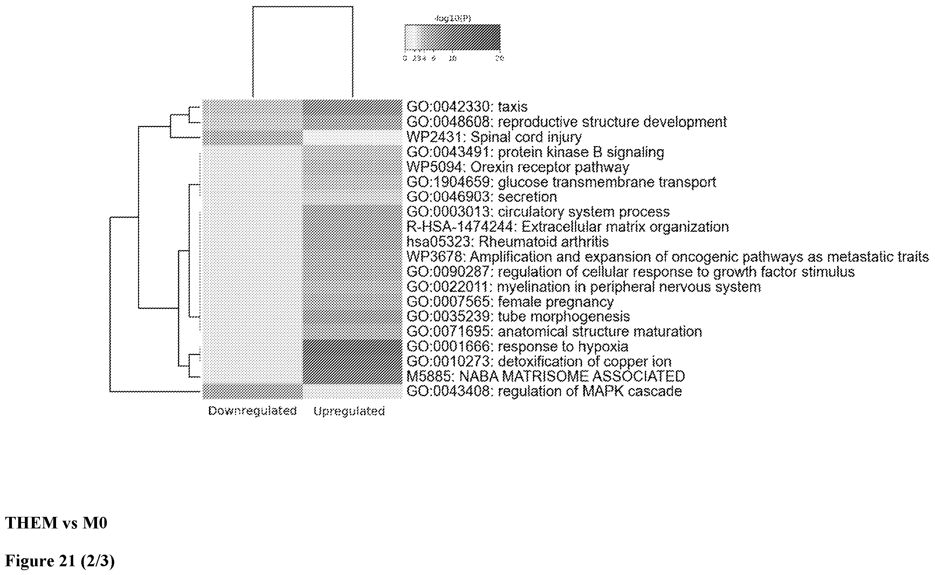

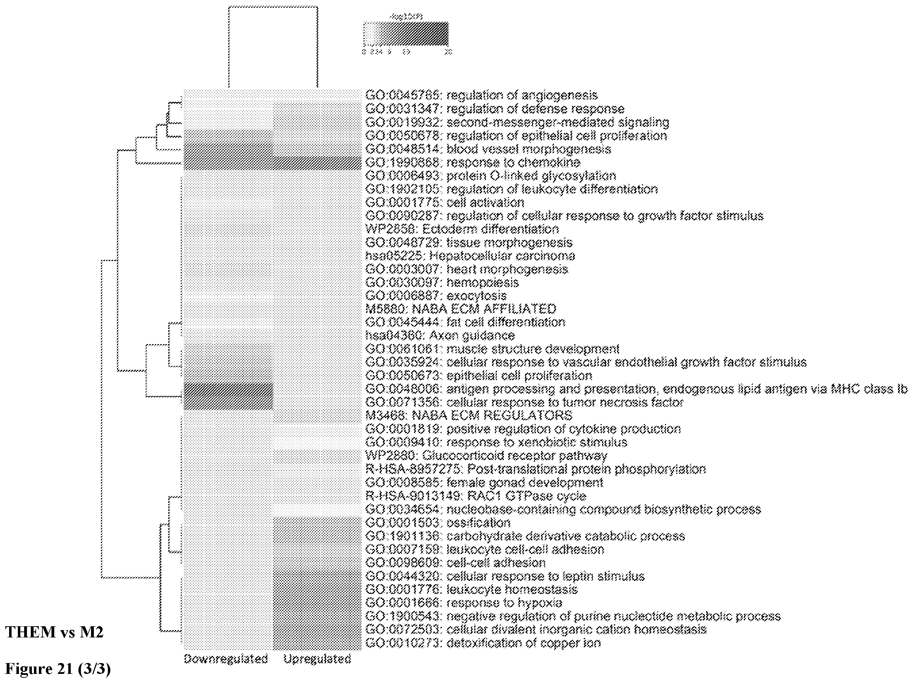

**Figure 21****: Heatmap of the top enriched Gene Ontology in THEM compared to TCM cultured without hypoxia, M0 and M2 macrophages.**
**Figure 22****: Differential gene expression of THEM versus M0, M2 and TCM cultured without hypoxia.**
   A) Vulcano plots showing the Log2 fold change of genes expressed by THEM compared to M0 (left panel), M2 (middle panel) and TCM cultured without hypoxia (right panel); B) Graphs showing the expression level of gene upregulated and highly expressed in THEM compare to M2 and M0; C) Graphs showing the expression level of gene downregulated at low or no level in THEM compare to M2 and M0.

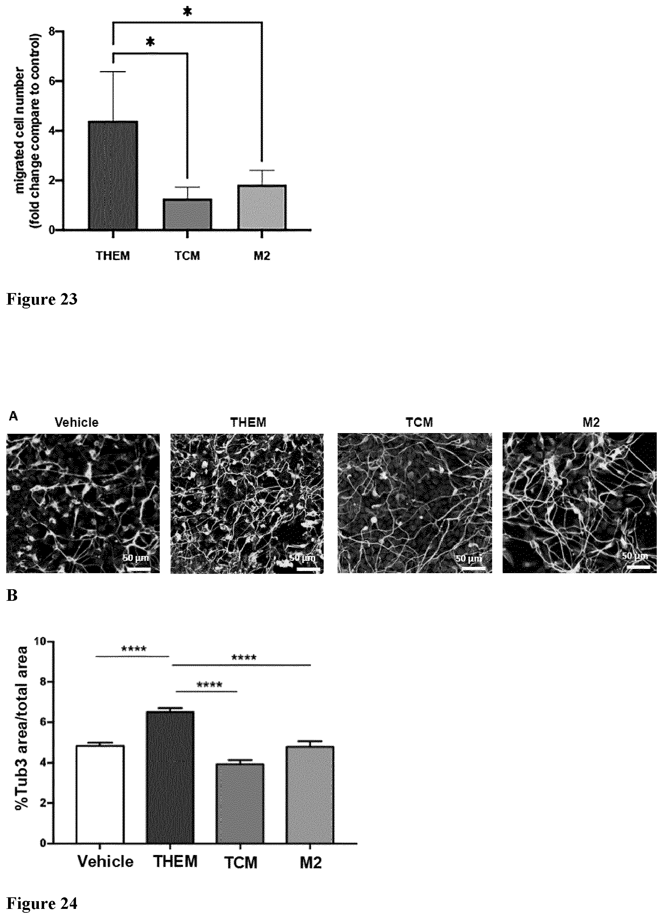

**Figure 23****: Migration assay for THEM, TCM, and M2 macrophages with SDF-1 as chemoattractant.**
   Transwell migration assays were performed in 24-well chemotaxis chambers (8-µm pore size) with SDF-1a (100 ng/ml) in the lower chamber as chemoattractant. Bars represent the fold changed compared to control of the number of migrated cells.
**Figure 24****: Murine THEM neuronal regenerative function on human motor neurons. (A)** Immunstaining of human iPSCs differentiated into motoneurons stained with the specific neuronal marker βIII tubulin (Tub3, green) and DAPI (blue) without co-culture **(CRTL),** with murine THEM co-colture **(THEM),** with **TCM** without hypoxia co-colture and with M2 co-colture **(M2). (B)** Graph representing Tub3 expression area normalized with the total area considered for the analysis in iPSCs-derived motor neurons without co-culture, with THEM, TCM without hypoxia and M2 co-colture. Tub3 expression area in THEM co-colture results statistically higher than all the other groups, indicating that THEM induced higher neuronal differentiation and neuronal process extension. Importantly, the expression of Tub3 observed with THEM co-colture is higher compare to TCM without hypoxia co-colture, indicating that hypoxia treatment is essential to define the effective regenerative phenotype of THEM.

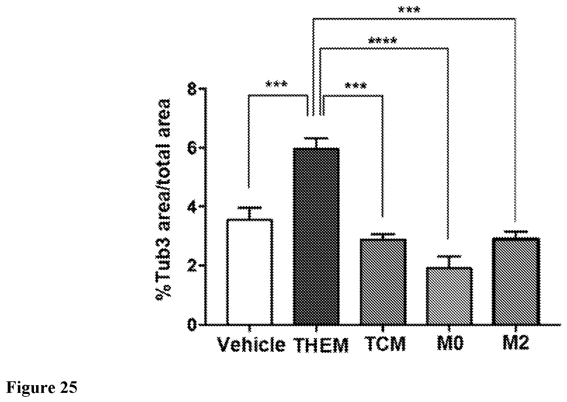

**Figure 25****: Human THEM neuronal regenerative function on human motor neurons.**
   **(A)** Graph representing Tub3 expression area normalized by the total area considered for the analysis in control, human THEM co-colture, human TCM co-colture, human M0 co-colture and in human M2 co-colture *in vitro.* As showed Tub3 expression area in human THEM co-colture results significantly higher than in control cells, M0 co-colture, M2 macrophages co-colture and human TCM co-colture suggesting a higher neuronal differentiation and neuronal process extension. In addition, the higher expression of Tub3 in THEM co-colture than in TCM co-colture indicates that hypoxia treatment is essential to determinate the proper effective phenotype of THEM.

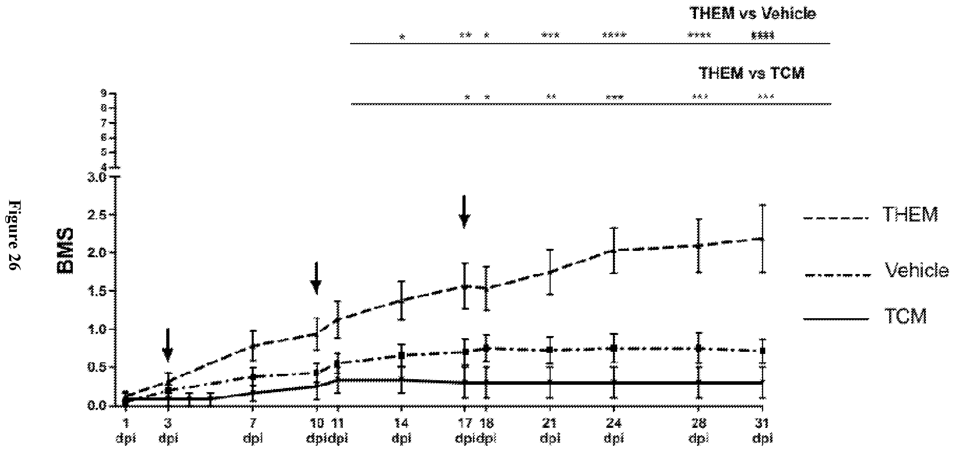

**Figure 26****: THEM and TCM *in vivo* neuronal regenerative function on spinal cord injured mice motor recovery.**
   Graph representing the BMS score of THEM; TCM and vehicle (no cells) transplanted animals. Black arrows indicate the time-points at which transplants were performed. THEM transplanted animals showed a gradual improvement of the locomotor performance that reach a score significantly higher (2.188± 0.195) than Vehicle score (0.711 ± 0.068) and TCM score (0.300 ± 0.200). statistical differences between experimental groups were analyzed by 2wayANOVA test and Sidak *post-test.* Data are showed as mean ± SEM. *p≤0.05, **p≤0.01, ***p≤0.001, ****p≤0.0001.

**TABLE 1. Gene expression. Table containing the list of up-regulated genes in mouse THEM vs mouse M2 macrophages obtained by sequencing messenger RNAs (RNAseq).**

| gene expression | gene_id | GENENAME |
|---|---|---|
| Abcc3 | ENSMUSG00000020865.16 | ATP-binding cassette, sub-family C (CFTR/MRP), member 3 |
| Ache | ENSMUSG00000023328.14 | acetylcholinesterase |
| Adamts6 | ENSMUSG00000046169.10 | a disintegrin-like and metallopeptidase with thrombospondin type 1 motif, 6 |
| Adamtsl4 | ENSMUSG00000015850.11 | ADAMTS-like 4 |
| Adora3 | ENSMUSG00000000562.5 | adenosine A3 receptor |
| Adrb2 | ENSMUSG00000045730.4 | adrenergic receptor, beta 2 |
| Antxr2 | ENSMUSG00000029338.13 | anthrax toxin receptor 2 |
| Aqp9 | ENSMUSG00000032204.13 | aquaporin 9 |
| Arrdc4 | ENSMUSG00000042659.15 | arrestin domain containing 4 |
| Atp8b4 | ENSMUSG00000060131.11 | ATPase, class I, type 88, member 4 |
| Cd82 | ENSMUSG00000027215.13 | CD82 antigen |
| Ctla2a | ENSMUSG00000044258.10 | cytotoxic T lymphocyte-associated protein 2 alpha |
| Cysltr1 | ENSMUSG00000052821.3 | cysteinyl leukotriene receptor 1 |
| F11r | ENSMUSG00000038235.4 | F11 receptor |
| Fcgr1 | ENSMUSG00000015947.10 | Fc receptor, IgG, high affinity I |
| Fcgr4 | ENSMUSG00000059089.4 | Fc receptor, IgG, low affinity IV |
| Firt3 | ENSMUSG00000051379.12 | fibronectin leucine rich transmembrane protein 3 |
| Fpr2 | ENSMUSG00000052270.7 | formyl peptide receptor 2 |
| Gipr | ENSMUSG00000030406.7 | gastric inhibitory polypeptide receptor |
| Gjal | ENSMUSG00000050953.10 | gap junction protein, alpha 1 |
| Gpr141 | ENSMUSG00000053101.3 | G protein-coupled receptor 141 |
| Gpr171 | ENSMUSG00000050075.8 | G protein-coupled receptor 171 |
| Gpr35 | ENSMUSG00000026271.15 | G protein-coupled receptor 35 |
| Grk5 | ENSMUSG00000003228.9 | G protein-coupled receptor kinase 5 |
| Gyp c | ENSMUSG00000090523.2 | glycophorin C |
| H2-Q6 | ENSMUSG00000073409.12 | histocompatibility 2, Q region locus 6 |
| H2-Q7 | ENSMUSG00000060550.16 | histocompatibility 2, Q region focus 7 |
| Havcr2 | ENSMUSG00000020399.14 | hepatitis A virus cellular receptor 2 |
| Ifitm3 | ENSMUSG00000025492.6 | interferon induced transmembrane protein 3 |
| Igf1r | ENSMUSG00000005533.10 | insulin-like growth factor I receptor |
| Il13ra1 | ENSMUSG00000017057.9 | interleukin 13 receptor, alpha 1 |
| Il18rap | ENSMUSG00000026068.11 | interleukin 18 receptor accessory protein |
| Il1r2 | ENSMUSG00000026073.13 | interleukin 1 receptor, type II |
| Il21r | ENSMUSG00000030745.9 | interleukin 21 receptor |
| Il4ra | ENSMUSG00000030748.9 | interleukin 4 receptor, alpha |
| Irak2 | ENSMUSG00000060477.14 | interleukin-1 receptor-associated kinase 2 |
| Itga1 | ENSMUSG00000042284.10 | integrin alpha 1 |
| Itga6 | ENSMUSG00000027111.16 | integrin alpha 6 |
| Itga9 | ENSMUSG00000039115.13 | integrin alpha 9 |
| ItgaI | ENSMUSG00000030830.18 | integrin alpha L |
| Itgam | ENSMUSG00000030786.18 | integrin alpha M |
| Itm2a | ENSMUSG00000031239.5 | integral membrane protein 2A |
| Jmjd6 | ENSMUSG00000056962.11 | jumonji domain containing 6 |
| Kcna3 | ENSMUSG00000047959.4 | potassium voltage-gated channel, shaker-related subfamily, member 3 |
| Ltb4r1 | ENSMUSG00000046908.5 | leukotriene B4 receptor 1 |
| Ly6a | ENSMUSG00000075602.10 | lymphocyte antigen 6 complex, locus A |
| Maged1 | ENSMUSG00000025151.16 | melanoma antigen, family D, 1 |
| Mfsd6 | ENSMUSG00000041439.15 | major facilitator superfamily domain containing 6 |
| Milr1 | ENSMUSG00000040528.15 | mast cell immunoglobulin like receptor 1 |
| Nectin2 | ENSMUSG00000062300.14 | nectin cell adhesion molecule 2 |
| Nectin4 | ENSMUSG00000006411.12 | nectin cell adhesion molecule 4 |
| Ninj1 | ENSMUSG00000037966.15 | ninjurin 1 |
| Nos2 | ENSMUSG00000020826.9 | nitric oxide synthase 2, inducible |
| Notch1 | ENSMUSG00000026923.15 | notch 1 |
| Notch2 | ENSMUSG00000027878.11 | notch 2 |
| Pik3ip1 | ENSMUSG00000034614.14 | phosphoinositide-3-kinase interacting protein 1 |
| Ptges | ENSMUSG000000050737.13 | prostaglandin E synthase |
| Ptgfm | ENSMUSG00000027864.9 | prostaglandin F2 receptor negative regulator |
| Ptgs2 | ENSMUSG00000032487.8 | prostaglandin-endoperoxide synthase 2 |
| Ptprf | ENSMUSG00000033295.14 | protein tyrosine phosphatase, receptor type, F |
| Rap2a | ENSMUSG00000051615.14 | RAS related protein 2a |
| Rgs11 | ENSMUSG00000024186.15 | regulator of G-protein signaling 11 |
| Ripk3 | ENSMUSG00000022221.14 | receptor-interacting serine-threonine kinase 3 |
| Slc16a3 | ENSMUSG00000025161.16 | solute carrier family 16 (monocarboxylic acid transporters), member 3 |
| Slc41a1 | ENSMUSG00000013275.9 | solute carrier family 41, member 1 |
| Spata13 | ENSMUSG00000021990.16 | spermatogenesis associated 13 |
| Stac2 | ENSMUSG00000017400.10 | SH3 and cysteine rich domain 2 |
| Tex14 | ENSMUSG00000010542.16 | testis expressed gene 14 |
| Ticam2 | ENSMUSG00000056130.10 | toll-like receptor adaptor molecule 2 |
| Tlr2 | ENSMUSG000000027995.10 | toll-like receptor 2 |
| Tlr5 | ENSMUSG00000079164.8 | toll-like receptor 5 |
| Tmem37 | ENSMUSG00000050777.7 | transmembrane protein 37 |
| Tnfrsf18 | ENSMUSG00000041954.18 | tumor necrosis factor receptor superfamily, member 18 |
| Tnfrsf1a | ENSMUSG00000030341.17 | tumor necrosis factor receptor superfamily, member 1a |
| Tnfrsf23 | ENSMUSG00000037613.16 | tumor necrosis factor receptor superfamily, member 23 |
| Trem1 | ENSMUSG00000042265.13 | triggering receptor expressed on myeloid cells 1 |
| Treml2 | ENSMUSG00000071068.7 | triggering receptor expressed on myeloid cells-like 2 |
| Tspan13 | ENSMUSG00000020577.17 | tetraspanin 13 |
| Unc13a | ENSMUSG00000034799.16 | unc-13 homolog A |
| Vamp2 | ENSMUSG00000020894.16 | vesicle-associated membrane protein 2 |

**TABLE 2. Secretome. Table containing the list of up-regulated genes in mouse THEM vs mouse M2 macrophages obtained by sequencing messenger RNAs (RNAseq) relative to secretome-specific transcripts (secret proteins).**

| **Secreted molecules** | **gene_id** | **GENENAME** |
|---|---|---|
| Acpp | ENSMUSG00000032561.15 | acid phosphatase, prostate |
| Adamtsl4 | ENSMUSG00000015850.11 | ADAMTS-like 4 |
| Arg1 | ENSMUSG00000019987.9 | arginase |
| Ctla2a | ENSMUSG00000044258.10 | cytotoxic T lymphocyte-associated protein 2 alpha |
| Cxcl1 | ENSMUSG00000029380.11 | chemokine (C-X-C motif) ligand 1 |
| Cxcl16 | ENSMUSG00000018920.11 | chemokine (C-X-C motif) ligand 16 |
| Cxcl2 | ENSMUSG00000058427.10 | chemokine (C-X-C motif) ligand 2 |
| Cxcl3 | ENSMUSG00000029379.10 | chemokine (C-X-C motif) ligand 3 |
| F13a1 | ENSMUSG00000033109.16 | coagulation factor XIII, A1 subunit |
| F5 | ENSMUSG00000026579.8 | coagulation factor V |
| Fam20a | ENSMUSG00000020614.13 | family with sequence similarity 20, member A |
| Fgf11 | ENSMUSG00000042826.13 | fibroblast growth factor 11 |
| Fn1 | ENSMUSG00000026193.15 | fibronectin 1 |
| Igf1r | ENSMUSG00000005533.10 | insulin-like growth factor I receptor |
| Il15 | ENSMUSG00000031712.10 | interleukin 15 |
| Il16 | ENSMUSG00000001741.12 | interleukin 16 |
| Il18 | ENSMUSG00000039217.13 | interleukin 18 |
| Il1b | ENSMUSG00000027398.13 | interleukin 1 beta |
| Isg15 | ENSMUSG00000035692.7 | ISG15 ubiquitin-like modifier |
| Lamc1 | ENSMUSG00000026478.14 | laminin, gamma 1 |
| Ltbp3 | ENSMUSG00000024940.11 | latent transforming growth factor beta binding protein 3 |
| Mgat4a | ENSMUSG00000026110.15 | mannoside acetylglucosaminyltransferase 4, isoenzyme A |
| Mif | ENSMUSG00000033307.7 | macrophage migration inhibitory factor |
| Mmp10 | ENSMUSG00000047562.3 | matrix metallopeptidase 10 |
| Mmp14 | ENSMUSG00000000957.11 | matrix metallopeptidase 14 (membrane-inserted) |
| Mmp19 | ENSMUSG00000025355.7 | matrix metallopeptidase 19 |
| Mmp27 | ENSMUSG00000070323.11 | matrix metallopeptidase 27 |
| Mmp8 | ENSMUSG00000005800.3 | matrix metallopeptidase 8 |
| Mmp9 | ENSMUSG00000017737.2 | matrix metallopeptidase 9 |
| Nampt | ENSMUSG00000020572.8 | nicotinamide phosphoribosyltransferase |
| Npc2 | ENSMUSG00000021242.9 | NPC intracellular cholesterol transporter 2 |
| Pf4 | ENSMUS600000029373.7 | platelet factor 4 |
| Rtn4rl2 | ENSMUSG00000050896.12 | reticulon 4 receptor-like 2 |
| Tgfb 3 | ENSMUSG00000021253.7 | transforming growth factor, beta 3 |
| Tgfbi | ENSMUSG00000035493.10 | transforming growth factor, beta induced |
| Thbs1 | ENSMUSG00000040152.8 | thrombospondin 1 |
| Timp1 | ENSMUSG00000001131.11 | tissue inhibitor of metalloproteinase 1 |
| Tnfsf13b | ENSMUSG00000031497.9 | tumor necrosis factor (ligand) superfamily, member 13b |
| Tnfsf8 | ENSMUSG00000028362.2 | tumor necrosis factor (ligand) superfamily, member 8 |
| Vegfa | ENSMUSG00000023951.17 | vascular endothelial growth factor A |

**TABLE 3. THEM identity gene expression. Table containing the list of univocally up-regulated genes in human THEM vs human TCM, M2, M0 obtained by sequencing messenger RNAs (RNAseq)**

| **UP regulated genes THEM vs TCM, M2, M0** | **Gene ID ENSEMBL** Ensembl release 105 - Dec 2021 ^{©} EMBL-EBI | **gene ID NCBI** | **gene name** |
|---|---|---|---|
| CXCR4 | ENSG00000121966 | 7852 | C-X-C Motif Chemokine Receptor 4 |
| CYTIP | ENSG00000115165 | 9595 | Cytohesin 1 Interacting Protein |
| SLC2A3 | ENSG00000059804 | 6515 | Solute Carrier Family 2 Member 3 |
| MT2A | ENSG00000125148 | 4502 | Metallothionein 2A |
| LEP | ENSG00000174697 | 3952 | Leptin |
| ANGPTL4 | ENSG00000167772 | 51129 | Angiopoietin like 4 |
| MT3 | ENSG00000087250 | 4504 | Metallothionein 3 |
| NIPAL4 | ENSG00000172548 | 348938 | NIPA like domain containing 4 |
| STC2 | ENSG00000113739 | 8614 | Stanniocalcin 2 |
| LOX | ENSG00000113083 | 4015 | Lysyl oxidase |
| NUPR1 | ENSG00000176046 | 26471 | Nuclear protein 1, transcriptional regulator |
| BICDL2 | ENSG00000162069 | 146439 | BICD Family Like Cargo Adaptor 2 |
| C4orf47 | ENSG00000205129 | 441054 | Chromosome 4 open reading frame 47 |
| ADAMTS10 | ENSG00000142303 | 81794 | ADAM metallopeptidase with thrombospondin type 1 motif 10 |
| IGLON5 | ENSG00000142549 | 402665 | IgLON family member 5 |
| PPFIA4 | ENSG00000143847 | 8497 | PTPRF interacting protein alpha 4 |
| PLOD2 | ENSG00000152952 | 5352 | Procollagen-lysine,2-oxoglutarate 5-dioxygenase 2 |
| ADSS1 | ENSG00000185100 | 122622 | Adenylosuccinate synthase 1 |
| DDIT4 | ENSG00000168209 | 54541 | DNA damage inducible transcript 4 |
| STC1 | ENSG00000159167 | 6781 | Stanniocalcin 1 |
| HIF1A-AS3 | ENSG00000258667 | 105370526 | HIF1A antisense RNA 3 |
| HIF1A-AS2 | NO CODE AVAILABLE | 100750247 | HIF1A antisense RNA 2 |
| MT1X | ENSG00000187193 | 4501 | Metallothionein 1X |

**TABLE 4. THEM identity gene expression. Table containing the list of univocally downregulated genes in human THEM vs human TCM, M2, M0 obtained by sequencing messenger RNAs (RNAseq).**

| **Down regulated genes THEM vs TCM,M2,M0** | **Gene ID ENSEMBL** Ensembl release 105 - Dec 2021 ^{©} EMBL-EBI | **gene ID NCBI** | **gene name** |
|---|---|---|---|
| PLXNA2 | ENSG00000076356 | 5362 | Plexin A2 |
| HSPH1 | ENSG00000120694 | 10808 | Heat Shock Protein Family H (Hsp110) Member 1 |
| CYCS | ENSG00000172115 | 54205 | Cytochrome C, Somatic |
| TIGAR | ENSG00000078237 | 57103 | TP53 Induced Glycolysis Regulatory Phosphatase |
| CLEC19A | ENSG00000261210 | 728276 | C-type lectin domain containing 19A |
| FERMT1 | ENSG00000101311 | 55612 | FERM domain containing kindlin 1 |
| REP15 | ENSG00000174236 | 387849 | RAB15 effector protein |
| DCLK2 | ENSG00000170390 | 166614 | Doublecortin like kinase 2 |
| TSPAN12 | ENSG00000106025 | 23554 | Tetraspanin 12 |
| TNFAIP8L2 | ENSG00000163154 | 79626 | TNF alpha induced protein 8 like 2 |
| GPBAR1 | ENSG00000179921 | 151306 | G protein-coupled bile acid receptor 1 |
| CIRBP-AS1 | ENSG00000267493 | 148046 | CIRBP antisense RNA 1 |
| ACTRT3 | ENSG00000184378 | 84517 | Actin related protein T3 |
| PLXNA2 | ENSG00000076356 | 5362 | Plexin A2 |
| EGR3 | ENSG00000179388 | 1960 | Early growth response 3 |
| MARS2 | ENSG00000247626 | 92935 | Methionyl-tRNA synthetase 2, mitochondrial |
| ZNF442 | ENSG00000198342 | 79973 | Zinc finger protein 442 |
| TWNK | ENSG00000107815 | 56652 | Twinkle mtDNA helicase |
| CCL8 | ENSG00000108700 | 6355 | C-C motif chemokine ligand 8 |
| NOP16 | ENSG00000048162 | 51491 | NOP16 nucleolar protein |
| TNIP3 | ENSG00000050730 | 79931 | TNFAIP3 interacting protein 3 |
| RRP9 | ENSG00000114767 | 9136 | Ribosomal RNA processing 9, U3 small nucleolar RNA binding protein |
| ALDH1B1 | ENSG00000137124 | 219 | Aldehyde dehydrogenase 1 family member B1 |
| MANEAL | ENSG00000185090 | 149175 | Mannosidase endo-alpha like |

**TABLE 5. THEM identity antigen expression. Table containing the list of surface antigens expressed by THEM**

| Surface Antigens | |
|---|---|
| Gene | Protein |
| PTPRC | CD45 |
| CD68 | CD68 |
| ITGAM | CD11b |
| CD163 | CD163 |
| CD206 | CD206 |
| CD80 | CD80 |
| CD86 | CD86 |
| CD184 | CXCR4 |
| CD192 | CCR2 |
| CD36 | CD36 |
| SLC2A1 | GLUT1 |
| SLC2A3 | GLUT3 |

**Table 6. Human THEM up-regulated genes related to wound healing, angiogenesis, detoxification and regulation of defence response, response to hypoxia, extracellular matrix remodelling neuronal survival and myelination Gene Ontology terms.**

| **genes name** | **Gene ENSAMBL** Ensembl release 105 - Dec 2021 ^{©} EMBL-EBI | **gene ID** | **gene name** |
|---|---|---|---|
| ADM | ENSG00000148926 | 133 | Adrenomedullin |
| ANGPTL4 | ENSG00000167772 | 51129 | Angiopoietin Like 4 |
| APLN | ENSG00000171388 | 8862 | Apelin |
| BNIP3 | ENSG00000176171 | 664 | BCL2 Interacting Protein 3 |
| CXCL8 | ENSG00000169429 | 3576 | C-X-C Motif Chemokine Ligand 8 |
| CXCR4 | ENSG00000121966 | 7852 | C-X-C Motif Chemokine Receptor 4 |
| DDIT4 | ENSG00000168209 | 54541 | DNA Damage Inducible Transcript 4 |
| EGLN3 | ENSG00000129521 | 112399 | Egl-9 Family Hypoxia Inducible Factor 3 |
| FGF11 | ENSG00000161958 | 2256 | Fibroblast Growth Factor 11 |
| HILPDA | ENSG00000135245 | 29923 | Hypoxia Inducible Lipid Droplet Associated |
| HK2 | ENSG00000159399 | 3099 | Hexokinase 2 |
| JAM2 | ENSG00000154721 | 58494 | Junctional Adhesion Molecule 2 |
| LEP | ENSG00000174697 | 3952 | Leptin |
| MMP9 | ENSG00000100985 | 4318 | Matrix Metallopeptidase 9 |
| MT1E | ENSG00000169715 | 4493 | Metallothionein 1E |
| MT1F | ENSG00000198417 | 4494 | Metallothionein 1F |
| MT1G | ENSG00000125144 | 4495 | Metallothionein 1G |
| MT1H | ENSG00000205358 | 4496 | Metallothionein 1H |
| MT1X | ENSG00000187193 | 4501 | Metallothionein 1X |
| MT2A | ENSG00000125148 | 4502 | Metallothionein 2A |
| MT3 | ENSG00000087250 | 4504 | Metallothionein 3 |
| NDRG1 | ENSG00000104419 | 10397 | N-Myc Downstream Regulated 1 |
| NUPR1 | ENSG00000176046 | 26471 | Nuclear Protein 1, Transcriptional Regulator |
| PDGFB | ENSG00000100311 | 5155 | Platelet Derived Growth Factor Subunit B |
| PFKFB3 | ENSG00000170525 | 5209 | 6-Phosphofructo-2-Kinase/Fructose-2,6-Biphosphatase 3 |
| PLIN2 | ENSG00000147872 | 123 | Perilipin 2 |
| PLOD2 | ENSG00000152952 | 5352 | Procollagen-Lysine,2-Oxoglutarate 5-Dioxygenase 2 |
| RORA | ENSG00000069667 | 6095 | RAR Related Orphan Receptor A |
| SLC2A1 | ENSG00000117394 | 6513 | Solute Carrier Family 2 Member 1 |
| SLC2A3 | ENSG00000059804 | 6515 | Solute Carrier Family 2 Member 3 |
| SULF2 | ENSG00000196562 | 55959 | Sulfatase 2 |
| VCAN | ENSG00000038427 | 1462 | Versican |
| VEGFA | ENSG00000112715 | 7422 | Vascular Endothelial Growth Factor A |
| VLDLR | ENSG00000147852 | 7436 | Very Low Density Lipoprotein Receptor |

### MATERIALS AND METHODS

### Protocol to obtain mouse THEM, M2 and TCM macrophages

To obtain THEM (Tumor and Hypoxia Educated Macrophages) fresh monocyte isolated from dT-Tomato male mouse bone marrow were cultured in cell culture medium (IMDM) supplemented with 10% heat-inactivated fetal bovine serum, 100 U/mL penicillin/streptomycin, 2 mM L-glutamine and murine Macrophage Colony-Stimulating Factor (M-CSF) in adhesion for 6 days in normoxia condition 37°C, 5% CO₂. Then the medium was modified with a medium consisting of 50% tumor supernatant (obtained from cultures of solid fibrosarcoma and glioma explants, from which the tumor-rich supernatant was selected) and 50% IMDM and the culture was maintained under hypoxic conditions (1% O2) for the last 18 hours.

To obtain TCM (Tumor Conditioned Macrophages) fresh monocyte isolated from dT-Tomato male mouse bone marrow were cultured in cell culture medium (IMDM) supplemented with 10% heat-inactivated fetal bovine serum, 100 U/mL penicillin/streptomycin, 2 mM L-glutamine and murine Macrophage Colony-Stimulating Factor (M-CSF) in adhesion for 6 days in normoxia condition 37°C, 5% CO₂. Then the medium was modified with a medium consisting of 50% tumor supernatant (obtained from cultures of solid fibrosarcoma and glioma explants, from which the tumor-rich supernatant was selected) and 50% IMDM and the culture was maintained in normoxia for the last 18 hours before the experiment.

To obtain M2 macrophages fresh monocyte isolated from dT-Tomato male mouse bone marrow were cultured in cell culture medium (IMDM) supplemented with 10% heat-inactivated fetal bovine serum, 100 U/mL penicillin/streptomycin, 2 mM L-glutamine and murine Macrophage Colony-Stimulating Factor (M-CSF) in adhesion for 1 week in normoxia condition at 37°C, 5% CO₂. Then they were incubated at 37°C, 5% CO₂, in normoxic condition (21% O2) with IMDM medium additioned with 20 ng/mL of IL-4 for the last 18h before the experiment.

### Protocol to obtain Human THEM, TCM, M2 and M0 macrophages

To obtain THEM, blood-derived CD14+ monocytes were *in vitro* cultured with 70% culture medium (RPMI + 10%FBS+ 100ng/ml M-CSF) and 30% tumor conditioned media for 6 days in normoxia followed by 18-24h of hypoxia. Tumor conditioned media is obtained from the supernatant of tumor lines or of resections of dissociated and plated *in vitro* tumors for a period of about 12-72 hours.

To obtain TCM (Tumor Conditioned Macrophages), blood-derived CD14+ monocytes were *in vitro* cultured with 70% culture medium (RPMI + 10%FBS+ 100ng/ml M-CSF) and 30% tumor conditioned media for 7 days in normoxia.

To obtain M0 not polarized macrophages, blood-derived CD14+ monocyte were *in vitro* cultured with 100% culture medium (RPMI + 10%FBS+ 100ng/ml M-CSF) for 7 days in normoxia.

To obtain M2 macrophages, M0 macrophages were generated and then polarized with the addition of IL4 (20ng/ml) for the last 18 hours.

### Transcriptomic analysis

Total RNA was extracted from mouse or human macrophages using the RNeasy Plus Micro Kit (Qiagen, Cat No. 74034) according to the manufacturer's protocol and RNA integrity was evaluated using the Fragment Analyzer (Agilent Technologies). RNAseq library preparation was performed starting from 100ng high-quality total RNA using the "TruSeq Stranded mRNA library prep kit" (Illumina, San Diego, USA). Briefly, the mRNA fraction was purified from total RNA by polyA capture, fragmented and subjected to cDNA synthesis. Barcoded DNA adapters were ligated to both ends of the double-stranded cDNA and subj ected to PCR amplification. The library products were evaluated using Fragment Analyzer (Agilent Technologies), then sequenced on an Illumina NextSeq500 sequencer using 75bp single-end reads, generating ~17 million reads per sample. Quality control was conducted using the software Scythe (v0.991) and Sickle (v1.33). Transcript expression levels were then quantified in each sample by running the computer software Salmon v.1.0.0 against the reference sequence of the mouse transcriptome (Gencode M23-GRCm38).

Differential expression analysis on sequence count data was performed using negative binomial distribution models as implemented in the library DESeq2 of R software (https://www.r-project.org/), using the Benjamini-Hochberg method to adjust the p-values for multiple comparisons. Transcript expression fold-changes were estimated for each expressed transcript in every comparison. The most relevant up-regulated genes were then summarized into biological processes and molecular functions by an enrichment analysis based on gene ontologies as implemented in the library dnet of R.

### Animal model of severe contusive spinal cord injury

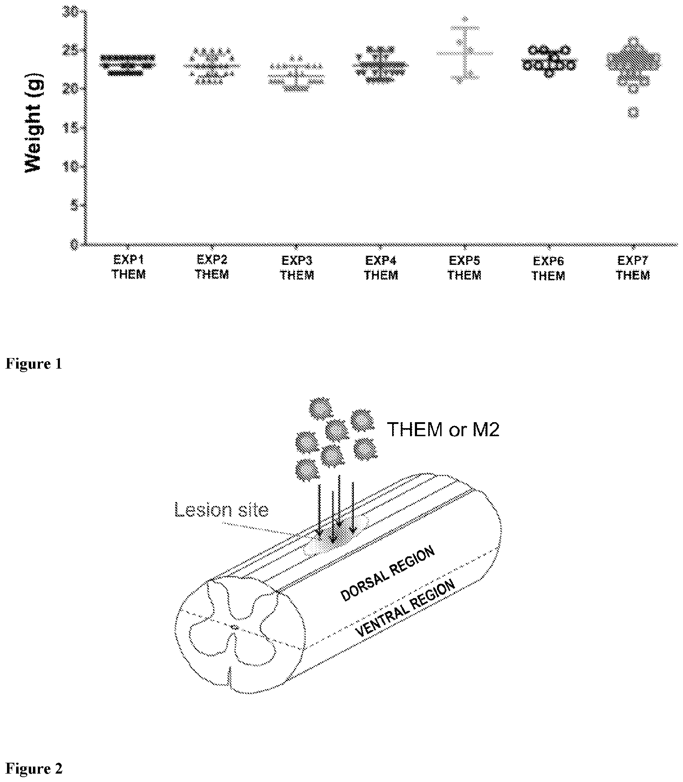

Male adult (7 week old) C57BL/6J mice purchased from Charles River were subjected to laminectomy and then severe contusive spinal cord injury at the level of the thoracic vertebra 11 (T11) as previously described (15, 16). Briefly, seven-week-old C57BL/6 male mice were anesthetized with 2% isoflurane, and laminectomy was performed at T11 level. A 5-gr rod was dropped from 6,25 mm height using a MASCIS Impactor and left in compression for 11 seconds. Subcutaneous injections of Baytril (25 mg/ml) and Rimadyl (50 mg/ml) were provided daily the first 7 days post injury (dpi). Animal care was performed daily all experiment long and included animals weight once a day and bladder emptying twice a day until 5 dpi and then once a day till the end of the experiment. Prior to performing the surgeries, all the animals were weighed and only the mice within a weight range between 17 and 31g were considered for the experiment and subjected to surgery. The weight of the animals at the time of surgery is shown in Figure 1. The surgical lesion and cellular transplantation resulted in the death of 26 out of 142 animals due to the severity of the procedures applied or due to complications during the recovery period.

### THEM, M2 and TCM macrophages transplantation protocol in the severe spinal injury model

The day of transplantation, THEM, TCM and M2 macrophages in culture were removed from the plates using Acutase, centrifuged to remove the supernatant and resuspended in saline at a concentration of 0.5*10⁶ cells/µl. For each transplantation session, each experimental animal belonging to the THEM, TCM, M2 or vehicle group received 4 injections (1 µl/injection) at 4 different points, separated from each other by 2 mm in the dorsal column (injection depth: 0.5 mm) in the spinal cord section centred on the lesion (Figure 2). The cell transplantation was performed at a rate of 0.5 µl/minute using a glass capillary connected with a microinjector and a stereotaxic apparatus. After each injection, the glass capillary was kept in place for 5 minutes to minimize the reflux of the cell suspension as much as possible. After 5 minutes, the dorsal wound was sutured and disinfected, and 1 mL of saline was administered subcutaneously. At the end of all the procedures, the animal was placed on a heating mat and monitored until complete awakening.

### Study design 1 evaluation of THEM efficacy

Once subjected to severe contusive spinal injury, the animals were divided into 3 experimental groups: a first group was subjected to multiple THEM transplantation, a second group was subjected to multiple M2 macrophage transplantation and finally a third group was subjected to sterile saline injections. Before subdividing the animals into the three experimental groups, on the day of the first transplant (3 days after the contusive spinal lesion, 3 dpi), the locomotor performance of the animals was assessed and only the animals with a severe spinal injury which resulted in a value between 0 and 0.5 in the Basso Mouse Scale analysis (BMS) were used for the next phase of the experimental plan. 3 or 4 transplants/saline injections were performed (time points 3 transplants: 3 dpi, 10 dpi, 17 dpi; time points 4 transplants: 3 dpi, 10 dpi, 17 dpi, and 24 dpi). The experiments ended 31 days after the spinal injury (31 dpi). At the end of the trial, the functional recovery values and histological features were evaluated in the experimental groups considered.

### Study design 2 Evaluation of THEM efficacy compared to TCM

Once subjected to severe contusive spinal injury, the animals were divided into 3 experimental groups: a first group was subjected to multiple THEM transplantation, a second group was subjected to multiple TCM macrophage transplantation and finally a third group was subjected to sterile saline injections. Before subdividing the animals into the three experimental groups, on the day of the first transplant (3 days after the contusive spinal lesion, 3 dpi), the locomotor performance of the animals was assessed and only the animals with a severe spinal injury which resulted in a value between 0 and 0.5 in the Basso Mouse Scale analysis (BMS) were used for the next phase of the experimental plan. 3 macrophages or saline injections were performed (time points 3 transplants: 3 dpi, 10 dpi, 17 dpi). The experiments ended 31 days after the spinal injury (31 dpi). At the end of the trial, the functional recovery values were evaluated in the experimental group considered.

### Locomotor evaluations

### Locomotor evaluations in open-field

The locomotor function of THEM-transplanted, M2-transplanted and Vehicle mice was evaluated at 1, 3, 4, 5, 7, 10, 11, 14, 17, 18, 21, 24, 28 and 31 dpi by using the Basso Mouse Scale (BMS) (14). The Basso Mouse Scale comprise 10 stages from "0" to "9", where "0" correspond to "no movement" and "9" to a normal locomotor functionality.

### Ankle flexibility analysis

The ankle joint flexibility analysis was performed from 14 DPI to evaluate the spasticity of the muscles involved in the ankle dorsiflexors and plantarflexors as previously described (16, 17). The following scores were assigned: "0" to the lack of movement (spastic condition, corresponding to an angle of 180°between the tibialis anterior and the paw), "0.25" to an angle of 135°, "0.5" to an angle of 90°, "0.75" to an angle of 45°, and "1" to a normal movement corresponding to an angle of 0°.

### Electromyography

*In vivo* electromyographic (EMG) recording of spontaneous muscle activity was performed in tibialis anterior (TA) and lateral gastrocnemius (LG) muscles. In total inventors recorded 32 TA muscles from 17 mice, and 29 LA muscles from 16 mice. In each animal, the recordings of different muscles were performed in sequence (i.e. not simultaneously). In anesthetized (isoflurane 2%) animals inventors inserted, through short skin incisions, two varnished-insulated 125 µm-thick stainless steel wires per muscle, with bare tips parallel to the longitudinal axis of the muscle and connected to the amplifier (CyberAmp 320, Axon Instruments, Foster City, CA). As reference electrode, inventors inserted a cut short 20G stainless steel needle under the skin in the back of the animal and connected it to ground. To maximize EMG selectivity, inventors recorded differentially and adjacent muscles were either denervated or their fibres cut transversally. EMG signal was 5000x amplified, 100-1200 Hz band pass filtered, further improved with hum pick-up suppression (Hum-Bug, Quest Scientific, Vancouver, Canada), digitized at 10KHz and acquired with Signal software (6.0.2, Cambridge Electronic Design, Cambridge, U.K.). To reduce movement artifacts, the animal was contained in ventral decubitus with tapes across the body, the limbs and the tail. Recording began 10 min after full recovery from anesthesia, and lasted 3 min for each muscle, during which time the animal was stimulated touching its whiskers, forelimbs and hindlimbs every 10-15 sec. The animal was then re-anesthetized in order to cut either the sciatic nerve bilaterally or the spinal cord at T10 (i.e. cranially to SCI level). A second session of EMG recording was then initiated 10 min after full recovery from anesthesia, and lasted 1 min for each muscle. At the end of the experiment the animal was perfused. EMG analysis (Signal and Spike2, 5.0.9, Cambridge Electronic Design) consisted of: i) quantification of the power of the electrical signal during contractions by measuring its root-mean-square (RMS) value and ii) quantification of the duration of the electrical signal during contractions. To measure RMS, every EMG trace was divided into 40 ms-long sections and RMS calculated in each section with a time constant of 10 ms. Inventors considered as RMS related to muscular activity those values above a cutoff level which was determined in each muscle as the average + 1.9 standard deviation RMS value after sciatic nerve or spinal cord section. Finally, inventors extracted RMS values corresponding to periods of strong muscular activity, by averaging the 10 largest RMS among all the measured values, irrespectively of their position along the trace.

### Histochemistry and immunofluorescence analysis

### Spinal cord fixation and processing

Animals were intracardially perfused with 4% paraformaldehyde (PFA) and 4% sucrose. Spinal cords were extracted, immersed overnight in 4% PFA, 4% sucrose, and stored in 30% sucrose at 4°C. For histochemical analysis, 1.5 cm of dissected spinal cords (0.75 cm rostral and 0.75 cm caudal from the site of the lesion) were cryosectioned (25 µm-thick transverse sections or 20 µm-thick longitudinal sections) and stored at -20°C before analysis.

### Luxol Fast Blue Staining

Myelin content was quantified in the spinal cord sections via by Luxol Fast Blue (LFB) staining (Sigma-Aldrich, Cat#L0294) as described (16, 18). Briefly, 0.1% LFB solution was prepared solubilizing LFB (Sigma-Aldrich) in 95% ethanol (EtOH, Carlo Erba) and 1.22% glacial acetic acid (Carlo Erba). Sections were hydrated in EtOH solutions (100, 95, 70, and 50%), followed by staining with 0.1% LFB solution at 40°C for 40 min. Sections were then rinsed with tap water and differentiated in 0.05% Li2CO3 solution (Sigma-Aldrich). Sections were dehydrated in EtOH solutions (50, 70, 95, and 100%), cleared in xylene (Carlo Erba) and mounted with Entellan (Merck-Millipore) for light microscopy analysis of myelin content (Zeiss Axioscop 2).

### Thiols staining

Mice were first transcardially perfused with a solution composed by PBS 1X added with 1.85% of iodoacetate and 1.25% of N-ethylmaleimide, and then with a solution containing 4% PFA, 1.85% iodoacetate and 1.25% N-ethylmaleimide. Spinal cords were extracted and left in 4% PFA O/N. After cryosectioning, samples were washed in PBS 1X for 10 min and incubated for 1 hour in a solution of 4 mM TCEP (Tris (2 carboxyethyl) phosphine hydrochloride) in PBS 1X. Samples were then incubated for 30 min in a solution of 0.1% CPM (7- Diethylamino-3-(4'-Maleimidylphenyl) -4-Methylcoumarin) in PBS 1X. After a wash in PBS 1X, samples were mounted with DABCO. After staining, slices were acquired with a fluorescence microscope and the hypoxic areas were as fluorescent blue spots. The areas were then quantified by threshold with ImageJ software [U.S.National Institutes of Health].

### Tissue immunofluorescence

Immunofluorescence were performed as previously described (16, 19). Briefly cryosections were permeabilized with 0.25%/0.5% Triton X-100, 2% BSA, and incubated with primary antibodies in blocking solution overnight at 4 °C. After rinsing 6 times for 5 min in blocking solution, appropriate secondary antibodies were applied for 4 h at room temperature. After final washing steps in blocking solution and then in PBS 1X, nuclear staining with TOPRO^{™}-3 (1:3000, Invitrogen Thermo Fisher Scientific) or 4'.6-Diamidino-2-Phenylindole (DAPI, 1:2000, Thermo Fisher Scientific, Cat#D-1306) was performed and slides were mounted using 1.4-Diazabicyclo (2.2.2) octane (DABCO, Sigma Aldrich, Cat#D-2522). Images were acquired using a 40x oil objective (Carl Zeiss LSM710 confocal microscope, Munich, Germany), and a 20x objective (Nikon Ti Eclipse fluorescent microscope). The following primary antibodies per used for immunofluorescence analysis: TOMM20 (mouse, 1:200, Abcam, Cat#AB56783), Neurofilament-200 (rabbit, 1:200, Sigma, Cat#N4142), NeuN (mouse, 1:200, Millipore, Cat#MAB377), anti-Choline Acetyltransferase Antibody ChAT (goat, 1:200, Millipore, Cat#AB144P), Ionized-calcium binding adaptor molecule 1 IBA1 (rabbit, 1:200, WAKO, Cat#019-19741), Cluster of Differentiation 206 CD206 (goat, 1:400, R&D system, Cat#AF2535,), Agrin (mouse, 1:200, Stressgen, Cat#AGR-520), collagen-I antibody (rabbit, 1.200, Abcam, Cat#AB34710), collagen-III antibody (mouse, 1.200 GeneTex, Cat#GTX26310), CD31 (Platelet endothelial cell adhesion molecule, PECAM-1) antibody (rat, 1:200, BD Biosciences, Cat#557355), Fibronectin (rabbit, 1:200, Dako, Cat#A0245), CD68 (rat, 1:200, Thermo Fischer Scientific, Cat#14-0681-82), GFAP (goat, 1:200, Abcam, Cat#ab53554). Appropriate secondary antibodies were used: donkey anti-mouse Alexa Fluor 488 (1:500, Thermo Fisher Scientific, Cat#A2120), donkey anti-rabbit Alexa Fluor 488 (1:500, Thermo Fisher Scientific, Cat#A21206), donkey anti-goat Alexa Fluor 488 (1:500, Jackson, Cat#AB - 2340428), donkey anti-rat Alexa Fluor 488 (1:500, Thermo Fischer Scientific, Cat#A11006), goat anti-mouse CY3 (goat, 1:500, Amersham, Cat#PA43002), donkey anti-rabbit Alexa Fluor 546 (donkey, 1:500, Thermo Fisher Scientific, Cat#A10040), donkey anti-goat Alexa Fluor 546 (donkey, 1:500, Invitrogen by Thermo Fisher Scientific, Cat#A-11056), donkey anti-mouse Alexa Fluor 647 (1:500, Thermo Fischer Scientific, Cat#A32787), donkey anti-rabbit Alexa Fluor 647 (1:500, Thermo Fischer Scientific, Cat#A32795), TO-PRO^{™}-3 (1:3000, Thermo Fisher Scientific) or DAPI (1:2000, Thermo Fisher Scientific).

### Image analysis and quantification

The quantitative analysis was done with NIH ImageJ software [U.S.National Institutes of Health]transversal sections and longitudinal sections of the spinal cord were analyzed in at least 3-5 technical replicates (glass slides). For the analysis normalized to the number of total nuclei, approximately 5115 ± 87 nuclei were considered. Three to five mice were considered for each analysis.

### Morphometric blood vessels (CD31) analysis

Spinal cord transversal section images were analysed using a custom-designed plugin run with FIJI 1.52p (U.S. National Institutes of Health). In each section, four regions of interest (ROI) corresponding to the dorsal left horn, the dorsal right horn, the ventral right horn and the ventral left horn were selected. The brightness and contrast of the images were adjusted, and the images were converted into binary images. A morphological analysis using "MorphoLibJ" (Legland, Arganda-Carreras & Andrey, 2016) was applied in order to close small gaps inside the same vessel. The images were then skeletonized and the skeletons were analysed using the plugin "Analyze Skeleton (2D/3D)". For the analysis the following parameters were considered: number of grouped vessels, average number of branches, mean length of the branches inside a single group of vessels, mean maximum branch length (i.e. the length of the longest branch of each group of vessels inside the ROI), average branch length of each group of vessels, Euclidean distance (the ratio between the length of the vessel and the euclidean distance gives a measure of the tortuosity of the vessel) and the number of total branches inside each field of interest.

### Transplanted macrophages distribution analysis

A portion of 0.5cm of tissue centred on the cyst was considered for transplanted THEM or M2 distribution analysis. Spinal cord parenchyma of injured mice that received three multiple transplantations (2x10⁶ cells) were analysed. Four to nine transversal sections (25µm) of the spinal cord of mice that received multiple transplantations were analysed in at least eight technical replicates (glass slides) on five animals. The slices were stained with 4'.6-Diamidino-2-Phenylindole (DAPI, Thermo Fisher Scientific, 1:2000) in order to identify the cell nuclei. The number of -tomato and DAPI double positive cells was manually counted with FIJI ImageJ 1.52p (U.S National Institute of Health).

### Human induced pluripotent stem cells (iPSCs)derived motor neuron co-culture with macrophages

iPSCs were obtained reprogramming skin biopsy-derived fibroblasts of three healthy donors with previously written informed consent from the participants as previously described (15, 16). Then the cells were differentiated into motor neurons as previously described (15). Human or mouse macrophages were co-cultured with differentiating iPSCs for 2 days, then the cells were fixed with 4% paraformaldehyde for immunofluorescence analysis. Immunofluorescence was performed by staining with anti-β3Tubulin (mouse, 1:400, Promega, Cat#G7121), donkey anti-mouse Alexa Fluor 488 (1:1000) antibodies as previously described (15) Images were acquired using a 20x objective (Nikon Ti Eclipse fluorescent microscope). For each glass slides, five fields were acquired and for each field 2x2 large images with a 20x objective were acquired.

### Statistical Analysis

Unless otherwise stated, n ≥ 3 samples or replicates were used for the statistical analysis. GraphPad Prism software (GraphPad Inc., La Jolla, CA, version 7.0) was used to perform unpaired two-sided Student's t-test, ordinary one-way or two-way analysis of variance (ANOVA) with repeated measures followed by Tukey post-test. Data are shown as mean ± SEM and statistical significance was set at p < 0.05.

****p < 0.0001; ***p < 0.001; **p < 0.01; *p < 0.05; ns not statistically significant

### RESULTS

### Mouse THEM and M2 macrophages show completely distinct molecular signature

In order to characterize the molecular signature of mouse THEM macrophages inventors analysed their whole transcriptomic profile and compared it with the transcriptome of the M2 macrophages. 3 samples were analyzed for each experimental group. The Principal Component Analysis (PCA) showed that THEM and M2 macrophages clustered in different groups confirming that they have completely distinct phenotypes (Figure 3). As expected M2 macrophages expressed high level of CD206, CD163, and Arg1 genes. The transcriptomic analysis of the secretome and recettome transcripts (See Table 1 and Table 2) also indicated that the most significant up-regulated genes in the THEM population were related to the functional categories of Gene Ontology related to angiogenesis (e.g., VEGFs and VEGFc) and extracellular matrix remodeling (Mmp8, Mmp9, Mmp10, Mmp12, Mmp14, Mmp19) (Figure 3). Overall these results showed that THEM have a specific molecular identity different from M2 macrophages.

### THEM improve motor abilities in a mouse model of severe SCI

### THEM promote motor recovery

In order to evaluate THEM *in vivo* regenerative potential, THEM, M2 or vehicle were injected in severe SCI mice as described in material and methods (Study design 1). The BMS evaluation was performed to evaluate the effect of the THEM and M2 transplantation on locomotor function recovery following severe contusive spinal cord injury. The locomotor performance of each animal was assessed at 1, 3, 7 dpi during the first week following the injury, and then twice a week (10, 14, 17, 18, 21, 24, 28, 31 dpi) until the end of the experiment (31 dpi.) As shown in Figure 4, the BMS value of the animals subjected to multiple THEM transplantation (1.792 ± 0.327) was significantly higher compared to that of the animals subjected to multiple M2 transplantation (0.375 ± 0.109) and to the animals injected with vehicle (0.712 ± 0.157). The statistical analysis showed a significant difference between the animals subjected to multiple THEM transplantation and vehicles in the BMS values at the time points 14, 17, 18, 21, 24, 28 and 31 dpi. Similarly, a significant difference between animals subjected to multiple THEM transplantation and animals subjected to multiple M2 macrophage transplantation was observed in the BMS values at the time points 17, 18, 21, 24, 28 and 31 dpi. These results suggested that only THEM are effective to significantly improve motor recovery of severe SCI mice compare to vehicle, while M2 didn't show any effect.

### THEM promote ankle flexibility

Several studies have shown that "spasticity" is one of the most common symptoms which develops as a result of spinal cord injury and this phenomenon is one of the main causes of disability in individuals with diseases affecting the central nervous system, such as spinal cord injury [4]. In order to determine whether the cellular transplant was able to prevent the onset of a spasticity condition, the flexibility performance relative to the ankle joint was evaluated. This condition was monitored daily from 15 dpi until the end of the experiment (31 dpi).

As shown in Figure 5, the ankle joint flexibility is significantly better in the animals subjected to multiple THEM transplantation (0.799 ± 0.037) with respect to that of the animals subjected to multiple M2 transplantation (0.659 ± 0.049) and that of the control animals (0.612 ± 0.045), suggesting faster recovery of muscle spasticity following spinal injury. The statistical analysis showed a significant difference between animals subjected to multiple THEM transplantation and vehicles in the ankle joint flexibility values at the time points 15, 21, 22, 26, 27, 28, 29, 30, and 31 dpi. Furthermore, as shown in Figure 5, the statistical analysis showed a significant difference in ankle flexibility values between animals subjected to multiple THEM transplantation and animals subjected to multiple M2 macrophage transplantation at the time point 28 dpi.

### THEMs restore the normal excitability of the flexor motor neurons

In order to verify the data obtained from the previous functional analyses, electromyographic (EMG) recordings of spontaneous potentials were carried out *in vivo* relative to the anterior tibial and lateral gastrocnemius muscles. Both the amplitude and duration of the electromyographic signals during muscle contraction were taken into consideration. The amplitude of the electromyographic traces is proportional to the number of activated motor neurons: both the transplanted and control animals showed electromyographic amplitudes reduced by about 50% with respect to the healthy animals. The durations of activation of the electromyographic traces are proportional to the excitability of the motor neurons which is increased following spinal cord injury (in particular the durations of activation of the electromyographic traces of the control animals are increased by 2.5 times).

The increased excitability of the motor neurons after spinal injury is due to the loss of functionality of both large-scale and local inhibitory circuits. In the animals subjected to multiple THEM transplantation, the duration of electromyographic activation of the lateral gastrocnemius muscle is statistically shorter (0.338s ± 0.048s) compared to that of the control animals (0.593s ± 0.101s), which is closer to the physiological condition of the muscle in healthy animals (0.231s ±0.038s) (Figure 6A). Also with regard to the anterior tibial muscle, the electromyographic activation durations in the animals subjected to multiple THEM transplantation were smaller (0.416s ± 0.058s) compare to that of the control animals (0.450s ± 0.077s). The healthy animals had a statistically shorter electromyographic anterior tibial muscle activation duration with respect to all the other experimental groups (0.161s ± 0.020s) (Figure 6B). These data indicated that the treatment with THEM restores the normal excitability of the flexor motor neurons, although it has no effect in terms of re-innervation.

### THEM promote the regeneration of severe SCI mice spinal cord tissue

Traumatic events which occur at the level of the spinal cord cause mechanical damage and consequent tissue degeneration. These events include: degradation of the cell membrane of axons, degeneration of the blood-brain barrier, demyelination, migration of immune cells, and death of spinal parenchymal cells [2, 5]. To study how THEM transplantation contributes to tissue regeneration of the spinal cord, the distribution of cells in the spinal parenchyma at 31 dpi and the histological features related to cyst formation, glial scar, number of neurons and in particular of cholinergic neurons (motor neurons), myelin content, activation of the immune response, extracellular matrix and tissue vascularization were first studied.

### Transplanted THEM distribution in spinal cord tissue

In order to assess the persistence of the transplanted cells, their viability and location, the distribution of the transplanted cells at the end of the experiment (31 dpi) was analyzed. The number of THEMs and M2s was quantified in spinal cord cross-sections following severe spinal cord injury and multiple transplantation representing a 0.6 cm long spinal region (9 spinal cord/animal cross-sections) centred at the site of the spinal injury. For the analysis, the absolute number of fluorescent red cells (dT-Tomato; THEM or M2) co-localizing with the nuclear marker 4',6-diamino-2-phenylindole (DAPI) was considered. The distribution of the THEMs was analyzed based on their different location in the spinal tissue (parenchyma, cyst area, dorsal/ventral meninges). As shown in Figure 7A-B, the THEMs and M2 macrophages persist in the spinal parenchyma even in the days following transplantation, and their presence is detected not only in the section of the spinal lesion but also in the neighboring sections. Therefore, the diffusion of the transplanted cells (THEM and M2 macrophages) is not only radial with respect to the transplantation site, but also longitudinal in the rostral and caudal direction. The percentage values were normalized on the total THEM or M2 cells present in the spinal cord cross-sections representing a marrow region equal to 0.6 cm in length (9 spinal cord/animal cross-sections). In the section corresponding to spinal trauma, although they are concentrated in the cyst area (53.54% ± 10.40% THEM, n=5; 53.10% ± 10.95% M2, n=3), a significant percentage is widespread in the surrounding parenchyma (46.46% ± 10.40% THEM, n=5; 46.90% ± 10.95% M2, n=3) (Figure 7 C). The percentage values were normalized over the total THEM or M2 cells in the spinal trauma section. Figure 7 D depicts the radial and longitudinal distribution diagram of the transplanted cells in relation to the spinal trauma site. Overall, these data suggested that the transplanted cells (THEM and M2 macrophages) are capable of surviving for days in the hostile environment established following spinal injury.

### THEMs reduce cyst and glial scar

Following a spinal injury, the formation of the glial scar and cyst at the injury site constitutes a compensatory mechanism which contributes to limiting the secondary injury resulting from the trauma [2]. Nevertheless, their presence prevents axonal regeneration, as they constitute a physical and molecular barrier [20]. Considering the importance of the glial cell response following spinal injury in functional recovery, immunofluorescence analyses using the astrocyte-specific marker Glia Acid Fibrillary Protein (GFAP) were performed to assess the effect of multiple THEM transplantation on glial scar formation and activation of the astrocyte response (Figure 8A-D). Spinal cord cross-sections representing a marrow region 0.6 cm in length (9 spinal cord/animal cross-sections) centred at the site of the spinal injury were analyzed. The area of the cyst compared to the total area of the glial scar is smaller in animals subjected to multiple THEM transplantation (21.38% ± 2.05%, n=3) with respect to the control animals (33.24% ± 0.87%, n=3) (Figure 8E). Furthermore, the glial scar generated by the astrocytes, delimiting the lesion area, is of equal size in the experimental groups analyzed (THEM: 6.79% ± 0.85%, n=3) with respect to the controls (6.69% ± 0.53%, n=3) (Figure 8F). The animals subjected to multiple THEM transplantation have a significantly smaller cyst area (2.03% ± 0.30%, n=3) compared to that of the controls (4.73% ± 0.15%, n=3) with respect to the area of the total section (Figure 9). These data in line with the increase tissue regeneration/limitation of degenerative mechanisms at the site of injury in animals subjected to multiple THEM transplantation.

### THEMs increase the neuronal and myelin content

Tissue degeneration of the spinal cord due to primary mechanical injury is followed by secondary damage resulting in a further loss of neuronal cells [5]. In order to assess the effect of multiple THEM transplantation on neuronal content following contusive spinal injury, immunofluorescence analyses were performed using the neuron-specific marker NeuN (Figure 10A-B). Spinal cord cross-sections representing a narrow region 1.5 cm in length (8 spinal cord/animal cross-sections) centred at the site of the spinal injury were analyzed. The percentage of NeuN+ cells was initially quantified. As shown in Figure 10C, the animals subjected to multiple THEM transplantation have a higher percentage of neurons (13.13% ± 0.632%, n=5) with respect to the control animals (6.81% ± 0.726%, n=4). Several neuronal populations are present in the parenchyma of the spinal cord. In particular, there are motor neurons which are specifically located at the level of lamina IX in the ventral region of the spinal cord [21]. In order to evaluate the effect of multiple THEM transplantation on motor neuron content following contusive spinal cord injury, immunofluorescence analyses were performed using the specific cholinergic neuron marker correlated with choline-acetyltransferase expression, ChAT (Figure 11). Spinal cord cross-sections representing a narrow region 1.5 cm in length (8 spinal cord/animal cross-sections) centred at the site of the spinal injury were analyzed. Also with regard to the percentage of ChAT+ cells, the animals subjected to multiple THEM transplantation have a higher percentage of motor neurons (0.412% ± 0.011%, n=4) with respect to the controls (0.245% ± 0.038%, n=4) (Figure 11C). The structural proteins most expressed at the axon level in mature neurons are neurofilaments (NFs). Their expression is closely related to axonal growth and the maintenance of neuronal homeostasis [22]. As a result of spinal cord injury, neuronal loss is observed at the level of the gray matter, accompanied by the loss of neurofilament proteins and myelin [22]. In order to assess the effect of multiple THEM transplantation on axonal regeneration, immunofluorescence analyses were performed using the specific marker for neurofilmanent protein NF200 (Figure 12A-B). Longitudinal sections of spinal cord representing a 3 mm wide marrow region (5 longitudinal sections of spinal/animal marrow) centred at the site of the spinal injury were analyzed. The animals subjected to multiple THEM transplantation had a significantly higher NF200 content (16.360% ± 2.090%, n=3) with respect to the controls (9.723% ± 0.133%; n=3) (Figure 12C). The phenomenon of demyelination causes resulting from a spinal injury cause significant consequences, contributing to the functional loss of nervous tissue [5]. In order to assess the effect of multiple THEM transplantation on spinal parenchyma remyelination, histological analyses were performed with Luxol Fast Blue (LFB), a specific dye for myelin detection (Figure 13A-B). Spinal cord cross-sections representing a narrow region 0.6 cm in length (8 spinal cord/animal cross-sections) centred at the site of the spinal injury were analyzed. As shown in Figure 13C, the animals subjected to multiple THEM transplantation have significantly higher myelin content (26.470% ±1.075%, n=5) with respect to the controls (19.870% ± 1.999%, n=3).

### THEMs increase the endogenous pro-regenerative immune response

The cascade of events resulting in spinal damage determines the propagation of the inflammatory response also in the tissues adjacent to the site of the spinal mechanical damage with negative and positive consequences on the regeneration of the medullary tissue [2, 5, 6]. In particular, considering the crucial role of macrophages in the innate immune response and their different functions dependent on their phenotypic polarization (M1 - pro-inflammatory macrophages; M2 - pro-regenerative macrophages), inventors evaluated the endogenous macrophage component and their relative phenotype. The most commonly used marker for the evaluation of the content in cells belonging to the microglia and lineage of monocyte/macrophage subpopulations is the molecule Ionized calcium-binding adapter molecule 1 (Iba1) [23] while the specific markers CD68 and CD206 were used for the evaluation of the macrophages and their M2 phenotype, respectively. In fact, the marker CD68 is linked to the expression by macrophages and other mononuclear phagocytes of a highly glycosylated type 1 transmembrane glycoprotein [24], while the marker CD206 is related to the expression of a mannose receptor present mainly on the surface of M2 macrophages [6].

In order to evaluate the effect of multiple THEM transplantation on the inflammatory response involved in severe spinal lesion patho-physiology, inventors then performed immunofluorescence analyses using the marker Iba1 (Figure 14A-B). To further investigate the macrophage component and its phenotype, inventors subsequently performed immunofluorescence analyses using the markers CD68 and CD206, respectively (Figure 15A-D). In the analysis, spinal cord cross-sections representing a narrow region 0.6 cm long (8 spinal cord/animal cross-sections) centred at the site of the spinal injury were considered. Figure 14C shows how after severe spinal injury, the percentage of microglia cells present in the spinal cord parenchyma increases in the spinal parenchyma of the animals subjected to multiple THEM transplantation with respect to the controls (5.820% ± 0.411%, n=5 animals subjected to THEM transplantation, 3.847% ± 0.597%, n=3 controls). Furthermore, the in-depth analysis of the macrophage component and its phenotype showed that the percentage of total macrophages is higher in the controls compare to the animals subjected to multiple THEM transplantation (5.484% ± 0.367%, n=5 animals subjected to THEM transplantation, 6.143% ± 0.554%, n=3 controls) (Figure 15E), however the M2 component is significantly higher (5.655% ± 0.497%, n=4 animals subjected to THEM transplantation, 2.320% ± 0.080%, n=3 controls) (Figure 15F).

These results suggested that THEM transplantation increase the percentage of pro-regenerative endogenous M2 macrophages of the SCI mice spinal cord.

### THEMs induce extracellular matrix remodelling

One of the main components of the extracellular environment is the extracellular matrix (ECM). It is characterized by the concentration of molecules which have a double effect on neuronal growth by promoting or inhibiting it. Following spinal trauma, an increase in the dissolved concentration of these molecules in the damaged area is observed, including fibronectin, agrin, collagen, and proteoglycans [2, 5, 20, 25]. Among the inhibitory molecules, the chondroitin-sulfate proteoglycan (CSPG) superfamily is the most represented and comprises several categories of molecules which influence axonal development and regeneration such as Neurocan and Aggrecan [20, 25]. Although in physiological situations some of these molecules do not involve any inhibitory phenomenon, following a trauma their accumulation at the level of the cyst and glial scar contributes to the formation of structures which prevent axonal regeneration [25]. Several processes are activated at the level of the injury to try to curb this phenomenon. A key role is played by metalloproteases, molecules well known for their role in ECM remodelling [26].

In order to evaluate the effect of multiple THEM transplantation on ECM remodelling following severe spinal injury, inventors performed immunofluorescence analyses using specific markers correlated with the major components of the ECM: Agrin and Fibronectin (Figure 16A-B). Cross-sections and longitudinal sections of spinal cord (at least 1 longitudinal/transverse section of spinal cord/animal) centred at the site of the spinal injury were analyzed. Morphometric analyses were performed to assess the content of Agrin and Fibronectin, assessing the extent of the positive areas (number of pixels) related to the respective areas of the cyst. Figure 16C-D shows how after severe spinal injury, the percentage of Agrin and Fibronectin molecules present at the cyst level decreases in the animals subjected to multiple THEM transplantations with respect to the controls (Agrin: 3.500% ± 1.112%, n=3 animals subjected to transplantation with THEM, 12.570% ± 2.140% control animals, n=3; Fibronectin: 5.190% ± 2.019%, n=3 animals subjected to transplantation with THEM, control animals 9.625% ± 3.375%, n=3).

### THEMs reduce hypoxia and induce angiogenesis

The spinal cord is characterized by a particularly complex vascular system, such that damage to major vessels or the alteration of blood flow regulation can result in the reduction or temporary blockage of tissue perfusion. Traumatic spinal injury can lead to the immediate cessation of tissue perfusion with long-term consequences which lead to chronic hypoxia and result in modification of the tissue redox state, essential for the normal function of physiology and cellular metabolism, and consequently neurodegeneration [5, 27]. Considering the results obtained from the transcriptome analysis experiment, and specifically the significant THEM upregulation of genes involved in vasculogenesis, inventors analyzed the morphology of the vessels and the status of possible oxygen deficiency (hypoxia) in the spinal cord parenchyma in animals subjected to THEM multiple transplantations and in control animals.

For the evaluation of hypoxia, free thiols were analyzed based on the use of N-ethylamide (NEM) and iodoacetic acid (IAA) for the alkylation of the thiols (Figure 17A-B). In fact, the thiols bound to protein cysteines are the groups most sensitive to fluctuations in cellular redox status. Under physiological conditions, the cytoplasm is characterized by a highly reducing condition capable of keeping most protein thiols in a reduced state. However, conditions of excessive oxidative stress can lead to the formation of molecular species which determine an irreversible oxidation of protein thiols, glutathione depletion and cell death. For the analysis of the hypoxic areas, spinal cord cross-sections representing a narrow region 1.5 cm long (at least 8 spinal cord/animal cross-sections) centred at the site of the spinal injury were considered. Figure 17C shows how after severe spinal injury, the percentage of hypoxic area decreases in the spinal cord parenchyma of the animals subjected to multiple THEM transplantation with respect to the vehicles. (3.173% ± 0.528%, n=3; animals subjected to THEM transplantation; 5.866% ± 0.745%, n=3 controls).

To evaluate the morphology of the vessels, inventors performed immunofluorescence analyses using the marker specific for endothelial cells, CD31 (Halder et al., 2019) (Figure 18 A-B). For the analysis of vessel morphology, spinal cord cross-sections representing a 0.5 cm long spinal region (at least 4 spinal cord/animal cross-sections) centred at the site of the spinal injury were considered. On the cross-sections considered, 5 areas of interest (ROI) were then selected on which the morphology analysis was carried out (number of vessels/field, average number of branches/vessel, maximum length of the vessels; tortuosity of the vessels). The vessel tortuosity value was calculated as the ratio of the total average length of the vessels in relation to the total average value of the Euclidean distance of all the branches. As shown in Figure 18C, the morphological analyses indicated a greater number of vessels in the spinal parenchyma of the control animals (37.070 ± 0.379, n=3) with respect to the animals subjected to multiple THEM transplantation (30.940 ± 3.592, n=3); however, the latter have vessels with a greater number of branches (2.110 ± 0.165 animals subjected to THEM transplantation, n=3; 1.793 ± 0.073 controls, n=3) (Figure 18D), and a significantly longer maximum length (55.410 ± 2.500 animals subjected to THEM transplantation, n=3, 43.450 ± 0.751 controls, n=3) (Figure 18E). The tortuosity value shows no difference between the two experimental groups considered (1.168 ± 0.005 animals subjected to THEM transplantation, n=3, 1.153 ± 0.015 controls, n=3), Figure 18F. These data suggested that THEM restored the vasculature by increasing vessel length and branching.

### In vitro regenerative effect of mouse THEM on human motor neurons

The data shown indicate that *in vivo* multiple THEM transplantation results in a pro-regenerative/protective effect of the spinal parenchyma on various fronts (at the neuronal level, the glial scar, the myelin component), suggesting that the effect of the transplanted cells is not limited to a single specific cell type. Considering the translational potential of the treatment under analysis, and in order to evaluate whether the effect of the THEMs observed *in vivo* was also applicable on human neuronal cells, inventors evaluated the effect of THEMs and M2 macrophages on cultures of human pluripotent stem cells differentiated into motor neurons. During the final phase of neuronal differentiation, THEMs or M2s were added to the cultures for 2 days. Once the differentiation phase was completed, the expression area of the neuronal specific marker βIII Tubulin (Tub3), was evaluated as an indicator of the number of dendrites and their extension (40 fields/donor, 4 images/field, n=3) (Figure 19A-C). As shown in Figure 19D, the area of expression of Tub3 is significantly greater in the co-cultures with THEMs (9.097% ± 0.253%) with respect to those with M2 macrophages (7.235% ± 0.492%) and the control vehicle cultures (6.423 ± 0.494). These results suggested that THEM promote greater neuronal differentiation and greater extension of dendrites of human motor neurons compare to M2 macrophages and control vehicles.

### Human THEMs show a unique molecular identity

Inventors performed whole transcriptome analysis to define the THEM specific molecular signature and to identify the role of hypoxia in THEM signature specification. They compared the whole gene expression of THEM, cultured with hypoxia, with that of TCM, macrophages cultured with tumor conditioning without hypoxia, polarized M2 macrophages, and not treated undifferentiated M0 macrophages. Analysis was performed on the transcriptome of 4 different donors for each sample. Principal component analysis (PCA) showed that THEM clustered in a group clearly separated from TCM cultured without hypoxia, M2, and M0 (Figure 20). This result indicated that i) THEM have a unique molecular signature, which require both tumor-conditioning and hypoxia; ii) THEM molecular signature is different from that of TCM cultured without hypoxia, M2, and M0 macrophages.

The molecular signatures of the different macrophage populations revealed that:
- THEM are characterized by high gene expression level of CYTIP, VEGFA, GLUT1, GLUT3, CXCR4, LFA-1, MMP9, MT2A, MT1G, ANGPTL4, NDRG1, HK2, and VCAN (Table 3, Table 6). THEM express very low/no level of PLXNA2, HSPH1, CYCS, TIGAR (Table 4), ALOX15, CCL8, and CCL26;
- TCM cultured without hypoxia express high level of TGFBI, DAB2, FUCA1, CYP1B1, MAFB, CCL2 and very low/no level of MT2A and MTFP1;
- M2 macrophages express high level of CD206, CD163, CD86, TREM2, IL1RN, IL10, STAT3, STAT6, fabp4, sphk1, HOMOX1, IRF4, PPAR-y, CCL22. M2 macrophage express very low/no low level of CCR7, IL2RA, and CXCL11. The transcriptome analysis confirmed the expected signatures for M2 macrophages (11, 12);
- M0 macrophages express high level of NINJ1, F13A1, SCARB1, and STAB1, AOAH, TLR7, A2M, FNBP1, CD209, SH3KBP1, and ITSN1 as previously described (13).

The most up-regulated genes in THEM cultured with hypoxia were associated to Gene Ontology related to: wound healing (Adm, Bnip3, Pdgfb, Vegfa), angiogenesis (Vegfa, Angptl4, Cxcl8, Lep, Rora, Apln), detoxification and regulation of defence response (Ndrg1, Mt1e, Mt1f, Mt1g, Mt1h, Mt1x, Mt2a, Mt3, Ddit4, Nupr1), response to hypoxia (Hk2, Pfkfb3, Slc2a1, Slc2a3, Cxcr4, Plin2, Adm, Bnip3, Lep, Rora, Ndrg1, Egln3, Mt3, Plod2, Hilpda, Angptl4), extracellular matrix remodelling (Mmp9, Vcan, Fgf11, Cxcl8, Lep, Pdgfb, Plod2, Vegfa, Angptl4, Sulf2, Egln3), and neuronal survival and myelination (Mt3, Jam2, Vldlr, Nupr1, Egln3). (Figure 21, Table 3 and Table 6).

THEM cultured with hypoxia statistically (p<0,05) differentially expressed a large number of genes compared to TCM cultured without hypoxia (4012 genes), M2 macrophages (6786 genes) and M0 macrophages (4097 genes), including the upregulation of PLXNA2, HSPH1, CYCS, TIGAR and the down regulation of PLXNA2, HSPH1, CYCS and TIGAR. (Figure 22).

THEM unique identity was characterized by the specific upregulation of 23 genes and down regulation of 24 genes compared to TCM, M2, M0 macrophages (Table 3 and Table 4).

### Human THEM chemotactic property

Inventors assessed the chemotactic property of THEM, TCM cultured without hypoxia and M2 macrophages to SDF-1 (100 ng/ml) by using transwell assay. Results showed that THEM have statistically significant higher chemotactic response to SDF-1 that TCM, and M2 macrophages (See Figure 23). These data are in line with the upregulation of the SDF-1 receptor CXCR4 gene expression in THEM compared to the others macrophages. These data suggested that hypoxia treatment, present only in THEM, is required for the increased chemotactic response to SDF-1.

### Hypoxia induction is required to confer human and mouse THEM in vitro neuronal regenerative function on human motor neurons

Inventors assessed neuronal regenerative function on human motor neurons of both human and mouse THEM.

In the spinal cord motor neurons extend their processes to connect the central nervous system to the periphery. Following SCI motor neurons are damaged and they are not able to regenerate neuronal processes. In order to assess the motor neuron regenerative potential of THEM, and to verify that hypoxia treatment is required for THEM neuronal regenerative function, inventors tested THEM capability to promote the generation of neuronal processes of motor neurons derived from human induced Pluripotent Stem Cells (iPSCs). Inventors compared the effect of murine THEM cultured with hypoxia, to TCM and murine M2 macrophages on iPSC-derived motor neuron neuronal process extension. Inventors co-cultured macrophages with iPSCs derived motor neurons for two days during the final phase of neuronal differentiation. Considering the correlation between the specific neuronal marker βIII-tubulin (Tub3) and the neuronal cytoskeleton (S S Easter Jr et al., J Neurosci, Initial tract formation in the mouse brain, 1993), inventors quantified the expression the Tub3 as indicator of the number of neuronal processes and of their extension. Tub3 expression area was quantified by threshold normalizing for the total area considered. As showed in Figure 24, the Tub3 expression resulted significantly higher in THEM co-colture (6,502% ± 0,204%) compared to the control (with no cells, CTRL, 4,83% ± 0,162%), the M2 macrophages co-colture (4,779% ± 0,284%) and TCM (3,937% ± 0,192%). These results showed that only THEM cultured with hypoxia are able to promote the increase of the neuronal processes and their extension of human motor neurons confirming their unique neural regenerative potential. Importantly TCM macrophages obtained with tumor conditioning without hypoxia were not able to increase the neuronal processes confirming the fundamental contribute of hypoxia to determinate the regenerative phenotype of THEM.

Inventors further assessed the regenerative effect on human motor neurons observed with mouse macrophages using also human macrophages. Inventors co-cultured human THEM cultured with hypoxia, TCM cultured with tumor conditioning without hypoxia, undifferentiated M0 human macrophages and polarized M2 human macrophages with motor neurons derived from human iPSCs. Following two days of co-culture (at the end of the differentiation protocol), inventors quantified the expression the Tub3 as indicator of the number of neuronal processes and of their extension. Tub3 expression area was quantified by threshold normalizing for the total area. Similar to what observed with murine THEM, this analysis showed that only hypoxia-treated human THEM increased neuronal differentiation and neuronal processes extension (human THEM: 5,96% ± 0,360%) compared to control (with no cells, CTRL, 3,55% ± 0,409%; p<0.001), TCM (2,873% ± 0,184%), M0 (1,94% ± 0,358%, P<0.01), M2 (2,89% ± 0,258%, P<0.01) (Figure 25). These results confirmed the human THEM unique property to induce neuronal differentiation and increased neuronal processes extension, and further supported the required role of hypoxia to obtain THEM regenerative signature.

### Hypoxia induction is required to obtain effective in vivo THEM

Inventors performed an additional *in vivo* experiment to confirm that: i) the presence of THEMs exert a beneficial effect on spinal cord neuronal tissue promoting its regeneration after SCI and ii) the role of hypoxia is fundamental to allow THEM to achieve their regenerative potential.

Inventors tested the *in vivo* effect of hypoxia-treated THEM on the locomotor functionality after severe contusive spinal cord injury (SCI). Inventors compared the regenerative effect of hypoxia-treated THEM with that of TCM macrophages cultured with tumor conditioning and without hypoxia analyzing the locomotory recovery with the Basso Mouse Scale (BMS). The different macrophages were injected at day 3, 14, 17 days post injury. Only mice that at 1 dpi showed a BMS score of less than 0.5 were considered in the analysis, animals with higher BMS score were discarded. As shown in Figure 26, hypoxia-treated THEM and not TCM cultured without hypoxia had effect on motor recovery following SCI mice. In particular, as shown in Figure 26, multiple THEM transplantations showed a significant improvement of the locomotor recovery compared to multiple transplantations of TCM (0.300 ± 0.200) or of vehicle (no cells, 0.711 ± 0.068). Statistical analysis showed a significant difference in the BMS score between THEM transplanted mice and vehicle injected mice at 14, 17, 18, 21, 24, and 28 dpi. Similarly, statistical analysis showed a significant difference in the BMS score between THEM transplanted mice compared to TCM at 17, 18, 21, 24, and 28 dpi. These data confirmed that hypoxia induction is required to obtain THEM regenerative function.

### REFERENCES

1. Iismaa, S.E., et al., Comparative regenerative mechanisms across different mammalian tissues. NPJ Regen Med, 2018. 3: p. 6.
2. Fawcett, J.W. and R.A. Asher, The glial scar and central nervous system repair. Brain Res Bull, 1999. 49(6): p. 377-91.
3. https://www.who.int/news-room/fact-sheets/detail/spinal-cord-injury. Spinal cord injury. Home/Newsroom/Fact sheets/Detail/Spinal cord injury 2013.
4. Ahuja, C.S., et al., Traumatic spinal cord injury. Nat Rev Dis Primers, 2017. 3: p. 17018.
5. McDonald, J.W. and C. Sadowsky, Spinal-cord injury. Lancet, 2002. 359(9304): p. 417-25.
6. Gensel, J.C. and B. Zhang, Macrophage activation and its role in repair and pathology after spinal cord injury. Brain Res, 2015. 1619: p. 1-11.
7. Jin, M.C., et al., Stem cell therapies for acute spinal cord injury in humans: a review. Neurosurg Focus, 2019. 46(3): p. E10.
8. Farzaneh, M., A. Anbiyaiee, and S.E. Khoshnam, Human Pluripotent Stem Cells for Spinal Cord Injury. Curr Stem Cell Res Ther, 2020. 15(2): p. 135-143.
9. Ma, S.F., et al., Adoptive transfer of M2 macrophages promotes locomotor recovery in adult rats after spinal cord injury. Brain Behav Immun, 2015. 45: p. 157-70.
10. Lammertse D.P., et al., Autologous incubated macrophage therapy in acute, complete spinal cord injury: results of the phase 2 randomized controlled multicenter trial. Spinal Cord, 2012 Sep;50(9):661-71
11. John C. Gensel et al., Brain Res., 1619, 1-11, 2015; Ma S-F et al., Brain Behav, Immun., 45, 157-170, 2015;
12. Martinez F.O., et al., Transcriptional profiling of the human monocyte-to-macrophage differentiation and polarization: new molecules and patterns of gene expression. J Immunology, 2006 Nov 15;177(10):7303-11
13. Rodriguez RM et al., Signal Integration and Transcriptional Regulation of the Inflammatory Response Mediated by the GM-/M-CSF Signaling Axis in Human Monocytes. Cell Report, Volume 29, Issue 4, 22 October 2019, Pages 860-872.e5
14. Basso, D.M., et al., Basso Mouse Scale for locomotion detects differences in recovery after spinal cord injury in five common mouse strains. J Neurotrauma, 2006. 23(5): p. 635-59.
15. Decimo I., et al., Nestin- and doublecortin-positive cells reside in adult spinal cord meninges and participate in injury-induced parenchymal reaction. Stem Cells, 2011. 29(12): p. 2062-76;
16. Dolci S., et al. Therapeutic induction of energy metabolism reduces neural tissue damage and increases microglia activation in severe spinal cord injury. Pharmacol Res, 2022 Feb 28;178:106149
17. Charles, J., et al., Musculoskeletal Geometry, Muscle Architecture and Functional Specialisations of the Mouse Hindlimb. PloS one, 2016. 11(4): e0147669;
18. Dolci S. et al., High Yield of Adult Oligodendrocyte Lineage Cells Obtained from Meningeal Biopsy; Front Pharmacol. 2017 Oct 12;8:703
19. Bifari F. et al., Neurogenic Radial Glia-like Cells in Meninges Migrate and Differentiate into Functionally Integrated Neurons in the Neonatal Cortex; Cell Stem Cell, 2017 Mar 2;20(3):360-373.e7
20. Bradbury, E.J. and E.R. Burnside, Moving beyond the glial scar for spinal cord repair. Nat Commun, 2019. 10(1): p. 3879.
21. Stifani, N., Motor neurons and the generation of spinal motor neuron diversity. Front Cell Neurosci, 2014. 8: p. 293.
22. Wang, H., et al., Neurofilament proteins in axonal regeneration and neurodegenerative diseases. Neural Regen Res, 2012. 7(8): p. 620-6.
23. Jeong, H.K., et al., Brain inflammation and microglia: facts and misconceptions. Exp Neurobiol, 2013. 22(2): p. 59-67.
24. Chistiakov, D.A., et al., CD68/macrosialin: not just a histochemical marker. Lab Invest, 2017. 97(1): p. 4-13.
25. Frantz, C., K.M. Stewart, and V.M. Weaver, The extracellular matrix at a glance. J Cell Sci, 2010. 123(Pt 24): p. 4195-200.
26. Zhang, H., et al., Matrix metalloproteinases and neurotrauma: evolving roles in injury and reparative processes. Neuroscientist, 2010. 16(2): p. 156-70.
27. Hernandez-Gerez, E., I.N. Fleming, and S.H. Parson, A role for spinal cord hypoxia in neurodegeneration. Cell Death Dis, 2019. 10(11): p. 861.

## Claims

1. An in vitro or ex vivo method for incubating a population of mononuclear phagocytes isolated from biological samples **characterized in that** ,
the incubation takes place under hypoxic conditions,
and the culture medium comprises factors released from solid tumor or tumor explants or tumor cell lines.

2. The method according to claim 1, wherein the incubated mononuclear phagocyte population is an in vitro culture of monocytes isolated from biological samples and/or wherein the culture medium comprises factors capable of differentiating monocytes into macrophages, preferably the Macrophage Colony-Stimulating Factor (M-CSF).

3. The method according to claim 1 or 2 wherein the population is incubated for from 6 hours or 12 hours to 20 days, more preferably 6 hours-10 days or preferably 6 hours-72 hours or 3-8 days, even more preferably 18 hours-24 hours or 6 days, even more preferably 18 hours or 7 days.

4. The method according to any one of previous claims, wherein the mononuclear phagocytes are macrophages.

5. The method according to one of the preceding claims, wherein the culture medium comprises a tumor supernatant said supernatant being obtained from cultures of solid tumor, tumor explants or tumor cell lines, preferably from fibrosarcoma or glioma, or a medium conditioned from the tumor (CTM) produced from tumor cell lines, tumor explants, or solid tumor, or from resections of dissociated and plated in vitro tumors, preferably for a period of about 6 hours-20 days, more preferably of about 12 hours-72 hours.

6. The method according to one of the preceding claims, where the culture medium consists of a cell culture medium, Eagle's minimal essential medium or derivatives thereof, and / or Roswell Park Memorial Institute (RPMI) 1640, and / or Media 199 and / or Fischer's medium and/or or Iscove's Modified Dulbecco's Medium (IMDM), and 1-99%, preferably 10-90%, more preferably 30-50%, even more preferably 30% or 50%, medium conditioned from the tumor (CTM) or tumor supernatant.

7. The mononuclear phagocytes preferably macrophages obtainable by the method according to any one of claims 1-6 wherein said phagocytes:
i) express at least one of the following genes: CXCR4, CYTIP, SLC2A3 and MT2A; and/or
ii) express low/no level of at least one of the following genes: PLXNA2, HSPH1, CYCS and TIGAR, wherein for low level is intended a gene expression level which is at least five fold lower than the level of expression the housekeeping gene beta-actin,
preferably wherein the at least one gene in i) is MT2A and/or the at least one gene in ii) is TIGAR.

8. An isolated mononuclear phagocyte preferably macrophage which:
i) expresses at least one of the following genes: CXCR4, CYTIP, SLC2A3 and MT2A; and/or
ii) expresses low/no level of at least one of the following genes: PLXNA2, HSPH1, CYCS and TIGAR, preferably TIGAR, wherein for low level is intended a gene expression level which is at least five fold lower than the level of expression of the housekeeping gene beta-actin,
wherein said mononuclear phagocyte expresses at least MT2A.

9. A mononuclear phagocyte preferably a macrophage of claim 7 or 8 wherein said phagocyte:
i) expresses: CXCR4, CYTIP, SLC2A3 and MT2A; and
ii) expresses low/no level: PLXNA2, HSPH1, CYCS and TIGAR, wherein for low level is intended a gene expression level which is at least five fold lower than the level of expression the housekeeping gene beta-actin,

10. The mononuclear phagocyte preferably a macrophage of any one of claims 7-8 wherein said phagocyte expresses CXCR4, MT2A MT1x and expresses low/no level of TIGAR.

11. A population of mononuclear phagocytes comprising at least one mononuclear phagocyte of any one of claims 7-10.

12. The mononuclear phagocytes of any one of claims 7-10 or the population of isolated mononuclear phagocytes of claim 11 preferably said mononuclear phagocytes being macrophages for medical use preferably wherein the phagocytes are administered from 2 days to 60 days or to 1 year after the lesion, preferably 4-21 or 15-60 days after the lesion.

13. The mononuclear phagocytes of any one of claims 7-10 or the population of isolated mononuclear phagocytes of claim 11 or 12 preferably said mononuclear phagocytes being macrophages for use in cell therapy and/or regenerative medicine, preferably in tissue or cell repair, in tissue or cell regeneration, in tissue remodeling, in the treatment and / or in the repair and / or in the healing of wounds, tissue loss in wounds, surgical ulcers, diabetic wounds, in the treatment of conditions of degeneration, including neurodegeneration, retinal degeneration, degeneration due to genetic diseases (such as ALS), autoimmune diseases (arthritis, collagenopathies) or even diabetes in which degeneration of pancreatic islets occurs, in the treatment and / or resolution of inflammation, of inflammation of the tissues, in the treatment of damages, damaged tissues and the like, preferably in the treatment of lesions **characterized by** loss of central nervous system embedded tissue, preferably in the treatment of a spinal lesion or injury, such as a severe spinal injury, or spinal cord injury, preferably a severe or contusive spinal cord injury, preferably wherein the phagocytes are administered from 2 days to 60 days or to 1 year after the lesion, preferably 4-21 or 15-60 days after the lesion.

14. Pharmaceutical composition comprising the mononuclear phagocytes of any one of claims 7-10 or the population of isolated mononuclear phagocytes of claim 11 o preferably said mononuclear phagocytes being macrophages and at least one pharmaceutically acceptable excipient.

## Patentansprüche

1. In-vitro- oder ex vivo-Verfahren zur Inkubation einer aus biologischen Proben isolierten Population mononukleärer Phagozyten, **dadurch gekennzeichnet, dass**,
die Inkubation unter hypoxischen Bedingungen stattfindet,
und das Kulturmedium Faktoren enthält, die von festen Tumoren oder Tumorexplantaten oder Tumorzelllinien freigesetzt werden.

2. Verfahren nach Anspruch 1, wobei die inkubierte mononukleäre Phagozytenpopulation eine In-vitro-Kultur von Monozyten ist, die aus biologischen Proben isoliert wurden, und/oder wobei das Kulturmedium Faktoren enthält, die in der Lage sind, Monozyten in Makrophagen zu differenzieren, vorzugsweise den Makrophagen-Kolonie-stimulierenden Faktor (NT-CSF).

3. Verfahren nach Anspruch 1 oder 2, wobei die Population 6 Stunden oder 12 Stunden bis 20 Tage, vorzugsweise 6 Stunden bis 10 Tage oder vorzugsweise 6 Stunden bis 72 Stunden oder 3 bis 8 Tage, noch bevorzugter 18 Stunden bis 24 Stunden oder 6 Tage, noch bevorzugter 18 Stunden oder 7 Tage lang inkubiert wird.

4. Verfahren nach einem der vorhergehenden Ansprüche, wobei die mononukleären Phagozyten Makrophagen sind.

5. Verfahren nach einem der vorhergehenden Ansprüche, wobei das Kulturmedium einen Tumorüberstand umfasst, wobei der Überstand erhalten wird aus Kulturen von festen Tumoren, Tumorexplantaten oder Tumorzelllinien, vorzugsweise von Fibrosarkomen oder Gliomen, oder ein aus dem Tumor konditioniertes Medium (CTM), das aus Tumorzelllinien, Tumorexplantaten oder festen Tumoren hergestellt wurde, oder aus Resektionen von dissoziierten und plattierten In-vitro-Tumoren, vorzugsweise für einen Zeitraum von etwa 6 Stunden bis 20 Tagen, noch bevorzugter von etwa 12 Stunden bis 72 Stunden.

6. Verfahren nach einem der vorhergehenden Ansprüche, wobei das Kulturmedium aus einem Zellkulturmedium, Eagle's minimal essentiellem Medium oder Derivaten davon und/oder Roswell Park Memorial Institute (RPMI) 1640 und/oder Media 199 und/oder Fischer's Medium und/oder oder Iscove's Modified Dulbecco's Medium (IMDM) und 1 bis 99 %, vorzugsweise 10 bis 90 %, bevorzugter 30 bis 50 %, noch bevorzugter 30 % oder 50 %, aus dem Tumor konditioniertes Medium (CTM) oder Tumorüberstand besteht.

7. Mononukleäre Phagozyten, vorzugsweise Makrophagen, erhältlich durch das Verfahren nach einem der Ansprüche 1 bis 6, wobei die Phagozyten:
i) mindestens eines der folgenden Gene exprimieren: CXCR4, CYTIP, SLC2A3 und MT2A; und/oder
ii) mindestens eines der folgenden Gene mit niedrigem Niveau/gar nicht exprimieren: PLXNA2, HSPH1, CYCS und TIGAR, wobei mit niedrigem Niveau ein Genexpressionsniveau gemeint ist, das mindestens fünfmal niedriger ist als das Expressionsniveau des Housekeeping-Gens beta-Actin, wobei vorzugsweise das mindestens eine Gen in i) MT2A und/oder das mindestens eine Gen in ii) TIGAR ist.

8. Isolierter mononukleärer Phagozyt, vorzugsweise Makrophage, der:
i) mindestens eines der folgenden Gene exprimiert: CXCR4, CYTIP, SLC2A3 und MT2A; und/oder
ii) mindestens eines der folgenden Gene mit niedrigem Niveau/gar nicht exprimiert: PLXNA2, HSPH1, CYCS und TIGAR, vorzugsweise TIGAR, wobei unter niedrigem Niveau ein Genexpressionsniveau zu verstehen ist, das mindestens fünfmal niedriger ist als das Expressionsniveau des Housekeeping-Gens beta-Actin, wobei der mononukleäre Phagozyt mindestens MT2A exprimiert.

9. Mononukleärer Phagozyt, vorzugsweise ein Makrophage nach Anspruch 7 oder 8, wobei der Phagozyt:
i) CXCR4, CYTIP, SLC2A3 und MT2A exprimiert; und
ii) PLXNA2, HSPH1, CYCS und TIGAR mit niedrigem Niveau/gar nicht exprimiert, wobei mit niedrigem Niveau ein Genexpressionsniveau gemeint ist, das mindestens fünfmal niedriger ist als das Expressionsniveau des Housekeeping-Gens Beta-Actin.

10. Mononukleärer Phagozyt, vorzugsweise ein Makrophage nach einem der Ansprüche 7 bis 8, wobei der Phagozyt CXCR4, MT2A MT1x exprimiert und TIGAR mit einem niedrigen Niveau/gar nicht exprimiert.

11. Population mononukleärer Phagozyten, umfassend mindestens einen mononukleären Phagozyten nach einem der Ansprüche 7 bis 10.

12. Mononukleäre Phagozyten nach einem der Ansprüche 7 bis 10 oder die Population isolierter mononukleärer Phagozyten nach Anspruch 11, wobei es sich bei den mononukleären Phagozyten vorzugsweise um Makrophagen zur medizinischen Verwendung handelt, wobei die Phagozyten 2 Tage bis 60 Tage oder bis 1 Jahr nach der Läsion, vorzugsweise 4 bis 21 oder 15 bis 60 Tage nach der Läsion, verabreicht werden.

13. Mononukleäre Phagozyten nach einem der Ansprüche 7 bis 10 oder die Population isolierter mononukleärer Phagozyten nach Anspruch 11 oder 12, wobei es sich bei den mononukleären Phagozyten vorzugsweise um Makrophagen zur Verwendung in der Zelltherapie und/oder der regenerativen Medizin handelt, vorzugsweise in der Gewebe- oder Zellreparatur, in der Gewebe- oder Zellregeneration, im Gewebeumbau, in der Behandlung und/oder in der Reparatur und/oder in der Heilung von Wunden, Gewebeverlust in Wunden, chirurgischen Geschwüren, diabetischen Wunden, in der Behandlung von Degenerationszuständen, einschließlich Neurodegeneration, Netzhautdegeneration, Degeneration aufgrund genetischer Krankheiten (wie ALS), Autoimmunkrankheiten (Arthritis, Kollagenopathien) oder auch Diabetes, bei denen es zu einer Degeneration der Pankreasinseln kommt, bei der Behandlung und/oder Auflösung von Entzündungen, von Gewebeentzündungen, bei der Behandlung von Schäden, beschädigten Geweben und dergleichen, vorzugsweise bei der Behandlung von Läsionen, die durch den Verlust von in das Zentralnervensystem eingebettetem Gewebe gekennzeichnet sind, vorzugsweise bei der Behandlung einer Wirbelsäulenläsion oder -verletzung, wie einer schweren Wirbelsäulenverletzung oder einer Rückenmarksverletzung, vorzugsweise einer schweren oder Rückenmarkkontusionsverletzung, wobei die Phagozyten vorzugsweise 2 Tage bis 60 Tage oder bis 1 Jahr nach der Läsion, vorzugsweise 4 bis 21 oder 15 bis 60 Tage nach der Läsion verabreicht werden.

14. Pharmazeutische Zusammensetzung, die die mononukleären Phagozyten nach einem der Ansprüche 7 bis 10 oder die Population isolierter mononukleärer Phagozyten nach Anspruch 11 oder vorzugsweise die mononukleären Phagozyten, bei denen es sich um Makrophagen handelt, und mindestens einen pharmazeutisch akzeptablen Hilfsstoff umfasst.

## Revendications

1. Procédé in vitro ou ex vivo pour l'incubation d'une population de phagocytes mononucléaires isolés d'échantillons biologiques **caractérisé en ce que**
l'incubation a lieu dans des conditions hypoxiques,
et le milieu de culture comprend des facteurs libérés d'une tumeur solide ou d'explants tumoraux ou de lignées de cellules tumorales.

2. Procédé selon la revendication 1, dans lequel la population de phagocytes mononucléaires incubée est une culture in vitro de monocytes isolés d'échantillons biologiques et/ou dans lequel le milieu de culture comprend des facteurs capables de différencier les monocytes en macrophages, de préférence le facteur de stimulation des colonies de macrophages (M-CSF).

3. Procédé selon la revendication 1 ou 2, dans lequel la population est incubée pendant de 6 heures ou 12 heures à 20 jours, plus préférablement de 6 heures à 10 jours ou de préférence de 6 heures à 72 heures ou de 3 à 8 jours, encore plus préférablement de 18 heures à 24 heures ou 6 jours, encore plus préférablement 18 heures ou 7 jours.

4. Procédé selon l'une quelconque des revendications précédentes, dans lequel les phagocytes mononucléaires sont des macrophages.

5. Procédé selon l'une des revendications précédentes, dans lequel le milieu de culture comprend un surnageant tumoral, ledit surnageant étant obtenu à partir de cultures de tumeur solide, explants tumoraux ou lignées de cellules tumorales, de préférence à partir de fibrosarcome ou de gliome, ou d'un milieu conditionné à partir de la tumeur (CTM) produit à partir de lignées de cellules tumorales, d'explants tumoraux ou de tumeur solide, ou à partir de résections de tumeurs in vitro dissociées et étalées, de préférence pendant une période d'environ 6 heures à 20 jours, plus préférablement d'environ 12 heures à 72 heures.

6. Procédé selon l'une des revendications précédentes, dans lequel le milieu de culture est constitué d'un milieu de culture cellulaire, du milieu essentiel minimal d'Eagle ou de dérivés de celui-ci, et/ou du Roswell Park Memorial Institute (RPMI) 1640, et/ou du milieu 199, et/ou du milieu de Fischer et/ou du milieu de Dulbecco modifié selon Iscove (IMDM), et de 1 à 99 %, de préférence de 10 à 90 %, plus préférablement de 30 à 50 %, encore plus préférablement 30 % ou 50 %, de milieu conditionné à partir de la tumeur (CTM) ou de surnageant tumoral.

7. Phagocytes mononucléaires, de préférence des macrophages pouvant être obtenus par le procédé selon l'une quelconque des revendications 1 à 6, dans lesquels lesdits phagocytes :
i) expriment au moins l'un des gènes suivants : CXCR4, CYTIP, SLC2A3 et MT2A ; et/ou
ii) expriment un niveau faible/nul d'au moins l'un des gènes suivants : PLXNA2, HSPH1, CYCS et TIGAR, dans lesquels un niveau faible signifie un niveau d'expression génique qui est au moins cinq fois inférieur au niveau d'expression du gène de ménage bêta-actine, de préférence dans lesquels l'au moins un gène dans i) est MT2A et/ou l'au moins un gène dans ii) est TIGAR.

8. Phagocyte mononucléaire isolé, de préférence un macrophage, qui :
i) exprime au moins l'un des gènes suivants : CXCR4, CYTIP, SLC2A3 et MT2A ; et/ou
ii) exprime un niveau faible/nul d'au moins l'un des gènes suivants : PLXNA2, HSPH1, CYCS et TIGAR, de préférence TIGAR, dans lesquels un niveau faible signifie un niveau d'expression génique qui est au moins cinq fois inférieur au niveau d'expression du gène de ménage bêta-actine, dans lequel ledit phagocyte mononucléaire exprime au moins MT2A.

9. Phagocyte mononucléaire, de préférence un macrophage, selon la revendication 7 ou 8, dans lequel ledit phagocyte :
i) exprime : CXCR4, CYTIP, SLC2A3 et MT2A ; et
ii) exprime un niveau faible/nul : PLXNA2, HSPH1, CYCS et TIGAR, dans lesquels un niveau faible signifie un niveau d'expression génique qui est au moins cinq fois inférieur au niveau d'expression du gène de ménage bêta-actine.

10. Phagocyte mononucléaire, de préférence un macrophage, selon l'une quelconque des revendications 7 à 8, dans lequel ledit phagocyte exprime CXCR4, MT2A, MT1x et exprime un niveau faible/nul de TIGAR.

11. Population de phagocytes mononucléaires comprenant au moins un phagocyte mononucléaire selon l'une quelconque des revendications 7 à 10.

12. Phagocytes mononucléaires selon l'une quelconque des revendications 7 à 10 ou population de phagocytes mononucléaires isolés selon la revendication 11, de préférence lesdits phagocytes mononucléaires étant des macrophages pour une utilisation médicale, de préférence dans lesquels les phagocytes sont administrés de 2 jours à 60 jours ou jusqu'à 1 an après la lésion, de préférence de 4 à 21 ou de 15 à 60 jours après la lésion.

13. Phagocytes mononucléaires selon l'une quelconque des revendications 7 à 10, ou population de phagocytes mononucléaires isolés selon la revendication 11 ou 12, de préférence lesdits phagocytes mononucléaires étant des macrophages pour une utilisation en thérapie cellulaire et/ou en médecine régénérative, de préférence dans la réparation tissulaire ou cellulaire, dans la régénération tissulaire ou cellulaire, dans le remodelage tissulaire, dans le traitement et/ou la réparation et/ou la cicatrisation de plaies, de perte tissulaire dans les plaies, d'ulcères chirurgicaux, de plaies diabétiques, dans le traitement d'affections dégénératives, notamment une neurodégénérescence, une dégénérescence rétinienne, une dégénérescence due à des maladies génétiques (telles que la SLA), à des maladies auto-immunes (arthrite, collagénopathies) ou même au diabète dans lequel une dégénérescence des îlots pancréatiques se produit, dans le traitement et/ou la résolution d'une inflammation, d'une inflammation des tissus, dans le traitement de lésions, de tissus endommagés et analogues, de préférence dans le traitement de lésions **caractérisées par** une perte de tissu intégré au système nerveux central, de préférence dans le traitement d'une lésion ou d'une blessure de la colonne vertébrale, telle qu'une lésion de la colonne vertébrale grave, ou une lésion de la moelle épinière, de préférence une lésion de la moelle épinière grave ou contusive, de préférence dans lesquels les phagocytes sont administrés de 2 jours à 60 jours ou jusqu'à 1 an après la lésion, de préférence de 4 à 21 jours ou de 15 à 60 jours après la lésion.

14. Composition pharmaceutique comprenant les phagocytes mononucléaires selon l'une quelconque des revendications 7 à 10 ou la population de phagocytes mononucléaires isolés selon la revendication 11 o de préférence lesdits phagocytes mononucléaires étant des macrophages, et au moins un excipient pharmaceutiquement acceptable.
